# EUROPEAN PATENT SPECIFICATION

(11) **EP 2 007 767 B1**
(45) Date of publication and mention of the grant of the patent: **02.11.2011**
(21) Application number: 07712872.6
(22) Date of filing: 09.03.2007
(51) Int. Cl.: C07D 487/04, A61K 31/517, A61P 35/00

(54) **PYRROLOPYRIMIDINE DERIVATIVES USED AS HSP90 INHIBITORS**
ALS HSP90-INHIBITOREN VERWENDETE PYRROLOPYRIMIDINDERIVATE
DERIVES DE PYRROLOPYRIMIDINE EN TANT QU'INHIBITEURS DE HSP90

(30) Priority: 11.03.2006 GB 0604944; 09.09.2006 GB 0617789
(43) Date of publication of application: 31.12.2008
(73) Proprietor: VERNALIS (R&D) LTD, Berkshire RG41 5UA (GB)
(72) Inventor: BROUGH, Paul, Andrew, Berkshire RG41 5UA (GB); DRYSDALE, Martin, James, Berkshire RG41 5UA (GB); DAVIES, Nicholas, Gareth, Morse, Berkshire RG41 5UA (GB); FOLOPPE, Nicolas, Noel, Berkshire RG41 5UA (GB); STOKES, Stephan, Berkshire RG41 5UA (GB)
(74) Representative: Walls, Alan James
(86) International application number: PCT/GB2007/000831
(87) International publication number: WO 2007/104944

(56) References cited:
- WO-A-2005/021552
- WO-A-2006/105372
- US-B1- 6 635 762
- SEELA F ET AL: "RIBOSIDIERUNG VON PYRROLO2,3-CPYRIMIDINEN IN GEGENWART STARKER BASEN RIBOSIDATION OF PYRROLO2,3-DPYRIMIDINES IN THE PRESENCE OF STRONGBASES" CHEMISCHE BERICHTE, VERLAG CHEMIE GMBH. WEINHEIM, DE, vol. 113, no. 8, 1980, pages 2808-2813, XP000995468 ISSN: 0009-2940

## Description

This invention relates to substituted bicyclic pyrrolopyrimidine compounds having HSP90 inhibitory activity, to the use of such compounds in medicine, in relation to diseases which are responsive to inhibition of HSP90 activity such as cancers, and to pharmaceutical compositions containing such compounds.

### Background to the invention

Molecular chaperones maintain the appropriate folding and conformation of proteins and are crucial in regulating the balance between protein synthesis and degradation. They have been shown to be important in regulating many important cellular functions, such as cell proliferation and apoptosis (Jolly and Morimoto, 2000; Smith et al., 1998; Smith, 2001).

### Heat Shock Proteins (Hsps)

Exposure of cells to a number of environmental stresses, including heat shock, alcohols, heavy metals and oxidative stress, results in the cellular accumulation of a number of chaperones, commonly known as heat shock proteins (Hsps). Induction of Hsps protects the cell against the initial stress insult, enhances recovery and leads to maintenance of a stress tolerant state. It has also become clear, however, that certain Hsps may also play a major molecular chaperone role under normal, stress-free conditions by regulating the correct folding, degradation, localization and function of a growing list of important cellular proteins.

A number of multigene families of Hsps exist, with individual gene products varying in cellular expression, function and localization. They are classified according to molecular weight, e.g., Hsp70, Hsp90, and Hsp27. Several diseases in humans can be acquired as a result of protein misfolding (reviewed in Tytell et al., 2001; Smith et al., 1998). Hence the development of therapies which disrupt the molecular chaperone machinery may prove to be beneficial. In some conditions (e.g., Alzheimer's disease, prion diseases and Huntington's disease), misfolded proteins can cause protein aggregation resulting in neurodegenerative disorders. Also, misfolded proteins may result in loss of wild type protein function, leading to deregulated molecular and physiological functions in the cell. Hsps have also been implicated in cancer. For example, there is evidence of differential expression of Hsps which may relate to the stage of tumour progression (Martin et al., 2000; Conroy et al., 1996; Kawanishi et al., 1999; Jameel et al., 1992; Hoang et al., 2000; Lebeau et al., 1991). As a result of the involvement of Hsp90 in various critical oncogenic pathways and the discovery that certain natural products with anticancer activity are targeting this molecular chaperone suggests that inhibiting the function of Hsp90 may be useful in the treatment of cancer. To this end, the first in class natural product 17AAG is currently in Phase II clinical trials.

### Hsp90

Hsp90 constitutes about 1-2% of total cellular protein. In cells, it forms dynamic multi-protein complexes with a wide variety of accessory proteins (referred to as co-chaperones) which appear responsible for regulating the chaperone function. It is essential for cell viability and it exhibits dual chaperone functions (Young et al., 2001). When cells undergo various environmental cellular stresses, Hsp90 forms a core component of the cellular stress response by interacting with many proteins after their native conformation has been altered. Environmental stresses, such as heat shock, heavy metals or alcohol, generate localised protein unfolding. Hsp90 (in concert with other chaperones) binds these unfolded proteins allowing adequate refolding and preventing non-specific aggregation (Smith et al., 1998).. In addition, recent results suggest that Hsp90 may also play a role in buffering against the effects of mutation, presumably by correcting the inappropriate folding of mutant proteins (Rutherford and Lindquist, 1998). However, Hsp90 also has an important regulatory role. Under normal physiological conditions, together with its endoplasmic reticulum homologue GRP94, Hsp90 plays a housekeeping role in the cell, maintaining the conformational stability and maturation of many client proteins. These can be subdivided into three groups: (a) steroid hormone receptors (e.g. estrogen receptor, progesterone receptor) (b) Ser/Thr or tyrosine kinases (e.g. Her2, Raf-1, CDK4, and Lck), and (c) a collection of apparently unrelated proteins, e.g. mutant p53 and the catalytic subunit of telomerase hTERT. It has also been shown recently that Hsp90 is responsible for stabilising and activating mutated kinases where the wild type kinase is not an Hsp90 client (for an example see the B-Raf story published in da Rocha Dias et al., 2005). All of these proteins play key regulatory roles in many physiological and biochemical processes in the cell. New client proteins of Hsp90 are being constantly identified; see http://www.picard.ch/downloads/Hsp90interactors.pdf for the most up to date list.

The highly conserved Hsp90 family in humans consists of four genes, namely the cytosolic Hsp90α and Hsp90β isoforms (Hickey et al., 1989), GRP94 in the endoplasmic reticulum (Argon et al., 1999) and Hsp75/TRAP1 in the mitochondrial matrix (Felts et al., 2000). Apart from the differences in sub-cellular localisation, very little is known about the differences in function between Hsp90α/β, GRP94 and TRAP1. Initial reports suggesting that certain client proteins were chaperoned by a specific Hsp90 (e.g. Her2 by Grp94 alone) appear to have been erroneous.

Hsp90 participates in a series of complex interactions with a range of client and regulatory proteins (Smith, 2001). Although the precise molecular details remain to be elucidated, biochemical and X-ray crystallographic studies (Prodromou et al., 1997; Stebbins et al., 1997) carried out over the last few years have provided increasingly detailed insights into the chaperone function of Hsp90.

Following earlier controversy on this issue, it is now clear that Hsp90 is an ATP-dependent molecular chaperone (Prodromou et al, 1997), with dimerisation of the nucleotide binding domains being essential for ATP hydrolysis, which is in turn essential for chaperone function (Prodromou et al, 2000a). Binding of ATP results in the formation of a toroidal dimer structure in which the N terminal domains are brought into closer contact with each other resulting in a conformational switch known as the 'clamp mechanism' (Prodromou and Pearl, 2000b). This conformational switching is, in part, regulated by the various co-chaperones associated with Hsp90 (Siligardi et al., 2004).

### Known Hsp90 Inhibitors

The first class of Hsp90 inhibitors to be discovered was the benzoquinone ansamycin class, which includes the compounds herbimycin A and geldanamycin. They were shown to reverse the malignant phenotype of fibroblasts transformed by the v-*Src* oncogene (Uehara et al., 1985), and subsequently to exhibit potent antitumour activity in both *in vitro* (Schulte et al., 1998) and *in vivo* animal models (Supko et al., 1995).

Immunoprecipitation and affinity matrix studies have shown that the major mechanism of action of geldanamycin involves binding to Hsp90 (Whitesell et al., 1994; Schulte and Neckers, 1998). Moreover, X-ray crystallographic studies have shown that geldanamycin competes at the ATP binding site and inhibits the intrinsic ATPase activity of Hsp90 (Prodromou et al., 1997; Panaretou et al., 1998). This interruption of the chaperone cycle (through blockage of the ATP turnover) causes the loss of the co-chaperone p23 from the complex and the targeting of the client proteins for degradation via the ubiquitin proteasome pathway. 17-Allylamino, 17-demethoxygeldanamycin (17AAG) retains the property of Hsp90 inhibition resulting in client protein depletion and antitumour activity in cell culture and xenograft models (Schulte et al, 1998; Kelland et al, 1999), but has significantly less hepatotoxicity than geldanamycin (Page et al, 1997). Of interest, 17AAG has been shown to be much more active on tumour cells than its affinity for purified Hsp90 would suggest. This has lead to the suggestion that tumour cells (but not non-tumourigenic cells) contain a high-affinity conformation of Hsp90 to which 17AAG binds more tightly, and confers tumour selectivity on Hsp90 inhibitors (Kamal et al., 2003). 17AAG is currently being evaluated in Phase II clinical trials.

Radicicol is a macrocyclic antibiotic shown to reverse the malignant phenotype of v-*Src* and v-*Ha*-*Ras* transformed fibroblasts (Kwon et al, 1992; Zhao et al, 1995). It was shown to degrade a number of signalling proteins as a consequence of Hsp90 inhibition (Schulte et al., 1998). X-ray crystallographic data confirmed that radicicol also binds to the N terminal domain of Hsp90 and inhibits the intrinsic ATPase activity (Roe et al., 1998). Radicicol lacks antitumour activity *in vivo* due to the unstable chemical nature of the compound.

Coumarin antibiotics are known to bind to bacterial DNA gyrase at an ATP binding site homologous to that of the Hsp90. The coumarin, novobiocin, was shown to bind to the carboxy terminus of Hsp90, i.e., at a different site to that occupied by the benzoquinone ansamycins and radicicol which bind at the N-terminus (Marcu et al., 2000b). However, this still resulted in inhibition of Hsp90 function and degradation of a number of Hsp90-chaperoned signalling proteins (Marcu et al., 2000a). Geldanamcyin cannot bind Hsp90 subsequent to novobiocin; this suggests that some interaction between the N and C terminal domains must exist and is consistent with the view that both sites are important for Hsp90 chaperone properties.

A purine-based Hsp90 inhibitor, PU3, has been shown to result in the degradation of signalling molecules, including Her2, and to cause cell cycle arrest and differentiation in breast cancer cells (Chiosis et al., 2001). Recent studies have identified other purine-based compounds with activity against Her2 and activity in cell growth inhibition assays (Dymock et al 2004; Kasibhatla et al 2003; Llauger et al 2005).

Patent publications WO 2004/050087, WO 2004/056782, WO 2004/072051, WO 2004/096212, WO 2005/000300, WO 2005/021552, WO 2005/034950 relate to Hsp90 inhibitors.

### Hsp90 as a Therapeutic Target

Due to its involvement in regulating a number of signalling pathways that are crucially important in driving the phenotype of a tumour, and the discovery that certain bioactive natural products exert their effects via Hsp90 activity, the molecular chaperone Hsp90 is currently being assessed as a new target for anticancer drug development (Neckers et al., 1999).

The predominant mechanism of action of geldanamycin, 17AAG, and radicicol involves binding to Hsp90 at the ATP binding site located in the N-terminal domain of the protein, leading to inhibition of the intrinsic ATPase activity of Hsp90 (Prodromou et al., 1997; Stebbins et al., 1997; Panaretou et al., 1998).

Inhibition of Hsp90 ATPase activity by 17AAG induces the loss of p23 from the chaperone-client protein complex interrupting the chaperone cycle. This leads to the formation of a Hsp90-client protein complex that targets these client proteins for degradation via the ubiquitin proteasome pathway (Neckers et al., 1999; Whitesell & Lindquist, 2005). Treatment with Hsp90 inhibitors leads to selective degradation of important proteins (for example Her2, Akt, estrogen receptor and CDK4) involved in cell proliferation, cell cycle regulation and apoptosis, processes which are fundamentally important in cancer.

The preclinical development of 17AAG as an anticancer agent has been well documented (Sausville et al., 2003) and is currently undergoing Phase II clinical trials. Phase I clinical trials results have been recently published (Banerji et al., 2005; Goetz et al., 2005; Ramanathan et al., 2005 and Grem et al., 2005). Of all these trials, the one conducted by Banerji et al. proved the most positive with a maximum dose of 450mg/m2/week achieved with PD marker responses in the majority of patients and possible antitumour activity in two patients

Inhibition of Hsp90 function has been shown to cause selective degradation of important signalling proteins involved in cell proliferation, cell cycle regulation and apoptosis, processes which are fundamentally important and which are commonly deregulated in cancer (Hostein et al., 2001). An attractive rationale for developing drugs against this target for use in the clinic is that by simultaneously depleting proteins associated with the transformed phenotype, one may obtain a strong antitumour effect and achieve a therapeutic advantage against cancer versus normal cells. These events downstream of Hsp90 inhibition are believed to be responsible for the antitumour activity of Hsp90 inhibitors in cell culture and animal models (Schulte et al., 1998; Kelland et al., 1999).

Recent work has shown that the acetylation status of Hsp90 also plays a role in the control of the chaperone cycle. Inhibition of HDAC6 by either small molecule inhibitors or through siRNA gene targeting interrupts the chaperone cycle. Such treatments cause client protein degradation in a fashion analogous to small molecule ATP site inhibitors (Kovacs et al, 2005; Aoyagi & Archer, 2005).

Recent reports (see Cowen et. al. Science 309, 2185 (2005) and Heitman, Science 309, 2175, 2005) also indicate that Hsp90 is required both for the emergence of fungal isolates resistant to antifungal agents, and for continued drug resistance once this has occurred. Hsp90 inhibitors therefore resensitise strains which have become resistant to, for example, azole antifungal agents (e.g. fluconazole) as well as newer agents such as echinocandins.

### Detailed description of the invention

In one broad aspect the present invention provides a compound of formula (I), or a pharmaceutically acceptable salt thereof: 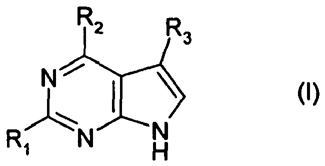 wherein
R₁ is hydrogen, fluoro, chloro, bromo, or a radical of formula ((1A):

   -X-Alk¹-(Z)ₘ-(Alk²)ₙ-Q (IA)

   wherein
   X is -O-, -S- -S(O)-, -SO₂-, or -NH-,
   Z is -O-, -S-, -(C=O)-, -(C=S)-, -S(O)-, -SO₂-, -NR^{A}-, or, in either orientation -C(=O)O-, -C(=O)NR^{A}-, -C(=S)NR^{A}-, -SO₂NR^{A}-, -NR^{A}C(=O)-, or -NR^{A}SO₂- wherein R^{A} is hydrogen or C₁-C₆ alkyl Alk¹ and Alk² are optionally substituted divalent C₁-C₃ alkylene or C₂-C₃ alkenylene radicals,
   m, n and p are independently 0 or 1, and
   Q is hydrogen or an optionally substituted carbocyclic or heterocyclic radical;
R₂ is optionally substituted phenyl;
R₃ is cyano (-CN), fluoro, chloro, bromo, methyl in which in which one or more hydrogen atoms are optionally replaced by fluorine atoms, ethyl in which in which one or more hydrogen atoms are optionally replaced by fluorine atoms, cyclopropyl, -OH, -CH₂OH,-C(=O)NH₂, -C(=O)CH₃, or -NH₂;
and wherein the term "optionally substituted" means unsubstituted or substituted with at least one substituent selected from (C₁-C₆)alkyl, (C₁-C₆)alkoxy, hydroxy, hydroxy(C₁-C₆)alkyl, mercapto, mercapto(C₁-C₆)alkyl, (C₁-C₆)alkylthio, monocyclic carbocyclic of 3-6 ring carbon atoms, monocyclic heterocyclic of 5 or 6 ring atoms, halo, trifluoromethyl, trifluoromethoxy, nitro, nitrile, oxo, -COOH, -COOR^{A}, -COR^{A}, -SO₂R^{A}, -CONH₂, -SO₂NH₂, -CONHR^{A}, -SO₂NHR^{A}, -CONR^{A}R^{B}, -SO₂NR^{A}R^{B}, -NH₂, -NHR^{A}, -NR^{A}R^{B}, -OCONH₂, -OCONHR^{A} , -OCONR^{A}R^{B}, -NHCOR^{A}, -NHCOOR^{A}, -NR^{B}COOR^{A}, -NHSO₂OR^{A}, -NR^{B}SO₂OR^{A}, -NHCONH₂, -NR^{A}CONH₂, -NHCONHR^{B}, -NR^{A}CONHR^{B}, -NHCONR^{A}R^{B}, or -NR^{A}CONR^{A}R^{B} wherein R^{A} and R^{B} are independently a (C₁-C₆)alkyl group; and in the case where the said substituent contains an alkyl radical, that alkyl radical may be substituted by a monocyclic carbocyclic group of 3-6 ring carbon atoms, or a monocyclic heterocyclic group of 5 or 6 ring atoms; and in the case where the said substituent is or comprises a monocyclic carbocyclic group of 3-6 ring carbon atoms, or a monocyclic heterocyclic group of 5 or 6 ring atoms, that ring may itself be substituted by any of the foregoing non-cyclic optional substituents.

The compounds are applicable to the treatment of diseases in which HSP90 activity is implicated, including use for immunosuppression or the treatment of viral disease, drug resistant fungal infection (since HSP90 inhibitors are able to resensitise strains which have become resistant to, for example, azole antifungal agents (e.g. fluconazole) as well as newer agents such as echinocandins), inflammatory diseases such as rheumatoid arthritis, asthma, multiple sclerosis, Type I diabetes, lupus, psoriasis and inflammatory bowel disease; cystic fibrosis angiogenesis-related disease such as diabetic retinopathy, haemangiomas, and endometriosis; or for protection of normal cells against chemotherapy-induced toxicity; or diseases where failure to undergo apoptosis is an underlying factor; or protection from hypoxia-ischemic injury due to elevation of Hsp70 in the heart and brain; scrapie/CJD, Huntingdon's or Alzheimer's disease. Use for the treatment of cancer is especially indicated.

As used herein, the term "(Cₐ-C_{b})alkyl" wherein a and b are integers refers to a straight or branched chain alkyl radical having from a to b carbon atoms. Thus when a is 1 and b is 6, for example, the term includes methyl, ethyl, n-propyl, isopropyl, n-butyl, isobutyl, sec-butyl, t-butyl, n-pentyl and n-hexyl.

As used herein the term "divalent (Cₐ-C_{b})alkylene radical" wherein a and b are integers refers to a saturated hydrocarbon chain having from a to b carbon atoms and two unsatisfied valences.

As used herein the term "(Cₐ-C_{b})alkenyl" wherein a and b are integers refers to a straight or branched chain alkenyl moiety having from a to b carbon atoms having at least one double bond of either E or Z stereochemistry where applicable. The term includes, for example, vinyl, allyl, 1- and 2-butenyl and 2-methyl-2-propenyl.

As used herein the term "divalent (Cₐ-C_{b})alkenylene radical" refers to a hydrocarbon chain having from a to b carbon atoms, at least one double bond, and two unsatisfied valences.

As used herein the term "cycloalkyl" refers to a saturated carbocyclic radical having from 3-8 carbon atoms and includes, for example, cyclopropyl, cyclobutyl, cyclopentyl, cyclohexyl, cycloheptyl and cyclooctyl.

As used herein the term "cycloalkenyl" refers to a carbocyclic radical having from 3-8 carbon atoms containing at least one double bond, and includes, for example, cyclopentenyl, cyclohexenyl, cycloheptenyl and cyclooctenyl.

As used herein the term "aryl" refers to a mono-, bi- or tri-cyclic carbocyclic aromatic radical, and includes aromatic monocyclic or bicyclic carbocyclic radicals fused to a non aromatic carbocyclic or heterocyclic ring. Illustrative of such radicals are phenyl, biphenyl and napthyl, and radicals of the formula: 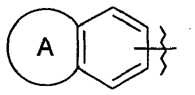 wherein ring A (i) is optionally substituted, (ii) has 5 or 6 ring members including the carbons of the phenyl ring to which it is fused, and (iii) has at least one heteroatom O, S or N hetero atom as a ring member.

As used herein the term "carbocyclic" refers to a cyclic radical whose ring atoms are all carbon, and includes aryl, cycloalkyl, and cycloalkenyl radicals.

As used herein the term "heteroaryl" refers to a mono-, bi- or tri-cyclic aromatic radical containing one or more heteroatoms selected from S, N and O. Illustrative of such radicals are thienyl, benzthienyl, furyl, benzfuryl, pyrrolyl, imidazolyl, benzimidazolyl, thiazolyl, benzthiazolyl, isothiazolyl, benzisothiazolyl, pyrazolyl, oxazolyl, benzoxazolyl, isoxazolyl, benzisoxazolyl, isothiazolyl, triazolyl, benztriazolyl, thiadiazolyl, oxadiazolyl, pyridinyl, pyridazinyl, pyrimidinyl, pyrazinyl, triazinyl, indolyl and indazolyl.

As used herein the unqualified term "heterocyclyl" or "heterocyclic" includes "heteroaryl" as defined above, and in particular refers to a mono-, bi- or tri-cyclic non-aromatic radical containing one or more heteroatoms selected from S, N and O, and to groups consisting of a monocyclic non-aromatic radical containing one or more such heteroatoms which is covalently linked to another such radical or to a monocyclic carbocyclic radical. Illustrative of such radicals are pyrrolyl, furanyl, thienyl, piperidinyl, imidazolyl, oxazolyl, isoxazolyl, thiazolyl, thiadiazolyl, pyrazolyl, pyridinyl, pyrrolidinyl, pyrimidinyl, morpholinyl, piperazinyl, indolyl, morpholinyl, benzfuranyl, pyranyl, isoxazolyl, benzimidazolyl, methylenedioxyphenyl, ethylenedioxyphenyl, maleimido and succinimido groups.

Unless otherwise specified in the context in which it occurs, the term "substituted" as applied to any moiety herein means substituted with at least one substituent, for example selected from (C₁-C₆)alkyl, (C₁-C₆)alkoxy (including methylenedioxy and ethylenedioxy substitution on adjacent carbon atoms of a carbocyclic or heterocyclic ring), hydroxy, hydroxy(C₁-C₆)alkyl, mercapto, mercapto(C₁-C₆)alkyl, (C₁-C₆)alkylthio, monocyclic carbocyclic of 3-6 ring carbon atoms, monocyclic heterocyclic of 5 or 6 ring atoms, halo (including fluoro and chloro), trifluoromethyl, trifluoromethoxy, nitro, nitrile (-CN), oxo, -COOH, -COOR^{A}, -COR^{A}, -SO₂R^{A}, -CONH₂, -SO₂NH₂ -CONHR^{A}, -SO₂NHR^{A}, -CONR^{A}R^{B}, -SO₂NR^{A}R^{B}, -NH₂, -NHR^{A}, -NR^{A}R^{B}, -OCONH₂, -OCONHR^{A} , -OCONR^{A}R^{B}, -NHCOR^{A}, -NHCOOR^{A}, -NR^{B}COOR^{A}, -NHSO₂OR^{A}, -NR^{B}SO₂OR^{A}, -NHCONH₂, -NR^{A}CONH₂, -NHCONHR^{B}, -NR^{A}CONHR^{B}, -NHCONR^{A}R^{B}, or -NR^{A}CONR^{A}R^{B} wherein R^{A} and R^{B} are independently a (C₁-C₆)alkyl group. In the case where the optional substituent contains an alkyl radical, that alkyl radical may be substituted by a monocyclic carbocyclic group of 3-6 ring carbon atoms, or a monocyclic heterocyclic group of 5 or 6 ring atoms. In the case where the optional substituent is or comprises a monocyclic carbocyclic group of 3-6 ring carbon atoms, or a monocyclic heterocyclic group of 5 or 6 ring atoms, that ring may itself be substituted by any of the non-cyclic optional substituents listed above. An "optional substituent" may be one of the substituent groups encompassed in the above description.

As used herein the term "salt" includes base addition, acid addition and quaternary salts. Compounds of the invention which are acidic can form salts, including pharmaceutically or veterinarily acceptable salts, with bases such as alkali metal hydroxides, e.g. sodium and potassium hydroxides; alkaline earth metal hydroxides e.g. calcium, barium and magnesium hydroxides; with organic bases e.g. N-ethyl piperidine, dibenzylamine and the like. Those compounds (I) which are basic can form salts, including pharmaceutically or veterinarily acceptable salts with inorganic acids, e.g. with hydrohalic acids such as hydrochloric or hydrobromic acids, sulphuric acid, nitric acid or phosphoric acid and the like, and with organic acids e.g. with acetic, tartaric, succinic, fumaric, maleic, malic, salicylic, citric, methanesulphonic and p-toluene sulphonic acids and the like. Any unqualified reference herein to a compound which falls within formula (I) is to be construed as a reference to that compound, irrespective of whether it is or is not in the form of salt.

For a review on suitable salts, see Handbook of Pharmaceutical Salts: Properties, Selection, and Use by Stahl and Wermuth (Wiley-VCH, Weinheim, Germany, 2002).

In common with many organic compounds useful in medicine, at least some of the compounds of the invention are expected to be recoverable as crystalline hydrates and solvates. Such hydrates and solvates are of course merely specific physico-chemical forms of the active compounds of the invention and therefore form part of the invention. Any unqualified reference herein to a compound which falls within formula (I) is to be construed as a reference to that compound, irrespective of whether it is or is not in the form of a hydrate or solvate. The term 'solvate' is used herein to describe a molecular complex comprising the compound of the invention and a stoichiometric amount of one or more pharmaceutically acceptable solvent molecules, for example, ethanol. The term 'hydrate' is employed when said solvent is water.

The nitrogens in the fused pyrimidine ring present in the compounds of the invention may be oxidesed to form N-oxides. Such N-oxides substantially retain the HSP90 inhibitory activity of the parent compounds, and are thus form part of the invention. Any unqualified reference herein to a compound which falls within formula (I) is to be construed as a reference to that compound, irrespective of whether it is or is not in the form of an N-oxide.

Compounds with which the invention is concerned which may exist in one or more stereoisomeric form, because of the presence of asymmetric atoms or rotational restrictions, can exist as a number of stereoisomers with R or S stereochemistry at each chiral centre or as atropisomeres with R or S stereochemistry at each chiral axis. The invention includes all such enantiomers and diastereoisomers and mixtures thereof.

So-called 'pro-drugs' of the compounds of formula (I) are certain derivatives of compounds of formula (I) which may have little or no pharmacological activity themselves can, when administered into or onto the body, be converted into compounds of formula (I) having the desired activity, for example, by hydrolytic cleavage. Such derivatives are referred to as 'prodrugs'. Further information on the use of prodrugs may be found in Pro-drugs as Novel Delivery Systems, Vol. 14, ACS Symposium Series (T. Higuchi and W. Stella) and Bioreversible Carriers in Drug Design, Pergamon Press, 1987 (ed. E. B. Roche, American Pharmaceutical Association).

Prodrugs can, for example, be produced by replacing appropriate functionalities present in the compounds of formula (I) with certain moieties known to those skilled in the art as 'pro-moieties' as described, for example, in Design of Prodrugs by H. Bundgaard (Elsevier, 1985).

Compounds of formula (I) are expected to form metabolites, that is, compounds formed *in vivo* upon administration of the drug. Some examples of metabolites include
(i) where the compound of formula (I) contains a methyl group, an hydroxymethyl derivative thereof (-CH₃ -> -CH₂OH):
(ii) where the compound of formula (I) contains an alkoxy group, an hydroxy derivative thereof (-OR -> -OH);
(iii) where the compound of formula (I) contains a tertiary amino group, a secondary amino derivative thereof (-NR¹R² -> -NHR¹ or -NHR²);
(iv) where the compound of formula (I) contains a secondary amino group, a primary derivative thereof (-NHR¹ -> -NH₂);
(v) where the compound of formula (I) contains a phenyl moiety, a phenol derivative thereof (-Ph -> -PhOH); and
(vi) where the compound of formula (I) contains an amide group, a carboxylic acid derivative thereof (-CONH₂ -> COOH).

### The group R₁

When R₁ is a radical of formula (IA):

-X-Alk¹-(Z)ₘ-(Alk²)ₙ-Q (IA)

X may be -O-, -S- -S(O)-, -SO₂-, or -NH-. At present -0- and -S- are preferred;
when present, Z may be -O-, -S-, -(C=O)-, -(C=S)-, -S(O)-, -SO₂-, -NR^{A}-, or, in either orientation -C(=O)O-, -C(=O)NR^{A}- , -C(=S)NR^{A}-, -SO₂NR^{A}-, -NR^{A}C(=O)-, or -NR^{A}SO₂- wherein R^{A} is hydrogen or C₁-C₆ alkyl. At present -NR^{A}- is preferred;
Alk¹ (and Alk² when present) may be, for example -CH₂-, -CH₂CH₂-,-CH₂CH₂CH₂-,-CH(CH₃)CH₂- or -CH₂CH=CH-;
m, n and p are independently 0 or 1. Thus, in one class of radicals (IA), m and n are both 0. In another class of radicals (IA), m is 1 and n is 0. In a further class of radicals (IA), m is 0 and n is 1;
Q may be hydrogen or an optionally substituted carbocyclic or heterocyclic radical. Examples of carbocyclic radicals Q include phenyl, cyclopropyl, cyclobutyl, cyclopentyl and cyclohexyl. Examples of heterocyclic radicals Q include heteroaryl radicals such as pyridyl, thienyl and furanyl, and non-aromatic heterocyclic radicals such as piperidinyl, piperazinyl, tetrahydropyrrolyl, and morpholinyl.
Currently it is preferred that Alk¹ and Alk² are unsubstituted. Q (when carbocyclic or heterocyclic) may be unsubstituted, but when substituted, optional substituents may be selected from, for example, methyl, ethyl, n- or isopropyl, vinyl, allyl, methoxy, ethoxy, n-propyloxy, isopropyloxy, benzyloxy, allyloxy, cyanomethoxy, fluoro, chloro, bromo, cyano, oxo, formyl, methyl-, ethyl-, or n-propyl-carbonyloxy, methyl- or ethylaminocarbonyl, and substituents of formula -O(CH₂)ₐZ¹ wherein a is 1, 2 or 3 and Z¹ is a primary, secondary, tertiary or cyclic amino group, or a C₁-C₆alkoxy group; or of formula -(Alk³)_{b}Z¹ wherein Alk³ is a divalent straight or branched chain (C₁-C₃) alkylene, b is 0 or 1, and Z¹ is a primary, secondary, tertiary or cyclic amino group, or a C₁-C₆alkoxy group.

One type of R₁ substituent has the formula -O(CH₂)ₙZ¹ or -S(CH₂)ₙZ¹ wherein n is 1, 2 or 3 and Z¹ is a primary, secondary, tertiary or cyclic amino group, the latter being optionally substituted, or a C₁-C₆alkoxy group. Specific examples of R₁ include hydrogen, methoxy, ethoxy, methylthio, ethylthio, hydroxyeththylthio, methylamino, diethylaminomethylthio, methylaminocarbonylmethylthio, and groups of formula (A) -(H): 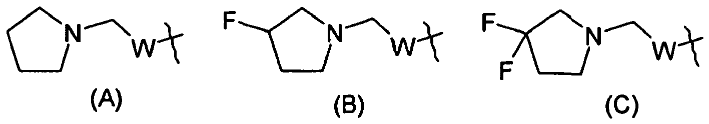 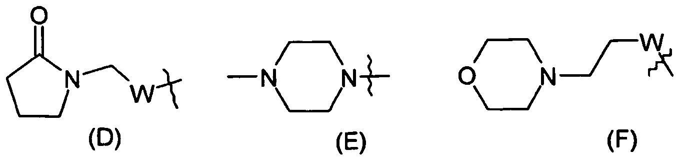 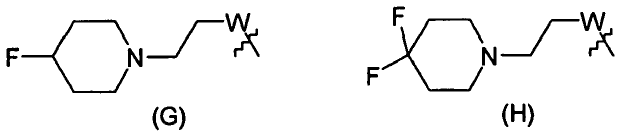 wherein W is -O- or -S-.

### The group R₂

R₂ is a optionally substituted phenyl. In one class of compounds (I) of the invention, R₂ is phenyl, optionally substituted by one or more substituents selected from methyl, trifuoromethyl, ethyl, n- or isopropyl, vinyl, allyl, methoxy, trifuoromethoxy, ethoxy, methylenedioxy, ethylenedioxy, n-propyloxy, benzyloxy, allyloxy, cyanomethoxy, fluoro, chloro, bromo, cyano, formyl, methyl-, ethyl-, or n-propyl-carbonyloxy, methyl- or ethylaminocarbonyl, and substituents of formula -O(CH₂)ₙZ¹ wherein n is 1, 2 or 3 and Z¹ is a primary, secondary, tertiary or cyclic amino group, or a C₁-C₆alkoxy group; or of formula -(Alk³)ₘZ¹ wherein Alk³ is a divalent straight or branched chain (C₁-C₃) alkylene, m is 0 or 1, and Z¹ is a primary, secondary, tertiary or cyclic amino group, the latter being optionally substituted, or a C₁-C₆alkoxy group. Optional substituents when R₂ is phenyl are preferably in the 2- and/or 4- and/or 5-position of the phenyl ring.

### The group R₃

At present, it is preferred that R₃ is cyano (-CN).

Particularly preferred at present are compounds of the formula (II): 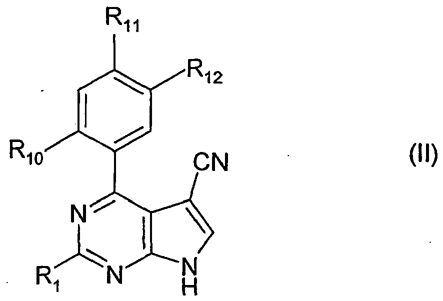
R₁ is (a) C₁-C₆ alkylthio or C₁-C₆ alkoxy in either of which one or more hydrogen atoms are optionally replaced by fluorine atoms, or (b) a substituent of formula -O(CH₂)ₙZ¹ or -S(CH₂)ₙZ¹ wherein n is 1, 2 or 3 and Z¹ is a primary, secondary, tertiary or cyclic amino group the latter being optionally substituted.
R₁₀ is H, Cl, Br, or -CH₃;
R₁₁ is hydrogen, Cl, Br, CN, methyl, ethyl, n- or iso-propyl, methoxy, ethoxy, vinyl or allyl; and
R₁₂ is (i) a radical of formula -O(CH₂)ₙZ¹ or -S(CH₂)ₙZ¹ wherein n is 1, 2 or 3 and Z¹ is (i) a primary, secondary, tertiary or cyclic amino group, or a C₁-C₆alkoxy group; or (ii) a radical of formula -(Alk³)ₘZ¹ wherein Alk³ is a divalent straight or branched chain (C₁-C₃) alkylene, m is 0 or 1, and Z¹ is a primary, secondary, tertiary or cyclic amino group, or a C₁-C₆alkoxy group.

Specific examples of compounds of the invention include those of the Examples herein.

There are multiple synthetic strategies for the synthesis of the compounds (I) with which the present invention is concerned, but all rely on known chemistry, known to the synthetic organic chemist. Thus, compounds according to formula (I) can be synthesised according to procedures described in the standard literature and are well-known to the one skilled in the art. Typical literature sources are *"*Advanced organic chemistry", 4th Edition (Wiley), J March, *"*Comprehensive Organic Transformation", 2nd Edition (Wiley), R.C. Larock , *"*Handbook of Heterocyclic Chemistry", 2nd Edition (Pergamon), A.R. Katritzky), review articles such as found in *"Synthesis", "Acc. Chem. Res." , "Chem. Rev",* or primary literature sources identified by standard literature searches online or from secondary sources such as *"Chemical Abstracts"* or *"Beilstein".* Such literature methods include those of the preparative Examples herein, and methods analogous thereto.

The compounds of the invention are inhibitors of HSP90 and are useful in the treatment of diseases which are responsive to inhibition of HSP90 activity such as cancers; viral diseases such as Hepatitis C (HCV) (Waxman, 2002); Immunosupression such as in transplantation (Bijlmakers, 2000 and Yorgin, 2000); Anti-inflammatory diseases (Bucci, 2000) such as Rheumatoid arthritis, Asthma, MS, Type I Diabetes, Lupus, Psoriasis and Inflammatory Bowel Disease; Cystic fibrosis (Fuller, 2000); Angiogenesis-related diseases (Hur, 2002 and Kurebayashi, 2001): diabetic retinopathy, haemangiomas, psoriasis, endometriosis and tumour angiogenesis. Also an Hsp90 inhibitor of the invention may protect normal cells against chemotherapy-induced toxicity and be useful in diseases where failure to undergo apoptosis is an underlying factor. Such an Hsp90 inhibitor may also be useful in diseases where the induction of a cell stress or heat shock protein response could be beneficial, for example, protection from hypoxia-ischemic injury due to elevation of Hsp70 in the heart (Hutter, 1996 and Trost, 1998) and brain (Plumier, 1997 and Rajder, 2000). An Hsp90 inhibitor - induced increase in Hsp70 levels could also be useful in diseases where protein misfolding or aggregation is a major causal factor, for example, neurogenerative disorders such as scrapie/CJD, Huntingdon's and Alzheimer's (Sittler, 2001; Trazelt, 1995 and Winklhofer, 2001)".

Accordingly, the invention also includes:
(i) A pharmaceutical or veterinary composition comprising a compound of formula (I) above, together with a pharmaceutically or veterinarily acceptable carrier.
(ii) The use of a compound a compound of formula (I) above in the preparation of a composition for composition for inhibition of HSP90 activity in vitro or in vivo.
(iii) A method of treatment of diseases or conditions which are responsive to inhibition of HSP90 activity in mammals which method comprises administering to the mammal an amount of a compound of formula (I) above effective to inhibit said HSP90 activity.

It will be understood that the specific dose level for any particular patient will depend upon a variety of factors including the activity of the specific compound employed, the age, body weight, general health, sex, diet, time of administration, route of administration, rate of excretion, drug combination and the causative mechanism and severity of the particular disease undergoing therapy. In general, a suitable dose for orally administrable formulations will usually be in the range of 0.1 to 3000 mg, once, twice or three times per day, or the equivalent daily amount administered by infusion or other routes. However, optimum dose levels and frequency of dosing will be determined by clinical trials as is conventional in the art.

The compounds with which the invention is concerned may be prepared for administration by any route consistent with their pharmacokinetic properties. The orally administrable compositions may be in the form of tablets, capsules, powders, granules, lozenges, liquid or gel preparations, such as oral, topical, or sterile parenteral solutions or suspensions. Tablets and capsules for oral administration may be in unit dose presentation form, and may contain conventional excipients such as binding agents, for example syrup, acacia, gelatin, sorbitol, tragacanth, or polyvinyl-pyrrolidone; fillers for example lactose, sugar, maize-starch, calcium phosphate, sorbitol or glycine; tabletting lubricant, for example magnesium stearate, talc, polyethylene glycol or silica; disintegrants for example potato starch, or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, syrup, methyl cellulose, glucose syrup, gelatin hydrogenated edible fats; emulsifying agents, for example lecithin, sorbitan monooleate, or acacia; non-aqueous vehicles (which may include edible oils), for example almond oil, fractionated coconut oil, oily esters such as glycerine, propylene glycol, or ethyl alcohol; preservatives, for example methyl or propyl p-hydroxybenzoate or sorbic acid, and if desired conventional flavouring or colouring agents.

For topical application to the skin, the drug may be made up into a cream, lotion or ointment. Cream or ointment formulations which may be used for the drug are conventional formulations well known in the art, for example as described in standard textbooks of pharmaceutics such as the British Pharmacopoeia.

The active ingredient may also be administered parenterally in a sterile medium. Depending on the vehicle and concentration used, the drug can either be suspended or dissolved in the vehicle. Advantageously, adjuvants such as a local anaesthetic, preservative and buffering agents can be dissolved in the vehicle.

Compounds of the invention may be administered together with other classes opf pharmaceutically active drugs. For example, for the treatment of cancers, combination therapy with two or more different classes of anticancer agent is a recognised and widespread practice. The present compounds may be used in such combination therapy, particularly where the other drug(s) have a mode of action different from HSP90 inhibition.

The following examples illustrate the preparation and activities of specific compounds of the invention and are not intended to be limiting of the full scope of the invention.

### General Procedures

All reagents obtained from commercial sources were used without further purification. Anhydrous solvents were obtained from commercial sources and used without further drying. Flash chromatography was performed with pre-packed silica gel cartridges (Strata SI-1; 61Å, Phenomenex, Cheshire UK or IST Flash II, 54Å, Argonaut, Hengoed, UK). Thin layer chromatography was conducted with 5 x 10 cm plates coated with Merck Type 60 F₂₅₄ silica gel.

The compounds of the present invention were characterized by LC/MS using a Hewlett Packard 1100 series LC/MSD linked to quadripole detector (ionization mode: electron spray positive or negative; column: Phenomenex Luna 3u C18(2) 30 x 4.6 mm; Buffer A prepared by dissolving 1.93g ammonium acetate in 2.5 L HPLC grade H₂O and adding 2 mL formic acid. Buffer B prepared by adding 132 mL buffer A to 2.5 L of HPLC grade acetonitrile and adding 2 mL formic acid; elution gradient 95:5 to 5:95 buffer A : buffer B over 3.75 minutes or 7.5 minutes. Flow rate = 2.0 mL/min) Retention Times (RT) are reported in minutes. Ionisation is positive unless otherwise stated.

Nuclear magnetic resonance (NMR) analysis was performed with a Brucker DPX-400 MHz NMR spectrometer. The spectral reference was the known chemical shift of the solvent. Proton NMR data is reported as follows: chemical shift (δ) in ppm, multiplicity (s = singlet, d = doublet, t = triplet, q = quartet, p = pentet, m = multiplet, dd = doublet of doublet, br = broad), integration, coupling constant.

Some compounds of the invention were purified by preparative HPLC. Preparative HPLC purifications were performed on a Waters FractionLynx MS Autopurification system with a Gemini^{®} 5 µM C18(2), 100 mm x 20 mm i.d. column from Phenomenex, running at a flow rate of 20 mL min⁻¹ with UV diode array detection (210 - 400 nm) and mass-directed collection. Gradients used for each compound are shown in Table 1.
**At pH 4**:Solvent A: HPLC grade Water + 10mM ammonium acetate + 0.08% v/v formic acid.
Solvent B: 95% v/v HPLC grade acetonitrile + 5% v/v Solvent A + 0.08% v/v formic acid.
**At pH 9**:Solvent A: HPLC grade Water + 10 mM ammonium acetate + 0.08% v/v ammonia solution.
Solvent B: 95% v/v HPLC grade acetonitrile + 5% v/v Solvent A + 0.08% v/v ammonia solution..

The mass spectrometer was a Waters Micromass ZQ2000 spectrometer operating in positive or negative ion electrospray ionisation modes, with a molecular weight scan range of 150 to 1000.

**Table 1 Preparative HPLC gradients**

| Time /min | %B for Compound no. | | | |
|---|---|---|---|---|
| | **8** | **9** | **11** | **12** |
| 0.0 | 5 | 5 | 5 | 5 |
| 0.5 | 20 | 25 | 30 | 35 |
| 7.0 | 40 | 45 | 50 | 55 |
| 7.5 | 95 | 95 | 95 | 95 |
| 9.5 | 95 | 95 | 95 | 95 |
| 10 | 5 | 5 | 5 | 5 |

IUPAC chemical names were generated using AutoNom Standard.

Some compounds of the invention can be made (by way of example) by a route typified by in scheme 1 (PG = protecting group).

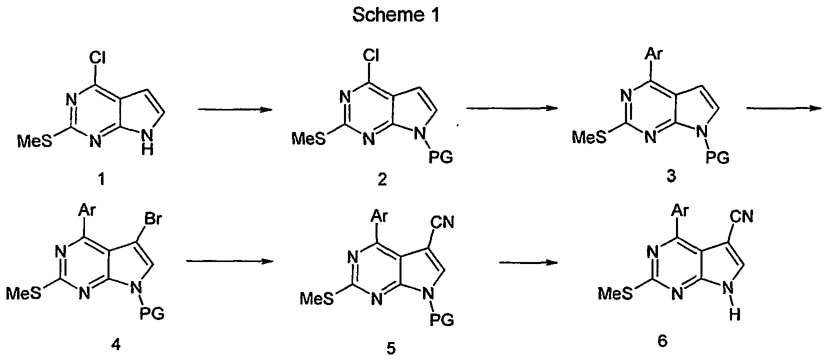

Some compounds of the invention can be made by the route typified by scheme 2 (PG = protecting group).

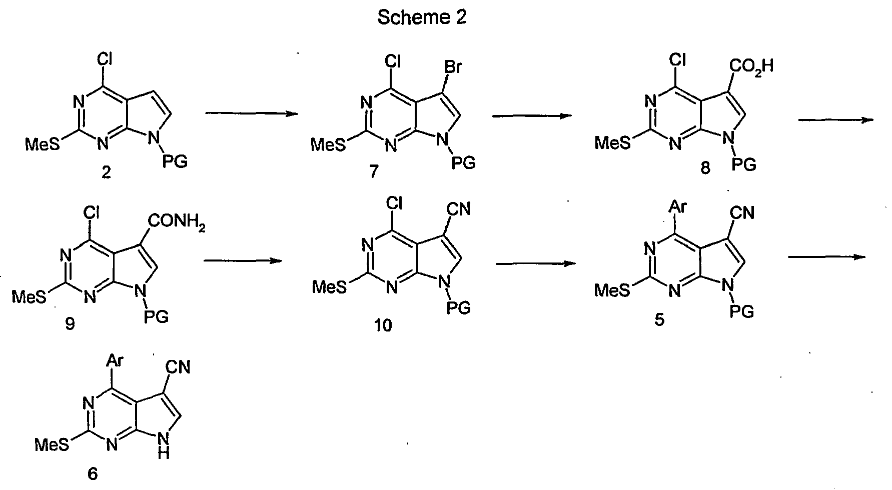

Some compounds of the invention can be made by the route typified by scheme 3 (PG = protecting group).

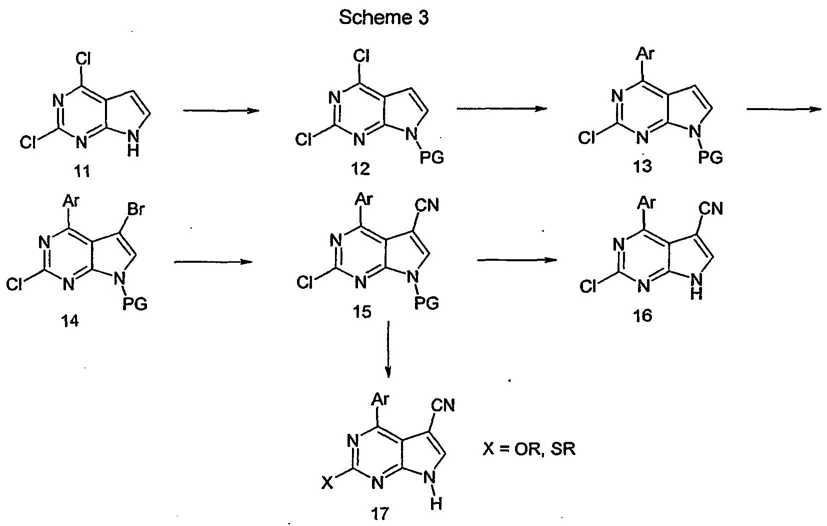

An alternative synthetic route to synthesise compounds such as 17 is shown in Scheme 4. This involves displacement of sulphones (18) by appropriate nucleophiles using methods and reagents known to those skilled in the art.

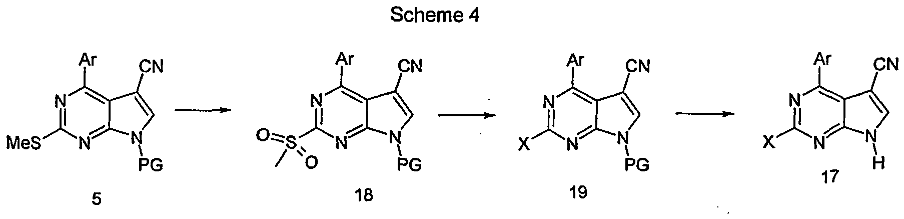

The Aryl group ("Ar" in schemes 1-4) can be manipulated further to create more examples of the invention, as outlined in scheme 5 (PG = protecting group).

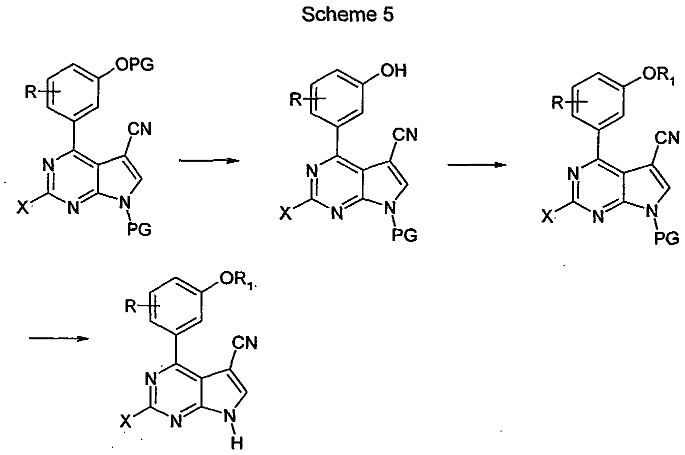

### Example 1

### 4-(2,4-dimethyl-phenyl)-2-methylsulfanyl-7H-pyrrolo[2,3-d]pyrimidine-5-carbonitrile

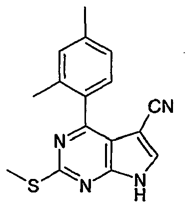

### Step 1

### 4-chloro-2-methylsulfanyl-7-(2-trimethylsilanyl-ethoxymethyl)-7H-pyrrolo[2,3-d]pyrimidine

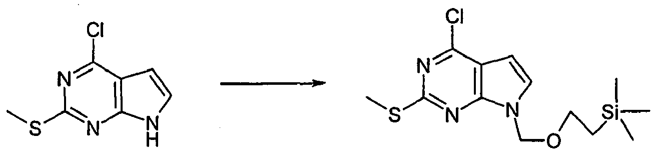

To a mixture of sodium hydride (276 mg; 6.89 mmol) in DMF (10ml) at 0°C was added drop-wise a solution of 4-chloro-2-methylsulfanyl-7H-pyrrolo[2,3-d]pyrimidine [prepared as detailed in Davoll. J. J. Chem. Soc. 1960, pp131-138] (1.145 g; 5.74 mmol) in anhydrous DMF (20 ml). When addition was complete, 2-(trimethylsilyl)ethoxymethyl chloride (1.32 ml; 7.46 mmol) was added drop-wise and the reaction mixture was stirred at 0°C for 1.5 hours then allowed to warm to ambient temperature. The reaction mixture was partitioned between water (100 ml) and ethyl acetate (100 ml). The organic phase was dried over Na₂SO₄ then filtered and filtrate solvents evaporated in vacuo. The crude product was purified by flash chromatography on silica gel (70g) eluting with a solvent gradient of 0 to 5% ethyl acetate in hexane to afford product as colourless oil (2.04g).
LC/MS: RT = 2.88 min; *m*/*z* = 332, 330 [M+H]⁺. Total run time 3.75 mins.

### Step2

### 4-(2,4-dimethyl-phenyl)-2-methylsulfanyl-7-(2-trimethylsilanyl-ethoxymethyl)-7H-pyrrolo[2,3-d]pyrimidine

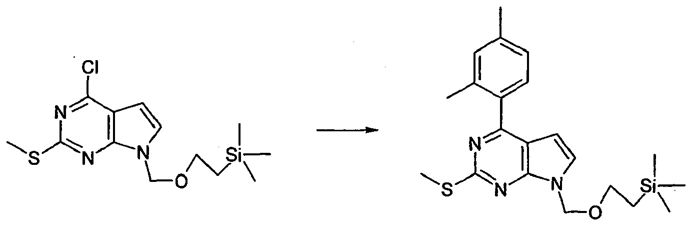

A mixture of 4-chloro-2-methylsulfanyl-7-(2-trimethylsilanyl-ethoxymethyl)-7H-pyrrolo[2,3-d]pyrimidine (2.04 g; 6.19 mmol), 1N sodium hydrogen carbonate (aq) 18.6 ml; 18.6 mmol), DMF (41 ml) and 2,4-dimethylphenylboronic acid was degassed by bubbling nitrogen through reaction mixture for 5 minutes. Dichlorobis(triphenylphosphine) palladium(II) (217 mg; 0.309 mmol) was added and reaction mixture was heated to 80°C for 2.25 hours under nitrogen atmosphere. Reaction mixture was allowed to cool to ambient temperature and then filtered through a pad of celite. The filter cake was washed with methanol and ethyl acetate and combined filtrate solvents were removed in vacuo and the residue partitioned between ethyl acetate (100ml) and sat. sodium chloride (aq) solution (100 ml). The organic phase was dried over Na₂SO₄ then filtered and filtrate solvents evaporated in vacuo. The crude product was purified by flash chromatography on silica gel (50g) eluting with a solvent gradient of 0 to 10% ethyl acetate in hexane to afford product as a yellow oil, (2.01 g).
LC/MS: RT = 3.06 min; *m*/*z* = 400 [M+H]⁺. Total run time 3.75 mins.

### Step 3

### 5-Bromo-4-(2,4-dimethyl-phenyl)-2-methylsulfanyl-7-(2-trimethylsilanyl-ethoxymethyl)-7H-pyrrolo[2,3-d]pyrimidine

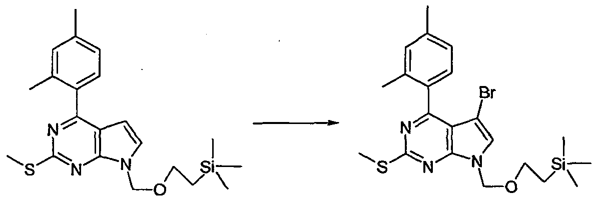

To a solution of 4-(2,4-dimethyl-phenyl)-2-methylsulfanyl-7-(2-trimethylsilanyl-ethoxymethyl)-7H-pyrrolo[2,3-d]pyrimidine (step 2) (100mg, 0.25 mmol) in CH₂Cl₂ (3 ml) at 0°C was added dropwise a solution of N-Bromosuccinimide in CH₂Cl₂ (45 mg, 0.25 mmol). After 5 minutes the reaction was allowed to warm to ambient temperature. The solution was evaporated *in vacuo* and the residue was partitioned between EtOAc (2 x 20 ml) and sat. aqueous sodium thiosulfate solution (20 ml). The combined organics were passed through a hydrophobic frit and evaporated *in vacuo.* The crude was applied to a column of SiO₂ (20 g) and eluted with Hexane - 5% EtOAc/Hexane (gradient) to afford the title compound as a colourless oil, 100 mg, 84%.
LC/MS: RT = 5.92 min; *m*/*z* = 480, 478 [M+H]⁺. Total run time 7.5 mins.

### Step 4

### 4-(2,4-dimethyl-phenyl)-2-methylsulfanyl-7-(2-trimethylsilanyl-ethoxymethyl)-7H-pyrrolo[2,3-d]pyrimidine-5-carbonitrile

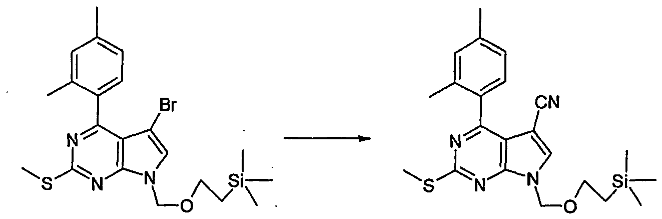

5-Bromo-4-(2,4-dimethyl-phenyl)-2-methylsulfanyl-7-(2-trimethylsilanyl-ethoxymethyl)-7H-pyrrolo[2,3-d]pyrimidine (90 mg, 0.188 mmol), CuCN (67 mg, 0.753 mmol), dppf (17 mg, 0.03 mmol), Pd₂(dba)₃ (7 mg, 0.04 mmol) and 1,4-dioxane (1.5 ml) were combined and then heated at 100°C overnight. The reaction had not gone to completion so further equivalents of CuCN, dppf and Pd₂(dba)₃ were added and the reaction heated for a further 2 h. The reaction mixture was allowed to cool to ambient temperature, and partitioned between EtOAc (2 x 20 ml) and sat. NaHCO₃ solution (20 ml). The combined organics were passed through a hydrophobic frit and evaporated *in vacuo* to give a crude solid (100 mg). The crude product was purified by flash chromatography on SiO₂ (20g) eluting with Hexane to 10% EtOAc/Hexane (gradient) to the title compound, 10 mg, 13 %.
LC/MS: RT = 2.94 min; = *m*/*z* = 425 [M+H]⁺. Total run time 3.75 mins.

### Step 5

### 4-(2,4-dimethyl-phenyl)-2-methylsulfanyl-7H-pyrrolo[2,3-d]pyrimidine-5-carbonitrile

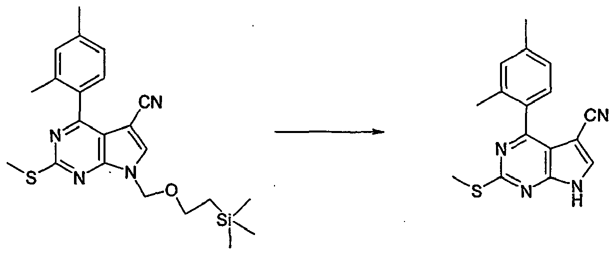

To a solution of 4-(2,4-dimethyl-phenyl)-2-methylsulfanyl-7-(2-trimethylsilanyl-ethoxymethyl)-7H-pyrrolo[2,3-d]pyrimidine-5-carbonitrile (10 mg, 0.024 mmol) in THF (0.4 ml), were added sequentially, ethylenediamine (0.005 ml, 0.071 mmol) and TBAF (1 M in THF, 0.15 ml, 0.142 mmol). The reaction mixture was heated at 50°C overnight. The reaction was allowed to cool to ambient temperature and was then partitioned between EtOAc (2 x 10 ml) and water (10 ml). The combined organics were passed through a hydrophobic frit and evaporated *in vacuo.* The resultant crude product was purified by flash chromatography on SiO₂ (5g) eluting with Hexane-40% EtOAc/Hexane (gradient) to afford the desired product as a solid, 5 mg, 72 %.
LC/MS: RT=2.44 Min; *m*/*z* = 295 [M+H]⁺. Total run time 3.75 mins.
¹H NMR (CD₃OD): δ 2.26(s, 3H); 2.43 (s, 3H); 2.65 (s, 3H);
7.19 (d, 1H, J=7.7 Hz); 7.23 (s, 1H); 7.30 (d, 1H, J=7.7 Hz); 8.11 (s, 1H) NH not observed.
This compound had activity 'A' in the fluorescence polarization assay described below.

### Example 2

### (2,4-dimethyl-phenyl)-2-methylsulfanyl-7H-pyrrolo[2,3-d]pyrimidine-5-carbonitrile

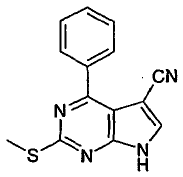

### Step 1

### 5-Bromo-4-chloro-2-methylsulfanyl-7-(2-trimethylsilanyl-ethoxymethyl)-7H-pyrrolo[2,3-d]pyrimidine

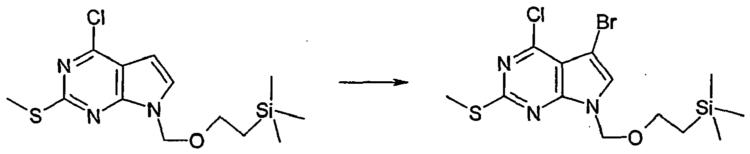

To a solution of 4-chloro-2-methylsulfanyl-7-(2-trimethylsilanyl-ethoxymethyl)-7H-pyrrolo[2,3-d]pyrimidine (0.5 g, 1.516 mmol) (example 1 step 2) in DMF (14 ml) at 0°C was added dropwise a solution of N-bromosuccinimide (270 mg, 1.516 mmol) in DMF (6 ml). After 5 minutes the reaction was allowed to warm to ambient temperature. The solution was partitioned between EtOAc (2 x 40ml) and water (40 ml). The combined organics were passed through a hydrophobic frit and evaporated *in vacuo.* The crude product was purified by flash chromatography on SiO₂ (50 g) eluting with Hexane - 5% EtOAc/Hexane (gradient) to afford the desired product as a white solid, 433 mg, 70%.
LC/MS: RT = 3.112 min; *m*/*z* = 410,408 [M+H]⁺. Total run time 3.75 mins

### Step 2

### 4-chloro-2-methylsulfanyl-7-(2-trimethylsilanyl-ethoxymethyl)-7H-pyrrolo[2,3-d]pyrimidine-5-carboxylic acid

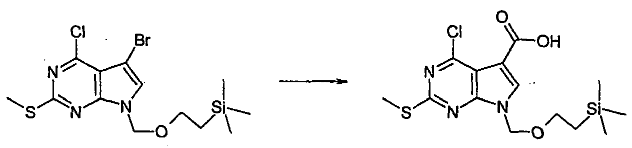

To a solution of n-butyl lithium (2.5M in hexanes, 0.24 ml, 0.59 mmol) in THF (0.5 ml) at 0°C was added slowly dropwise a solution of 5-Bromo-4-chloro-2-methylsulfanyl-7-(2-trimethylsilanyl-ethoxymethyl)-7H-pyrrolo[2,3-d]pyrimidine (200mg, 0.489 mmol) in THF (2 ml). After 2 minutes, crushed solid CO₂ was added and the mixture was left to warm to ambient temperature. Acetic acid was added then water (20 ml) and the mixture extracted with EtOAc (2 x 20 ml). The combined organics were passed through a hydrophobic frit and evaporated *in vacuo* to afford the desired crude product as a white solid, 167 mg, 91%.
LC/MS: RT=2.664 min; *m*/*z* = 374 [M+H]⁺. Total run time 3.75 mins

### Step 3

### 4-Chloro-2-methylsulfanyl-7-(2-trimethylsilanyl-ethoxymethyl)-7H-pyrrolo[2,3-d]pyrimidine-5-carboxylic acid amide

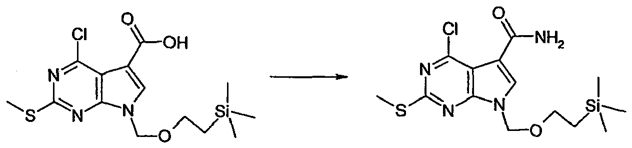

To a solution of 4-chloro-2-methylsulfanyl-7-(2-trimethylsilanyl-ethoxymethyl)-7H-pyrrolo[2,3-d]pyrimidine-5-carboxylic acid (100mg, 0.268 mmol) in CH₂Cl₂ (1.5 ml) was added oxalyl chloride (2M in CH₂Cl₂, 0.17 ml, 0.349 mmol) followed by a few drops of DMF. After 10 min the reaction mixture was evaporated *in vacuo* then re-dissolved in CH₂Cl₂ (3 ml). Aqueous ammonia solution (2 ml) was added and the mixture was stirred vigorously for 15 minutes. Water (10 ml), and CH₂Cl₂ (10 ml) were added and the resultant phases separated. The aqueous phase was extracted with further CH₂Cl₂ (15 ml). The combined organics were passed through a hydrophobic frit and evaporated *in vacuo.* The crude product was applied to a column of SiO₂ (20 g) eluting with CH₂Cl₂ - 5% MeOH/CH₂Cl₂ (gradient) to afford the title compound as a yellow solid, 77 mg, 77%.
LC/MS: RT = 2.47 min; *m*/*z* = 373, 375 [M+H]⁺. Total run time 3.75 mins.

### Step 4

### 4-chloro-2-methylsulfanyl-7-(2-trimethylsilanyl-ethoxymethyl)-7H-pyrrolo[2,3-d]pyrimidine-5-carbonitrile

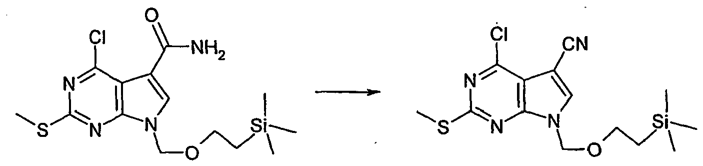

To a solution of 4-chloro-2-methylsulfanyl-7-(2-trimethylsilanyl-ethoxymethyl)-7H-pyrrolo[2,3-d]pyrimidine-5-carboxylic acid amide (73 mg, 0.196 mmol) in CH₂Cl₂ at 0°C was added Et₃N followed by TFAA (0.03 ml, 0.21 mmol) slowly dropwise. The stirred reaction mixture was the allowed to warm to ambient temperature. Further CH₂Cl₂ (5 ml) was then added and the organic phase was washed with sat. NaHCO₃ solution (15 ml). The organic layer was passed through a hydrophobic frit and evaporated *in vacuo.* The crude product was purified by flash chromatography on SiO₂ (20 g) eluting with Hexane - 20% EtOAc/Hexane (gradient) to afford the title compound as a white solid, 60 mg, 86%.
LC/MS: RT = 2.84 min; *m*/*z* = 357, 355 [M+H]⁺. Total run time 3.75 mins.

### Step 5

### 2-methylsulfanyl-4-phenyl-7-(2-trimethylsilanyl-ethoxymethyl)-7H-pyrrolo[2,3-d]pyrimidine-5-carbonitrile

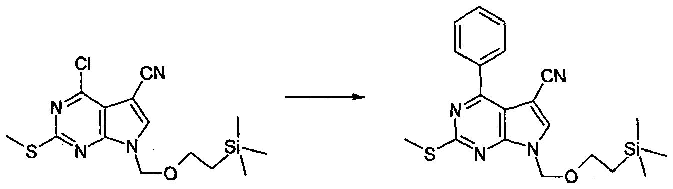

A mixture of 4-chloro-2-methylsulfanyl-7-(2-trimethylsilanyl-ethoxymethyl)-7H-pyrrolo[2,3-d]pyrimidine-5-carbonitrile (54 mg, 0.152 mmol), phenylboronic acid (24 mg, 0.198 mmol), Pd₂Cl₂(PPh₃)₂ (5 mg, 0.0076 mmol), NaHCO₃ aqueous solution (1M, 0.46 ml, 0.456 mmol) and DMF was degassed by bubbling N₂ through the mixture for 5 min. The reaction was then heated under a nitrogen atmosphere at 80°C for 3 h. The mixture was allowed to cool and was then partitioned between EtOAc (2 x 15 ml) and brine (15 ml). The combined organics were passed through a hydrophobic frit and evaporated *in vacuo*. The crude product was purified by flash chromatography on SiO₂ (20 g) eluting with Hexane - 20% EtOAc/Hexane (gradient) to afford the desired product as a white solid, 50 mg, 83%.
LC/MS: RT = 2.912 min; *m*/*z* = 397 [M+H]⁺. Total run time 3.75 mins.

### Step 6

### 2-methylsulfanyl-4-phenyl-7H-pyrrolo[2,3-d]pyrimidine-5-carbonitrile

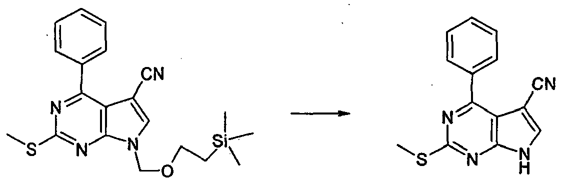

To a solution of 2-methylsulfanyl-4-phenyl-7-(2-trimethylsilanyl-ethoxymethyl)-7H-pyrrolo[2,3-d]pyrimidine-5-carbonitrile (50 mg, 0.126 mmol) in THF (1 ml) was added ethylenediamine (0.025 ml, 0.378 mmol) followed by tetrabutylamonium fluoride (1 M solution in THF, 0.76 ml, 0.756 mmol). The reaction mixture was heated at 50°C overnight. The reaction was allowed to cool to ambient temperature and was then partitioned between EtOAc (2x15 ml) and water (15 ml). The combined organics were passed through a hydrophobic frit and evaporated *in vacuo.* The resultant crude product was purified by flash chromatography on SiO₂ (20g) eluting with 10% EtOAc/Hexane - 40% EtOAc/Hexane (gradient) to afford the title compound as a white solid, 17 mg, 51 %.
LC/MS: RT = 2.313 min; *m*/*z* = 267 [M+H]⁺. Total run time 3.75 mins
¹H NMR (d₆ DMSO): δ 2.60 (s, 3H); 7.5-7.6 (m, 3H); 7.8-7.9 (m, 2H); 8.50 (s, 1H); 13.21, (s, 1H).
This compound had activity 'A' in the fluorescence polarization assay described below.

### Example 3

### 4-(4-cyano-phenyl)-2-methylsulfanyl-7H-pyrrolo[2,3-d]pyrimidine-5-carbonitrile

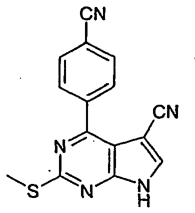

The title compound was prepared by the route outlined in scheme 2 and by way of the methods of example 2, using 4-cyanophenyl boronic acid in the appropriate step. LC/MS: RT = 3.56 min; *m*/*z* = 290 [M-H]⁻ (negative ionisation). Total run time 7.5 mins. ¹H NMR (d₆ DMSO): δ 2.61 (s, 3H); 8.05 (d, 1H, J=8.2 Hz), 8.07 (d,1H, J=8.2Hz); 8.56 (s, 1H);13.32 (brs, 1H).
This compound had activity 'A' in the fluorescence polarization assay described below.

### Example 4

### 4-(2-methyl-4-fluoro-phenyl)-2-methylsulfanyl-7H-pyrrolo[2,3-d]pyrimidine-5-carbonitrile

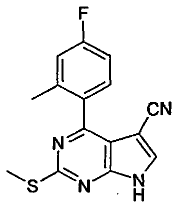

### Step 1

### 4-[(2-methyl-4-fluoro-phenyl]-2-methylsulfanyl-7-(2-trimethylsilanyl-ethoxymethyl)-7H-pyrrolo[2,3-d]pyrimidine-5-carbonitrile

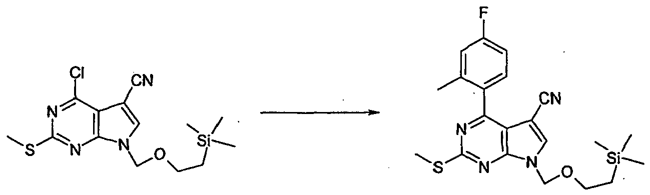

The title compound was prepared by the route outlined in scheme 2 and by way of the methods of example 2, using 2-methyl-4-fluorophenyl boronic acid and 4-chloro-2-methylsulfanyl-7-(2-trimethylsilanyl-ethoxymethyl)-7H-pyrrolo[2,3-d]pyrimidine-5-carbonitrile in the appropriate step (cross coupling)
LC/MS: RT = 2.89 min; *m*/*z* = 429 [M+H]⁺. Total run time 3.75 mins

### Step 2

### 4-(2-methyl-4-fluoro-phenyl)-2-methylsulfanyl-7H-pyrrolo[2,3-d]pyrimidine-5-carbonitrile

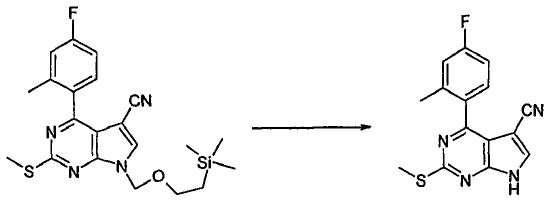

The title compound was made by way of the method of example 1 step 5 (TBAF mediated SEM deprotection). The crude product was purified by flash chromatography on silica gel, eluting with ethyl acetate and hexane mixture to afford an off white solid. LC/MS: RT = 2.40 min; *m*/*z* = 299 [M+H]⁺. Total run time 3.75 mins.
¹H NMR (d₆ DMSO): δ, 2.22 (s, 3H); 2.57 (s, 3H); 7.1-7.2 (m, 1H); 7.26 (dd, 1H, J=10.1, 2.2 Hz), 7.46 (dd,1H, J=8.6, 6.1 Hz); 8.44 (s, 1H); 13.19 (brs,1H).
This compound had activity 'A' in the fluorescence polarization assay described below.

### Example 5

### 5-Bromo-4-(2,4-dimethyl-phenyl)-2-methylsulfanyl-7H-pyrrolo[2,3-d]pyrimidine

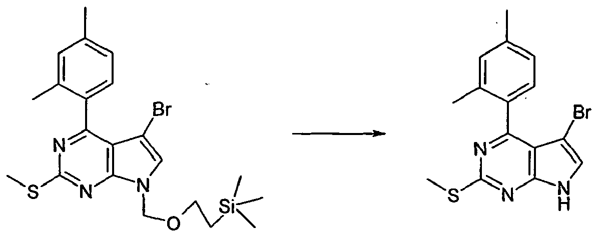

The title compound was prepared by treating 5-Bromo-4-(2,4-dimethyl-phenyl)-2-methylsulfanyl-7-(2-trimethylsilanyl-ethoxymethyl)-7H-pyrrolo[2,3-d]pyrimidine (example 1, step 3) with tetra butylammonium fluoride using the method outlined in example 1 step 5. Purification was by flash chromatography on silica gel eluting with Ethyl acetate /hexane mixture..
LC/MS: RT = 4.44 Min; *m*/*z* = 350, 348 [M+H]⁺. Total run time 7.5 mins.
¹H NMR (d₆ DMSO): δ 2.06 (s, 3H); 2.35 (s, 3H); 2.57 (s, 3H); 7.08-7.20 (m, 3H), 7.62 (s, 1H); 12.48 (s, 1H).
This compound had activity 'B' in the fluorescence polarization assay described below.

### Example 6

### 4-(2,4-dimethyl-phenyl)-5-methyl-2-methylsulfanyl-7H-pyrrolo[2,3-d]pyrimidine

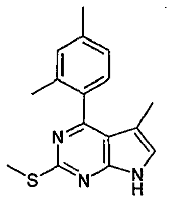

### Step 1

### 4-(2,4-dimethyl-phenyl)-5-methyl-2-methylsulfanyl-7-(2-trimethylsilanyl-ethoxymethyl)-7H-pyrrolo[2,3-d]pyrimidine

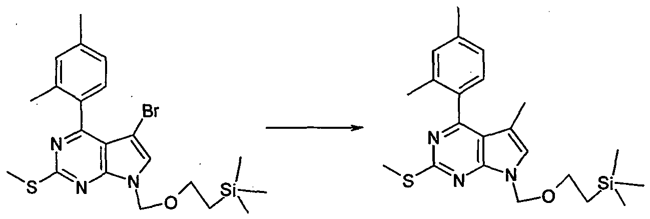

To a solution of n-Butyl lithium (2.5M; 0.10ml; 0.253 mmol) in anhydrous THF (2 ml) cooled in CO₂-acetone bath under a nitrogen atmosphere was added a solution of 5-bromo-4-(2,4-dimethyl-phenyl)-2-methylsulfanyl-7-(2-trimethylsilanyl-ethoxymethyl)-7H-pyrrolo[2,3-d]pyrimidine (110 mg; 0.23 mmol) in anhydrous THF (1.4 ml) drop-wise. When addition was complete, methyl iodide (72 µL; 1.15 mmol) was added and the reaction mixture stirred for 5 minutes, cooling bath was removed and reaction mixture allowed to warm to ambient temperature. The reaction mixture was partitioned between sat. NH₄Cl (aq) solution and ethyl acetate. The organic phase was passed through a hydrophobic frit and solvents removed in vacuo to give a oil which was purified by flash chromatography eluting 0 to 10% gradient of ethyl acetate in hexane affording product as a colourless oil (80 mg; 84%).
LC/MS: RT = 3.08 min; *m*/*z* = 414 [M+H]⁺. Total run time 3.75 mins.

### Step 2

### 4-(2,4-dimethyl-phenyl)-5-methyl-2-methylsulfanyl-7H-pyrrolo[2,3-d]pyrimidine

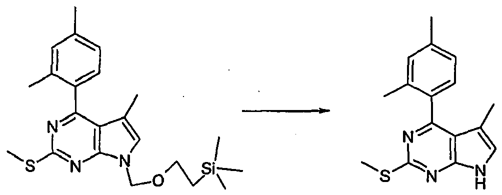

The title compound was prepared by treating 4-(2,4-dimethyl-phenyl)-5-methyl-2-methylsulfanyl-7-(2-trimethylsilanyl-ethoxymethyl)-7H-pyrrolo[2,3-d]pyrimidine with tetrabutylammonium fluoride using the method outlined in example 1 step 5. Purification was by flash chromatography on silica gel eluting with ethyl acetate / hexane mixture; followed by trituration with diethyl ether to afford title compound as a colourless solid.
LC/MS: RT = 2.63 Min; *m*/*z* = 284 [M+H]⁺. Total run time 3.75 mins.
¹H NMR (d₆ DMSO): δ 1.68 (s, 3H); 2.05 (s, 3H); 2.35 (s, 3H); 2.52 (s, 3H); 7.08-7.12 (m, 4H); 11.75 (s, 1H).
This compound had activity 'B' in the fluorescence polarization assay described below.

### Example 7

### 4-[(3-(2-Diethylamino-ethoxy)-phenyl]-2-methylsulfanyl-7H-pyrrolo[2,3-d]pyrimidine-5-carbonitrile

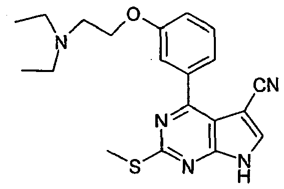

### Step1

### 4-[(3-Hydroxy-phenyl]-2-methylsulfanyl-7-(2-trimethylsilanyl-ethoxymethyl)-7H-pyrrolo[2,3-d]pyrimidine-5-carbonitrile

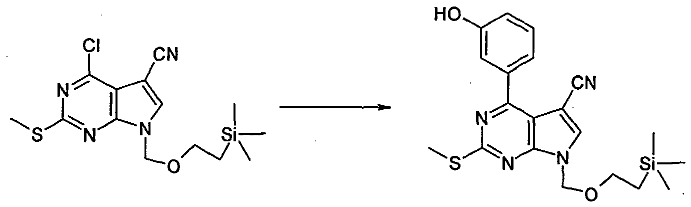

The title compound was prepared by the route outlined in scheme 2 and by way of the methods of example 2, using 3-hydroxyphenyl boronic acid and 4-chloro-2-methylsulfanyl-7-(2-trimethylsilanyl-ethoxymethyl)-7H-pyrrolo[2,3-d]pyrimidine-5-carbonitrile in the appropriate step (cross coupling).
LC/MS RT = 2.746 min; *m*/*z* = 413 [M+H]⁺. Total run time 3.75 mins.

### Step 2

### 4-[(3-(2-Diethylamino-ethoxy)-phenyl]-2-methylsulfanyl-7-(2-trimethylsilanyl-ethoxymethyl)-7H-pyrrolo[2,3-d]pyrimidine-5-carbonitrile

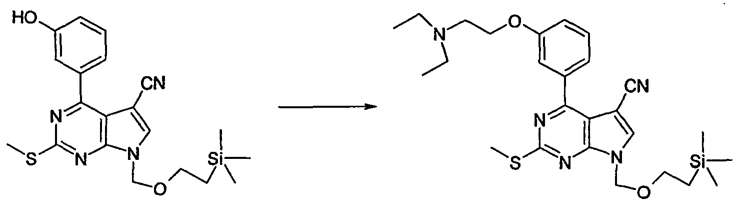

Cesium carbonate (73 mg; 0.225 mmol) was added to a solution of 4-[(3-Hydroxyphenyl]-2-methylsulfanyl-7H-pyrrolo[2,3-d]pyrimidine-5-carbonitrile (37 mg; 0.09 mmol) in DMF (1.5 ml), 2-Bromo-N,N-diethylethylamine hydrobromide (26 mg; 0.1 mmol) was added followed by a catalytic amount of KI and the suspension heated , at 110°C, for 18hrs. The resulting suspension was allowed to cool and partitioned between ethyl acetate and aqueous ammonia. The phases were separated, poured through a hydrophobic frit and the crude product was purified by chromatography on silica gel eluting with mixtures of dichloromethane and methanol (0 to 15% gradient of Methanol in dichloromethane), to afford product as a yellow solid 28 mg; 61%.
LC/MS: RT=2.243 min; *m*/*z* = 512 [M+H]⁺. Total run time 3.75 mins.

### Step 3

### 4-[(3-(2-Diethylamino-ethoxy)-phenyl]-2-methylsulfanyl-7H-pyrrolo[2,3-d]pyrimidine-5-carbonitrile

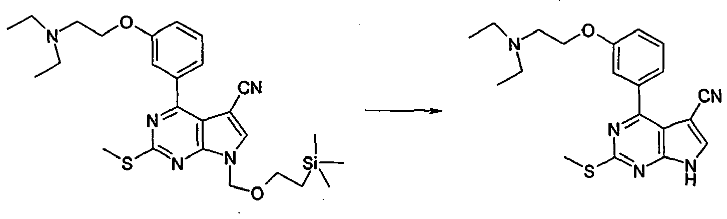

The title compound was prepared by reacting 4-[(3-(2-Diethylamino-ethoxy)-phenyl]-2-methylsulfanyl-7-(2-trimethylsilanyl-ethoxymethyl)-7H-pyrrolo[2, 3-d]pyrimidine-5-carbonitrille with tetrabutylammonium fluoride and ethylene diamine in THF, using the method outlined in example 1 step 5. Purification was by flash chromatography on silica gel eluting with gradient 1% triethylamine in dichloromethane to 1% triethylamine; 15% methanol; 84% dichloromethane to afford title compound as a pale yellow solid.
LC/MS: RT = 1.69 Min; *m*/*z* = 382 [M+H]⁺. Total run time 3.75 mins.
¹H NMR (d₆ DMSO): δ 0.99 (t, 6H, J=7.1Hz); 2.56-2.65 (m, 4H), 2.60 (s, 3H); 2.86 (t, 2H, J=5.8Hz); 4.18 (t, 2H, J=5.9Hz); 7.14 (d, 1H, J=7.9Hz); 7.38-7.50 (m, 3H); 8.49 (s, 1H); 12.91 (brs; 1H).
This compound had activity 'B' in the fluorescence polarization assay described below.

### Example 8

### 2-Chloro-4-(2,4-dimethyl-phenyl)-7H-pyrrolo[2,3-d]pyrimidine-5-carbonitrile

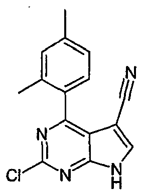

### Step1

### 6-Amino-5-(2,2-diethoxyethyl)-pyrimidine-2,4-diol

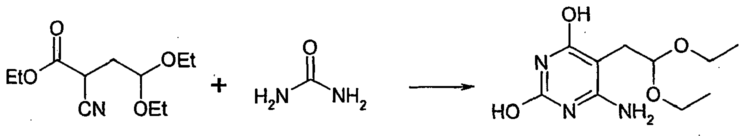

To a solution of urea (5.24 g; 87.2 mmol) in anhydrous ethanol (200 ml), under a nitrogen atmosphere, was added 2-cyano-4,4-diethoxy butyric acid ethyl ester [prepared as detailed in Davoll. J. J. Chem. Soc., 1960, pip131-138] (20 g; 87.2 mmol) followed by sodium ethoxide (11.88 g; 172.6 mmol). The reaction mixture was heated at reflux overnight. The reaction was allowed to cool to ambient temperature and then water (500 ml) and acetic acid (5 ml) were added. The solution was cooled to approximately 5 °C and a pale brown solid formed which was collected by filtration (8.4 g; 40%).
LC/MS: RT = 1.37 min; *m*/*z* = 198 [M -EtOH]⁺. Total run time 3.75 mins
¹H NMR (d₆ DMSO): δ 1.07 (t, 3H); 2.40 (d, 2H); 3.39 (m, 2H); 3.60 (m, 2H); 4.45 (t, 1H); 10.08 (s, 1H); 10.8 (br s, 1H).

### Step2

### 7H-Pyrrolo[2,3-d]pyrimidine-2,4-diol

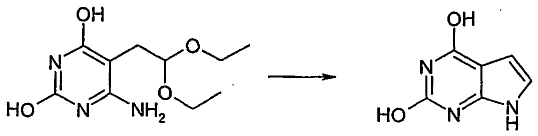

6-Amino-5-(2,2-diethoxyethyl)-pyrimidine-2,4-diol (2.57 g; 10.6 mmol) was stirred in HCl (0.2 M; 80 ml) at ambient temperature for 1.5 h. The suspension was then filtered giving the desired product as a pale brown solid (1.28 g; 80%).
LC/MS: RT = 0.54 min; *m*/*z* = 152 [M+H]⁺. Total run time 3.75 mins

### Step 3

### 2,4-Dichloro-7H-pyrrolo[2,3-d]pyrimidine

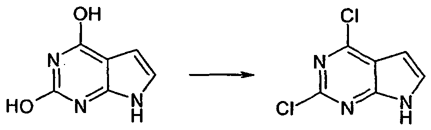

A solution of 7H-Pyrrolo[2,3-d]pyrimidine-2,4-diol (1.28 g; 8.5 mmol) in phenylphosphonic dichloride (7 ml) was heated at 165 °C for 2 h. The hot reaction mixture was then poured slowly onto ice water (150 ml) and extracted with ethyl acetate (2 x 100 ml). The organic extract was washed with water (100 ml) followed by sat. sodium chloride (aq) solution (100 ml). The organic phase was dried over Na₂SO₄ then filtered and filtrate solvents evaporated in vacuo. The crude product was purified by flash chromatography on silica gel (20g) eluting with 75% ethyl acetate in hexane to afford the desired product as a yellow solid, (0.45 g; 28%).
LC/MS: RT = 1.98 min; *m*/*z* = 188 [M+H]⁺. Total run time 3.75 mins

### Step 4

### 2,4-Dichloro-7-(2-trimethylsilanyl-ethoxymethyl)-7H-pyrrolo[2,3-d]pyrimidine

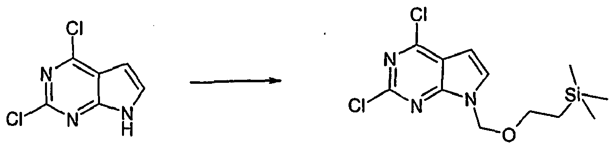

To a mixture of sodium hydride (115 mg; 2.88 mmol) in DMF (4 ml) at 0°C was added drop-wise a solution of 2,4-Dichloro-7H-pyrrolo[2,3-d]pyrimidine (0.45 g; 2.4 mmol) in anhydrous DMF (2 ml). When addition was complete, 2-(trimethylsilyl)ethoxymethyl chloride (0.55 ml; 3.12 mmol) was added drop-wise and the reaction mixture was stirred at 0°C for 1.5 hours then allowed to warm to ambient temperature. The reaction mixture was partitioned between water (50 ml) and ethyl acetate (50 ml). The organic phase was dried over Na₂SO₄ then filtered and filtrate solvents evaporated in vacuo. The crude product was purified by flash chromatography on silica gel (10g) eluting with a solvent of 15% ethyl acetate in hexane to afford product as yellow oil (0.65 g; 85%).
LC/MS: RT = 2.84 min; *m*/*z* = 320, 318 [M+H]⁺. Total run time 3.75 mins.

### Step 5

### 5-Bromo-2,4-dichloro-7-(2-trimethlysilanyl-ethoxymethyl)-7H-pyrrolo[2,3-d]pyrimidine

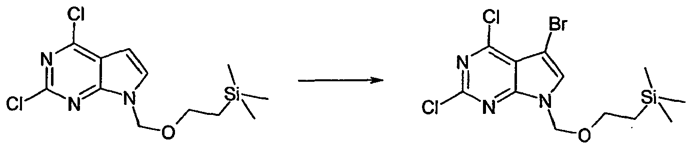

To a solution of 2,4-Dichloro-7-(2-trimethylsilanyl-ethoxymethyl)-7H-pyrrolo[2,3-d]pyrimidine (1.81 g; 5.7 mmol) in DMF (8 ml) at 0°C was added dropwise a solution of N-Bromosuccinimide in DMF (1.02 g; 5.7 mmol). After 1 h the solution was partitioned between EtOAc (100ml) and water (100 ml). The organic extract was washed with water (100 ml) followed by sat. sodium chloride (aq) solution (100 ml). The organic phase was dried over Na₂SO₄ then filtered and the filtrate solvents evaporated in vacuo providing an orange oil. Trituration in hexane provided the desired product as a yellow solid (1.39 g; 61%).
LC/MS: RT = 2.94 min; *m*/*z* = 400,398,396 [M+H]⁺. Total run time 3.75 mins.
¹H NMR (d₆ DMSO): δ 0.00 (s, 9H); 0.92 (t, 2H); 3.61 (t, 2H); 5.64 (s, 2H); 8.26 (s, 1H).

### Step 6

### 2,4-Dichloro-7-(2-trimethylsilanyl-ethoxymethyl)-7H-pyrrolo[2,3-d]pyrimidine-5-carboxylic acid .

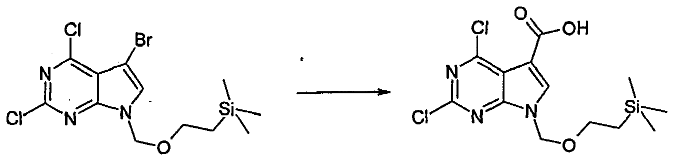

To a solution of n-butyl lithium (2.5M in hexanes; 1.18 ml; 2.95 mmol) in THF (10 ml) at - 78°C was added slowly dropwise a solution of 5-Bromo-2,4-dichloro-7-(2-trimethylsilanyl-ethoxymethyl)-7H-pyrrolo[2,3-d]pyrimidine (980 mg; 2.46 mmol) in THF (2 ml). After 5 minutes CO₂ was bubbled through the mixture and the mixture was left to warm to ambient temperature. Acetic acid was added then water (50 ml) and the mixture extracted with EtOAc (2 x 50 ml). The combined organics were dried over Na₂SO₄ then filtered and the filtrate solvents evaporated in vacuo leaving a green solid. Trituration in hexane afforded the desired product as a pale green solid (431 mg, 48%).
LC/MS: RT=2.60 min; *m*/*z* = 364/362 [M+H]⁺. Total run time 3.75 mins

### Step 7

### 2,4-Dichloro-7-(2-trimethlysilanyl-ethoxymethyl-7H-pyrrolo[2,3-d]pyrimidine-5-carboxylic acid amide

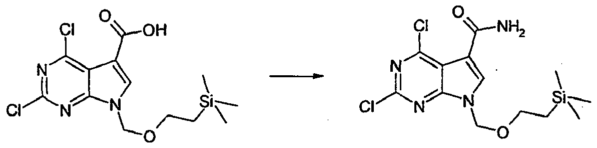

To a solution of 2,4-dichloro-7-(2-trimethylsilanyl-ethoxymethyl)-7H-pyrrolo[2,3-d]pyrimidine-5-carboxylic acid (320 mg; 0.89 mmol) in CH₂Cl₂ (10 ml) was added oxalyl chloride (2M in CH₂Cl₂, 0.58 ml; 1.16 mmol) followed by a few drops of DMF. After 20 min the reaction mixture was evaporated *in vacuo* then re-dissolved in CH₂Cl₂ (10 ml). Aqueous ammonia solution (6 ml) was added and the mixture was stirred vigorously for 3 hours. Water (50 ml), and CH₂Cl₂ (50 ml) were added and the resultant phases separated. The aqueous phase was extracted with further CH₂Cl₂ (50 ml). The combined organics were dried over Na₂SO₄ then filtered and the filtrate solvents evaporated in vacuo. The crude product was applied to a column of SiO₂ (20 g) eluting with 2% MeOH/CH₂Cl₂ to afford the title compound as a white solid (0.146 g; 46%).
LC/MS: RT = 2.42 min; *m*/*z* = 363, 361 [M+H]⁺. Total run time 3.75 mins.
¹H NMR (d₆ DMSO): δ 0.00 (s, 9H); 0.92 (t, 2H); 3.62 (t, 2H); 5.67 (s, 2H); 7.49 (br s, 1H); 7.88 (br s, 1H); 8.29 (s, 1H).

### Step 8

### 2,4-Dichloro-7-(2-trimethylsilanyl-ethoxymethyl)-7H-pyrrolo[2,3-d]pyrimidine

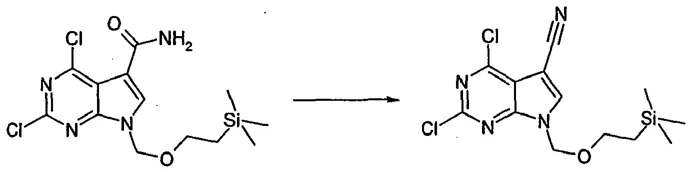

To a solution of 2,4-dichloro-7-(2-trimethylsilanyl-ethoxymethyl)-7H-pyrrolo[2,3-d]pyrimidine-5-carboxylic acid amide (146 mg; 0.405 mmol) in CH₂Cl₂ (10 ml) at 0°C was added Et₃N (0.12 ml; 0.87 mmol) followed by TFAA (0.06 ml; 0.43 mmol) slowly dropwise. The stirred reaction mixture was the allowed to warm to ambient temperature. Further CH₂Cl₂ (10 ml) was then added and the organic phase was washed with sat. NaHCO₃ solution (20 ml). The organic layer was dried over Na₂SO₄ then filtered and the filtrate solvents evaporated in vacuo. The crude product was purified by flash chromatography on SiO₂ (10 g) eluting with 10% EtOAc/Hexane to afford the title compound as a white solid (92 mg; 66%).
LC/MS: RT = 2.78 min; *m*/*z* = 345, 343 [M+H]⁺. Total run time 3.75 mins.
¹H NMR (d₆ DMSO): δ 0.00 (s, 9H); 0.99 (t, 2H); 3.62 (t, 2H); 5.69 (s, 2H); 8.96 (s, 1H).

### Step 9

### 2-Chloro-4-(2,4-dimethyl-phenyl)-7-(2-trimethylsilanyl-ethoxymethyl)-7H-pyrrolo[2,3-d]pyrimidine-5-carbonitrile

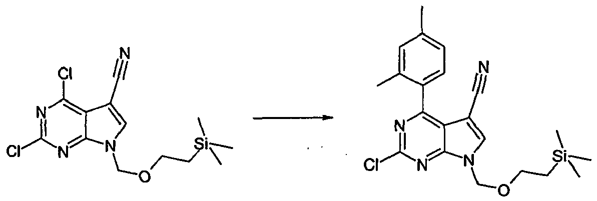

A mixture of 2,4-dichloro-7-(2-trimethylsilanyl-ethoxymethyl)-7H-pyrrolo[2,3-d]pyrimidine-5-carbonitrile (75 mg; 0.22 mmol), 2,4-dimethylphenylboronic acid (49 mg; 0.33 mmol), Pd(dppf)Cl₂ (10 mg; 0.012 mmol), K₂CO₃ (90 mg; 0.65 mmol) and THF/ H₂O (10:1; 2 ml) was degassed by bubbling N₂ through the mixture for 5 min. The reaction was then microwaved at 120 °C for 20 minutes. The mixture was allowed to cool and was then partitioned between EtOAc (2 x 20 ml) and brine (20 ml). The combined organics were dried over Na₂SO₄ then filtered and the filtrate solvents evaporated in vacuo. The crude product was purified by flash chromatography on SiO₂ (10 g) eluting with 10% EtOAc/Hexane to afford the desired product as a white solid (40 mg; 44%).
LC/MS: RT = 2.91 min; *m*/*z* = 415, 413 [M+H]⁺. Total run time 3.75 mins.
¹H NMR (d₆ DMSO): δ 0.00 (s, 9H); 0.94 (t, 2H); 2.27 (s, 3H); 2.45 (s, 3H); 3.68 (t, 2H); 5.73 (s, 2H); 7.25 (d, 1H); 7.30 (s, 1H), 7.40 (d, 1H); 8.89 (s, 1H).

### Step 10

### 2-Chloro-4-(2,4-dimethyl-phenyl)-7H-pyrrolo[2,3-d]pyrimidine-5-carbonitrile

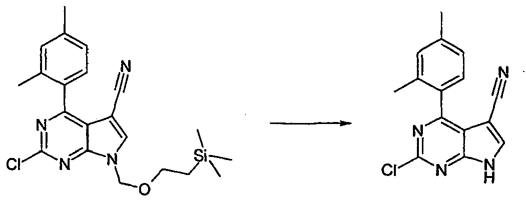

To a solution of 2-Chloro-4-(2,4-dimethyl-phenyl)-7-(2-trimethylsilanyl-ethoxymethyl)-7H-pyrrolo[2,3-d]pyrimidine-5-carbonitrile (40 mg; 0.097 mmol) in THF (2 ml) was added ethylenediamine (0.019 ml; 0.29 mmol) followed by tetrabutylamonium fluoride (1 M solution in THF; 0.58 ml; 0.58 mmol). The reaction mixture was heated at 50°C overnight. The reaction was allowed to cool to ambient temperature and was then partitioned between EtOAc (2x15 ml) and water (15 ml). The combined organics were dried over Na₂SO₄ then filtered and the filtrate solvents evaporated in vacuo. The resultant crude product was purified by prep HPLC, (pH = 4), to afford the desired product as a white solid (2.3 mg; 8.4%).
LC/MS: RT = 2.36 min; *m*/*z* = 283 [M+H]⁺. Total run time 3.75 mins.
¹H NMR (d₆ Acetone): δ 2.32 (s, 3H); 2.42 (s, 3H); 7.21 (d, 1H); 7.24 (s, 1H), 7.39 (d, 1H); 8.46 (s, 1H), NH not seen.
This compound had activity 'A' in the fluorescence polarization assay described below.

### Example 9

### 4-(2,4-dimethyl-phenyl)-7H-pyrrolo[2,3-d]pyrimidine-5-carbonitrile

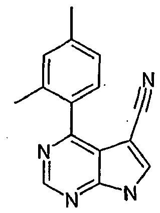

### Step 1

### 6-Amino-5-(2,2-diethoxy-ethyl)-pyrimidin-4-ol

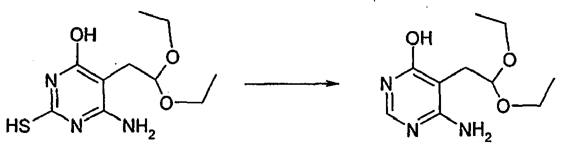

6-amino-5-(2,2-diethoxy-ethyl)-2-mercapto-pyrimidin-4-ol pyrimidine 3.0g (11.6 mmol) [prepared as detailed in Davoll. J., J. Chem. Soc. 1960, pp131-138] was dissolved in a mixture of water (150 ml and aqueous ammonia solution (9 ml) and heated to 90°C. Aliquots (2-3 ml) of a suspension of Raney Nickel were added to the reaction mixture until TLC and LC/MS analysis showed the reaction to be complete. The reaction mixture was allowed to cool to ambient temperature and filtered through a pad of celite. The filter cake was washed with water (2 x 25 mL) and the combined aqueous filtrate was freeze dried to afford the title compound as an off-white powder 2.23 g (85%)
LC/MS: RT = 1.37 min; *m*/*z* = 182 [M-EtOH+H]⁺. Total run time 3.75 mins.
¹H NMR (d₆ DMSO): δ 1.07 (brt, 6H); 3.40 (m, 2H); 3.59 (m, 2H); 4.56 (brt, 1H); 6.07 (brs, 2H); 7.70 (s, 1H); 11.43 (brs, 1H).

### Step2

### 7H-Pyrrolo[2,3-d]pyrimidine-4-ol

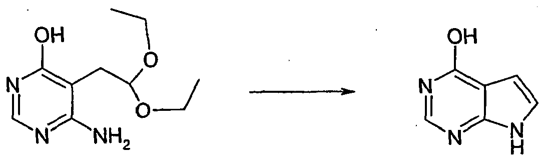

12.8M Hydrochloric acid (1.2 ml) was added to a suspension of 6-Amino-5-(2,2-diethoxyethyl)-pyrimidin-4-ol (2.23g 9.8 mmol) in water (60 ml) was stirred at ambient temperature for 2.5 hrs. The mixture was then cooled with an ice water bath and then filtered. The filtered solids were dried in vacuo to afford title compound as a yellow solid 1.2g (90%).
LC/MS: RT = 0.572 min; *m*/*z* = 158 [M+Na]⁺. Total run time 3.75 mins.
¹H NMR (d₆ DMSO): δ 6.40 (dd, 1H); 7.03 (dd, 1H); 7.82 (s, 1H); 11.74 (brs, 1H); 11.83 (brs, 1H).

### Step 3

### 4-Chloro-7H-Pyrrolo[2,3-d]pyrimidine

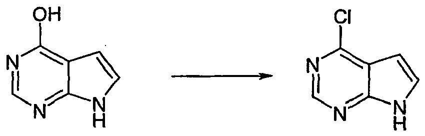

Phosphorous oxychloride was added to 7H-Pyrrolo[2,3-d]pyrimidine-4-ol (1.15 g, 8.5 mmol) and the reaction was heated under N₂ atmosphere to 100°C for 2.5 hours. The initial suspension becomes homogeneous dark suspension which was then allowed to cool to room temperature. Excess phosphorous oxychloride was removed in vacuo and the residue was cooled in ice bath and crushed ice was added with stirring. The mixture was diluted with water (20 ml) and extracted with ethyl acetate (2 x 30 ml). The combined organic extracts were washed with sat NaCl (aq) solution, then dried over anhydrous Na₂SO₄. Mixture was filtered and filtrate solvents removed in vacuo to afford a white solid (0.811 g; (62%).
LC/MS: RT = 1.619 min; *m*/*z* = 154 [M+H]⁺. Total run time 3.75 mins.
¹H NMR (d₆ DMSO): δ 6.60 (dd, 1H, J = 3.5, 1.8Hz); 7.69 (dd, 1H, J = 3.6, 2.3 Hz); 8.59 (s,1H), 12.57 (brs 1H).

### Step 4

### 4-Chloro-7-(2-trimethylsilanyl-ethoxymethyl)-7H-pyrrolo[2,3-d]pyrimidine

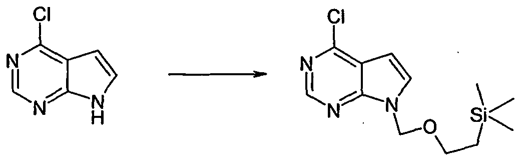

The title compound was prepared from 4-Chloro-7H-Pyrrolo[2,3-d]pyrimidine (0.805 g; 5.24 mmol) using the method of example 1 step 1. Product was purified by flash chromatography on silica gel (25g) eluting with 2-25% gradient of ethyl acetate in hexane. This afforded the title compound as colorless oil, 1.31g (87%).
LC/MS: RT = 0.572 min; *m*/*z* = 384 [M+H]⁺. Total run time 3.75 mins.
¹H NMR (d₆ DMSO): δ -0.66 (s, 9H); 0.90 (t, 2H); 3.51 (t, 2H); 5.64 (s, 2H); 6.66 (d, 1H); 7.38 (d, 1H); 8.66 (s, 1H).

### Step 5

### 4-(2,4-Dimethyl-phenyl)-7-(2-trimethylsilanyl-ethoxymethyl)-7H-pyrrolo[2,3-d]pyrimidine

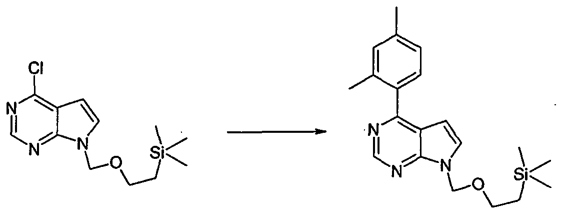

This compound was prepared by way of the method of example 1 step 2. Thus 4-Chloro-7-(2-trimethylsilanyl-ethoxymethyl)-7H-pyrrolo[2,3-d]pyrimidine (1.29g, 4.54 mmol) was reacted with 2,4-dimethylphenylboronic acid (0.818g; 1.2 equiv), dichlorobis(triphenylphosphine)palladium (II) and sodium bicarbonate in DMF/H₂O mix, and the crude product purified by flash chromatography on silica gel (25g) eluting with gradient of 3-30% ethyl acetate in hexane to afford title compound as a colorless oil, (1.29g; 80%).
LC/MS: RT = 2.87 min; *m*/*z* = 354 [M+H]⁺. Total run time 3.75 mins.

### Step 6

### 5-Bromo-4-(2,4-dimethyl-phenyl)-7-(2-trimethylsilanyl-ethoxymethyl)-7H-pyrrolo[2,3-d]pyrimidine

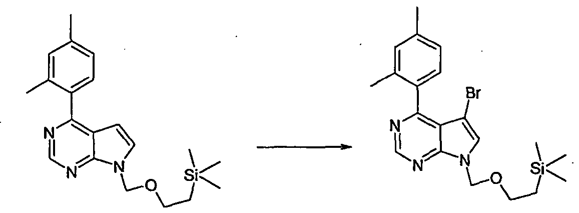

A solution of N-bromosuccinimide (0.639 g, 3.59 mmol) in DMF (10 ml) was added to an ice-bath cooled stirred solution of 4-(2,4-Dimethyl-phenyl)-7-(2-trimethylsilanyl-ethoxymethyl)-7H-pyrrolo[2,3-d]pyrimidine (1.27g, 3.59 mmol) in DMF (20 ml). Reaction mixture was then stirred at ambient temperature for 18 hours. DMF was removed *in vacuo* and residue was partitioned between ethyl acetate (150 ml) and water (150 ml). The phases were separated and the aqueous phase was re-extracted with ethyl acetate (50 ml). Combined organic phases were washed with sat NaCl (aq) solution and dried over Na₂SO₄. Mixture was filtered and filtrate solvent removed to afford a brown oil which was purified by flash chromatography on silica gel (50 g) eluting with gradient of 0-30% ethyl acetate in hexane to afford title compound as a colorless oil. (0.772 g; 49%).
LC/MS: RT = 2.940 min; *m*/*z* = 434,432 [M+H]⁺ (bromine isotope splitting pattern observed). Total run time 3.75 mins.

### Step 7

### 4-(2,4-Dimethyl-phenyl)-7-(2-trimethylsilanyl-ethoxymethyl)-7H-pyrrolo[2,3-d]pyrimidine-5-carboxylic acid.

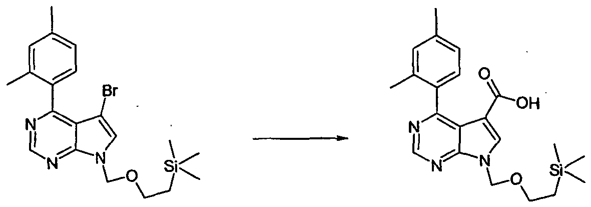

This compound was made by way of the method of example 2 step 2. Thus 0.77 g, 1.78 mmol) of 5-Bromo-4-(2,4-dimethyl-phenyl)-7-(2-trimethylsilanyl-ethoxymethyl)-7H-pyrrolo[2,3-d]pyrimidine was reacted with n-butyl liium and carbon dioxide to afford a crude product which was purified by flash chromatography on silica gel (50g) eluting with gradient of 25-100% ethyl acetate in hexane to afford title compound as a colorless solid, (0.307 g; 43%).
LC/MS: RT = 2.584 min; *m*/*z* = 398 [M+H]⁺. Total run time 3.75 mins.

### Step 8

### 4-(2,4-Dimethyl-phenyl)-7-(2-trimethylsilanyl-ethoxymethyl)-7H-pyrrolo[2,3-d]pyrimidine-5-carboxylic acid amide

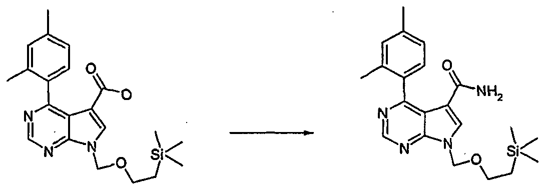

This compound was made by way of the method of example 2 step 3. Thus 0.304 g, 0.76 mmol) of 4-(2,4-dimethyl-phenyl)-7-(2-trimethylsilanyl-ethoxymethyl)-7H-pyrrolo[2,3-d]pyrimidine-5-carboxylic acid was reacted with oxalyl chloride and ammonia to afford a crude product (brown oil) which was purified by flash chromatography on silica gel (25g) eluting with gradient of 50-100% ethyl acetate in hexane to afford title compound as a colorless solid, (0.135 g; 45%).
LC/MS: RT = 2.446 min; *m*/*z* = 397 [M+H]⁺. Total run time 3.75 mins.

### Step 9

### 4-(2,4-Dimethyl-phenyl)-7-(2-trimethylsilanyl-ethoxymethyl)-7H-pyrrolo[2,3-d]pyrimidine-5-carbonitrile

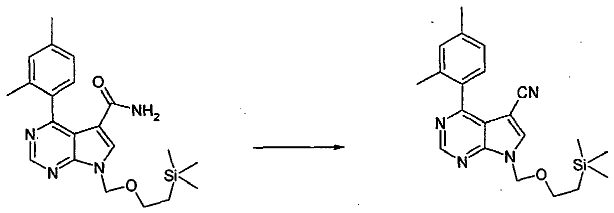

This compound was made by way of the method of example 2 step 4. Thus 0.133 g, 0.34 mmol) of 4-(2,4-dimethyl-phenyl)-7-(2-trimethylsilanyl-ethoxymethyl)-7H-pyrrolo[2,3-d]pyrimidine-5-carboxylic acid amide was reacted with trifluoroacetic anhydride to afford a crude product (brown oil) which was purified by flash chromatography on silica gel (25g) eluting with gradient of 20-70% ethyl acetate in hexane to afford title compound as a colorless solid, (0.075 g; 59%).
LC/MS: RT = 2.789 min; *m*/*z* = 379 [M+H]⁺. Total run time 3.75 mins.

### Step 10

### 4-(2,4-Dimethyl-phenyl)-7H-pyrrolo[2,3-d]pyrimidine-5-carbonitrile

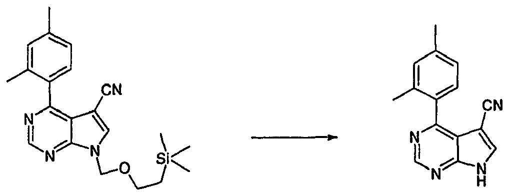

This compound was made by way of the method of example 2 step 6. Thus 0.075 g, 0.20 mmol) of 4-(2,4-dimethyl-phenyl)-7-(2-trimethylsilanyl-ethoxymethyl)-7H-pyrrolo[2,3-d]pyrimidine-5-carbonitrile was reacted with TBAF to afford a crude product (brown oil) which was purified by flash chromatography on silica gel (10g) eluting with 3:2 ethyl acetate: hexane to afford title compound as a colorless solid, (0.075 g; 59%).
LC/MS: RT = 2.034 min; *m*/*z* = 249 [M+H]⁺. Total run time 3.75 mins.
¹H NMR (d₆ DMSO): δ 2.18 (s, 3H); 2.37 (s, 3H); 7.14 (d, 1H, J=7.5 Hz); 7.20 (s, 1H); 7.30 (d, 1H, J=7.5 Hz); 8.52 (s, 1H); 8.97 (s, 1H); 13.34 (s, 1H).
This compound had activity 'B' in the fluorescence polarization assay described below.

### Example 10

### 4-(2,4-Dimethyl-phenyl)-2-methylsulfanyl-5-trifluoromethyl-7H-pyrrolo[2,3-d]pyrimidine

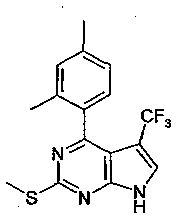

### Step1

### 4-(2,4-dimethyl-phenyl)-2-methylsulfanyl-5-trifluoromethyl-7-(2-trimethylsilanyl-ethoxymethyl)-7H-pyrrolo[2,3-d]pyrimidine

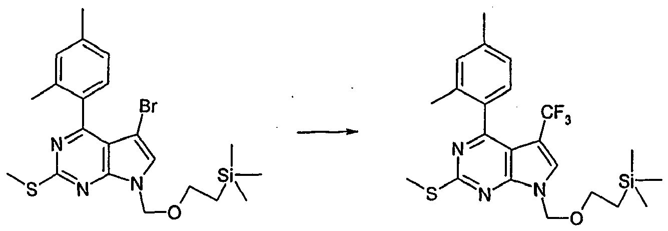

5-Bromo-4-(2,4-dimethyl-phenyl)-2-methylsulfanyl-7-(2-trimethylsilanyl-ethoxymethyl)-7H-pyrrolo[2,3-d]pyrimidine (example 1, step3) (100 mg, 0.209 mmol), Cul (80 mg, 0.418 mmol), sodium trifluoroacetate (57 mg, 0.418 mmol), toluene (0.5 ml) and DMF (1 ml) were combined under N₂ and heated to 170°C overnight. The reaction mixture was allowed to cool to RT and was then partitioned between EtOAc (2x15 ml) and water (15 ml). The organics were passed through a hydrophobic frit and evaporated *in vacuo*. The resultant crude was purified by flash chromatography on SiO₂ (20g) eluting with Hexane-6% EtOAc/Hexane (gradient) to afford the desired protected product together with dehalogenated product.

### Step2

### 4-(2,4-Dimethyl-phenyl)-2-methylsulfanyl-5-trifluoromethyl-7H-pyrrolo[2,3-d]pyrimidine

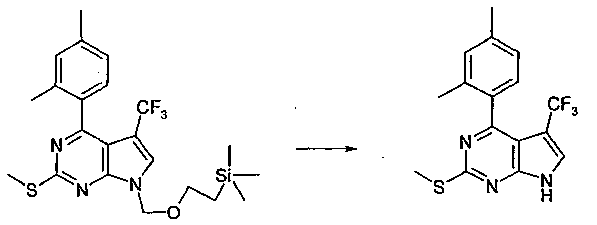

The product from step 1 was de-protected using the method of example 1 step 5. The final product was purified by HPLC (performed at pH 4) to afford the title compound as an off-white solid, 7 mg, 10%.
LC/MS: RT=2.68 Min; *m*/*z* = 349 [M+H]⁺. Total run time 3.75 mins.
¹H NMR (d₆ DMSO): δ 1.92 (s, 3H); 2.34 (s, 3H); 2.55 (s, 3H); 7.04-7.15 (m, 3H); 8.08 (s, 1H); NH not observed.
This compound had activity 'B' in the fluorescence polarization assay described below.

### Example 11

### 5-Cyclopropyl-4-(2,4-dimethyl-phenyl)-2-methylsulfanyl-7H-pyrrolo[2,3-d]pyrimidine

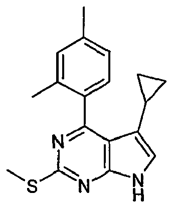

5-Bromo-4-(2,4-dimethyl-phenyl)-2-methylsulfanyl-7-(2-trimethylsilanyl-ethoxymethyl)-7H-pyrrolo[2,3-d]pyrimidine (example 1, step 3) (100 mg, 0.209 mmol), Pd(OAc)₂ (3 mg, 0.01 mmol), P(Cy)₃ (57 mg, 0.418 mmol), K₃PO₄ (1.70 mg, 0.80 mmol), cyclopropylboronic acid (25 mg, 0.30 mmol) toluene (1.0 ml) and water (0.05 ml) were combined under N₂ and the mixture degassed by bubbling N₂ through it for 5 min. The reaction was then heated at 100°C for 2h. The reaction mixture was allowed to cool to RT and was then partitioned between EtOAc (2 x 15 ml) and water (15 ml). The organics were dried (Na₂SO₄) and passed through a hydrophobic frit and evaporated *in vacuo*. The resultant crude product was purified by flash chromatography on SiO₂ (10g) eluting with Hexane-5% EtOAc/Hexane (gradient) to afford the desired protected and some dehalogenated produc (17 mg). This compound mixture was deprotected using the method outlined in example 1 step 5). The crude product was purified by HPLC (performed at pH 4) to afford the title compound as an off-white solid, 4 mg, 6%.
LC/MS: RT=2.70 min; *m*/*z* = 310 [M+H]⁺. Total run time 3.75 mins.
¹H NMR (d₆ DMSO): δ 0.36-0.40 (m, 4H); 1.06 m, 1H); 2.10 (s, 3H); 2.34 (s, 3H); 2.52 (s, 3H); 7.00 (m, 1H); 7.09 (d, 1H, J = 7.5 Hz); 7.14 (s, 1H); 7.20 (d, 1H, J = 7.5 Hz); 11.7 (brs, 1H).
This compound had activity 'C' in the fluorescence polarization assay described below.

### Example 12

### 4-(4-Fiuoro-2-methyl-phenyl)-2-methoxy-7H-pyrrolo[2,3-d]pyrimidine-5-carbonitrile

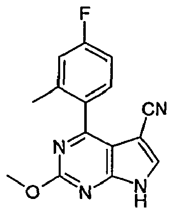

The title compound made by way of the route outlined in scheme 2 and scheme 4.

### Step1

### 4-(4-Fluoro-2-methyl-phenyl)-2-methanesulfonyl-7-(2-trimethylsilanyl-ethoxymethyl)-7H-pyrrolo[2,3-d]pyrimidine-5-carbonitrile

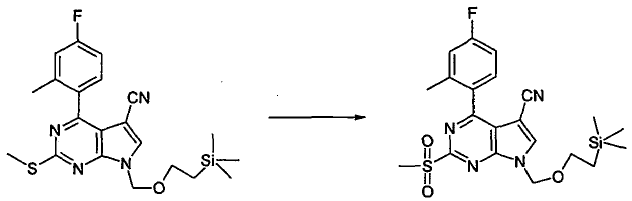

To a solution of 4-[(2-methyl-4-fluoro-phenyl]-2-methylsulfanyl-7-(2-trimethylsilanyl-ethoxymethyl)-7H-pyrrolo[2,3-d]pyrimidine-5-carbonitrile (189 mg, 0.44 mmol) (example 4 step1) in CH₂Cl₂ (8.5 ml) at 0°C was added drop wise a solution of *m*CPBA (396 mg, 1.76 mmol) in CH₂Cl₂ (8.5 ml). After addition was complete the reaction was allowed to warm to RT. After 1h the reaction mixture was washed with 5% Na₂S₂O₃ solution (20 ml). The aqueous layer was extracted with further CH₂Cl₂ (20 ml). The combined organics were then washed with sat. NaHCO₃ sol. (40 ml). The organics were then passed through a hydrophobic frit and evaporated *in vacuo*. The resultant crude product was purified by flash chromatography on SiO₂ (25g) eluting with 20% EtOAc/Hexane-45% EtOAc/Hexane (gradient) to afford the title compound as a colourless oil, 187 mg, 92%.
LC/MS: RT=2.65 Min; *m*/*z* = 461 [M+H]⁺. Total run time 3.75 mins.

### Step 2

### 4-(4-Fluoro-2-methyl-phenyl)-2-methoxy-7H-pyrrolo[2,3-d]pyrimidine-5-carbonitrile

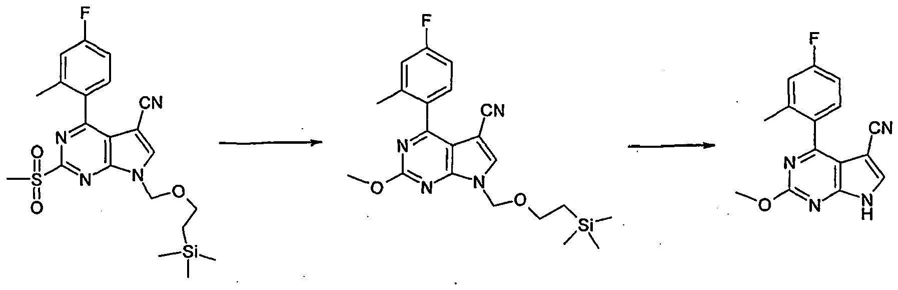

To a mixture of KO*^{t}*Bu (20 mg, 0.17 mmol) in THF (1 ml) at 0°C under N₂, MeOH (0.007 ml, 0.17 mmol) was added followed by a solution of 4-(4-Fluoro-2-methyl-phenyl)-2-methanesulfonyl-7-(2-trimethylsilanyl-ethoxymethyl)-7H-pyrrolo[2,3-d]pyrimidine-5-carbonitrile (40 mg, 0.087 mmol) in THF (0.5 ml) drop wise. After 30 min. the reaction mixture was partitioned between EtOAc (2x10 ml) and sat. NaHCO₃ solution (15 ml). The organics were then passed through a hydrophobic frit and evaporated *in vacuo* to give the crude protected product. This was deprotected with tetrabutylammonium fluoride using the method outlined in example 1 step 5. Purification was by flash chromatography on silica gel (10g) eluting with 10% EtOAc/Hexane-50% EtOAc/Hexane (gradient) to afford the title compound as a white solid, 12 mg, 48%.
LC/MS: RT=2.16 Min; *m*/*z* = 283 [M+H]⁺. Total run time 3.75 mins.
¹H NMR (d₆ DMSO): δ 2.23 (s, 3H); 3.96 (s, 3H); 7.17 (m, 1H); 7.26 (dd, 1H, J = 10.4 and 2.3 Hz); 7.44 (dd, 1H, J = 8.4, 6.0 Hz); 8.37 (s, 1H); 13.06 (brs, 1H).
This compound had activity 'B' in the fluorescence polarization assay described below.

### Example 13

### 4-(4-Fluoro-2-methyl-phenyl)-2-(2-pyrrolidin-1-yl-ethoxy)-7H-pyrrolo[2,3-d]pyrimidine-5-carbonitrile

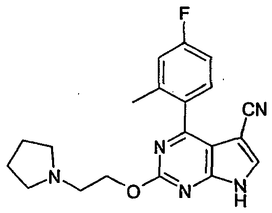

The title compound was prepared using the route outline in scheme 2 and scheme 4 using the methods outlined in example 12. Thus 4-(4-Fluoro-2-methyl-phenyl)-2-methanesulfonyl-7-(2-trimethylsilanyl-ethoxymethyl)-7H-pyrrolo[2,3-d]pyrimidine-5-carbonitrile was reacted with 2-pyrrolidine-1-yl-ethanol and the resulting product deprotected with TBAF.
LC/MS: RT=1.63 Min; *m*/*z* = 366 [M+H]⁺. Total run time 3.75 mins.
¹H NMR (d₆ DMSO): δ 1.68-1.74 (brm, 4H); 2.23 (s, 3H); 2.60-2.67 (brm, 4H); 2.93 (brt, 2H); 4.46 (t, 2H, J = 5.8 Hz); 7.17 (m, 1H); 7.23 (dd, 1H, J = 10.2 and 2.5 Hz); 7.44 (dd, 1H, J = 8.6, 6.0 Hz); 8.37 (s, 1H); 12.7 (brs, 1H).
This compound had activity 'C' in the fluorescence polarization assay described below.

### Example 14

### 4-(4-Fluoro-2-methyl-phenyl)-2-(2-morpholin-4-yl-ethoxy)-7H-pyrrolo[2,3-d]pyrimidine-5-carbonitrile

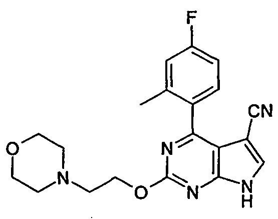

The title compound was prepared using the route outline in scheme 2 and scheme 4 using the methods out lined in example 12. Thus 4-(4-Fluoro-2-methyl-phenyl)-2-methanesulfonyl-7-(2-trimethylsilanyl-ethoxymethyl)-7H-pyrrolo[2,3-d]pyrimidine-5-carbonitrile was reacted with 2-morpholin-4-yl-ethanol and the resulting product de-protected with TBAF.
LC/MS: RT=1.62 Min; *m*/*z* = 382 [M+H]⁺. Total run time 3.75 mins.
¹H NMR (d₆ DMSO): δ 2.23 (s, 3H); 2.41-2.49 (m, 4H); 2.72 (t, 2H, J = 5.8 Hz); 3.55 (t, 4H, J = 4.5 Hz); 3.60 (t, 2H, J = 5.5 Hz); 4.52 (t, 2H, J = 5.8 Hz); 7.17 (m, 1H); 7.23 (dd, 1H, J = 10.1 and 2.6 Hz); 7.44 (dd, 1H, J = 8.3, 6.1 Hz); 8.36 (s, 1H); 13.0 (brs, 1H).
This compound had activity 'B' in the fluorescence polarization assay described below.

### Example 15

### 4-(4-Fluoro-2-methyl-phenyl)-2-[2-(2-oxo-pyrrolidin-1-yl)-ethoxy]-7H-pyrrolo[2,3-d]pyrimidine-5-carbonitrile

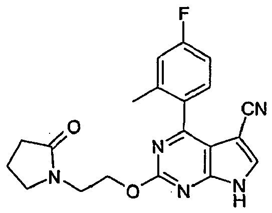

The title compound was prepared using the route outline in scheme 2 and scheme 4 using the methods out lined in example 12. Thus 4-(4-Fluoro-2-methyl-phenyl)-2-methanesulfonyl-7-(2-trimethylsilanyl-ethoxymethyl)-7H-pyrrolo[2,3-d]pyrimidine-5-carbonitrile was reacted with 1-(2-hydroxy-ethyl-pyrrolidin-2-one and the resulting product de-protected with TBAF.
LC/MS: RT=2.023 Min; *m*/*z* = 380 [M+H]⁺. Total run time 3.75 mins.
¹H NMR (d₆ DMSO): δ 1.89 (m, 2H); 2.19 (t, 2H, J = 8.4 Hz); 2.23 (s, 3H); 3.45 (t, 2H, J = 6.8 Hz); 3.60 (t, 2H, J = 5.5 Hz); 4.45 (t, 2H, J = 5.5 Hz); 7.17 (m, 1H); 7.26 (dd, 1H, J = 10.2 and 2.6 Hz); 7.44 (dd, 1H, J = 8.6, 6.1 Hz); 8.38 (s, 1H); 13.0 (brs, 1H).
This compound had activity 'B' in the fluorescence polarization assay described below.

### Example 16

### 4-(4-Fluoro-2-methyl-phenyl)-2-(4-methyl-piperazin-1-yl)-7H-pyrrolo[2,3-d]pyrimidine-5-carbonitrile

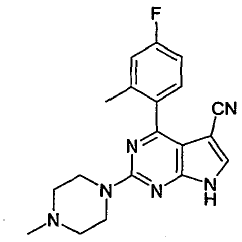

### Step1

### 4-(4-Fluoro-2-methyl-phenyl)-2-(4-methyl-piperazin-1-yl)-7-(2-trimethylsilanyl-ethoxymethyl)-7H-pyrrolo[2,3-d]pyrimidine-5-carbonitrile

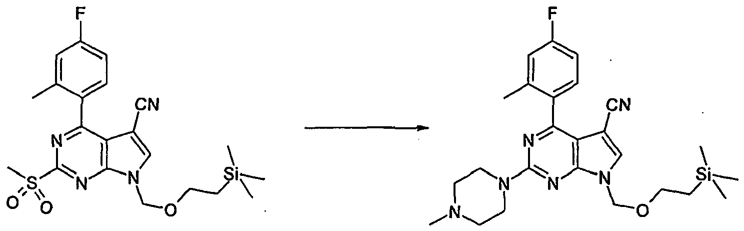

4-[(2-methyl-4-fluoro-phenyl]-2-methylsulfanyl-7-(2-trimethylsilanyl-ethoxymethyl)-7H-pyrrolo[2,3-d]pyrimidine-5-carbonitrile (example 12 step 1) (50 mg, 0.11 mmol), 1-methylpiperazine (0.024 ml, 0.22 mmol), and anhydrous DMF (0.5 ml) were combined under N₂ and heated at 100°C for 3 h. The reaction was allowed to cool to RT and was partitioned between EtOAc (2x10 ml) and sat. NaHCO₃ solution (10 ml). The organics were then passed through a hydrophobic frit and evaporated *in vacuo* to give the crude protected product. This was purified by flash chromatography on silica gel (10g) eluting with 10% EtOAc/Hexane-50% EtOAc/Hexane (gradient) to afford the title compound as colourless oil, 29 mg, 55%.
LC/MS: RT=2.14 Min; *m*/*z* = 481 [M+H]⁺. Total run time 3.75 mins.

### Step 2

### 4-(4-Fluoro-2-methyl-phenyl)-2-(4-methyl-piperazin-1-yl)-7H-pyrrolo[2,3 d]pyrimidine-5-carbonitrile

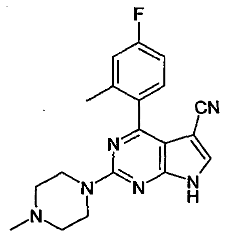

4-(4-Fluoro-2-methyl-phenyl)-2-(4-methyl-piperazin-1-yl)-7-(2-trimethylsilanyl-ethoxymethyl)-7H-pyrrolo[2,3-d]pyrimidine-5-carbonitrile was deprotected with tetrabutylammonium fluoride using the method outlined in example 1 step 5. Purification was by flash chromatography on silica gel (10g) eluting with CH₂Cl₂-6% MeOH/CH₂Cl₂ (gradient) to afford the title compound as a yellow solid, 4 mg, 18%.
LC/MS: RT=1.67 Min; *mlz* = 351 [M+H]⁺. Total run time 3.75 mins.
¹H NMR (d₆ DMSO): δ 0.93 (t, 6H, J = 7.1 Hz); 2.53 (q, 4H, J = 7.1 Hz); 2.75 (brt, 2H, J= 8.0 Hz); 3.28 (m, 2H); 7.17 (m, 1H); 7.25 (dd, 1H, J = 10.2 and 2.6 Hz); 7.44. (dd, 1H, J = 8). 13.0 brs 1H.
This compound had activity 'C' in the fluorescence polarization assay described below.

### Example 17

### 4-(4-Fluoro-2-methyl-phenyl)-2-methylamino-7H-pyrrolo[2,3-d]pyrimidine-5-carbonitrile

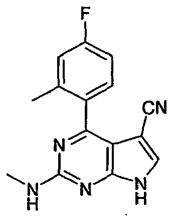

The title compound was prepared using the methods out lined in example 16, and the route outlined in scheme 2 and scheme 4. Thus 4-(4-Fluoro-2-methyl-phenyl)-2-methanesulfonyl-7-(2-trimethylsilanyl-ethoxymethyl)-7H-pyrrolo[2,3-d]pyrimidine-5-carbonitrile was reacted with methylamine and the resulting product de-protected with TBAF.
LC/MS: RT=2.08 Min; *m*/*z* = 282 [M+H]⁺. Total run time 3.75 mins.
¹H NMR (d₆ DMSO): δ 2.21 (s, 3H); 2.83 (d, 3H, J = 4.8 Hz); 7.11 (m, 1H); 7.20 (dd, 1H, J = 10.1 and 2.5 Hz); 7.12-7.21 (brs, 1H); 7.36 (dd, 1H, J = 8.3 and 6.0 Hz); 8.02 (s, 1H); 12.44 (brs, 1H).
This compound had activity 'B' in the fluorescence polarization assay described below.

### Example 18

### 2-Ethyl-4-(4-fluoro-2-methyl-phenyl)-7H-pyrrolo[2,3-d]pyrimidine-5-carbonitrile

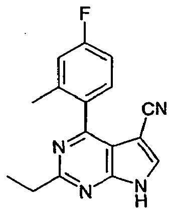

EtMgBr (0.04 ml, 0.11 mmol, 3M solution in Et₂O) was added to a solution of 4-[(2-methyl-4-fluoro-phenyl]-2-methylsulfanyl-7-(2-trimethylsilanyl-ethoxymethyl)-7H-pyrrolo[2,3-d]pyrimidine-5-carbonitrile (example 12 step 1) (50 mg, 0.11 mmol) at 0°C. After 20 min. the reaction was partitioned between EtOAc (2 x 15 ml) and water (15 ml). The organics were then passed through a hydrophobic frit and evaporated *in vacuo* to give the crude protected product. This product was deprotected with tetrabutylammonium fluoride using the method outlined in example 1 step 5. Purification was by flash chromatography on silica gel (10g) eluting with CH₂Cl₂-6% MeOH/CH₂Cl₂ (gradient) to afford the title compound as a beige solid, 24 mg, 79%.
LC/MS: RT=2.20 Min; *m*/*z* = 281 [M+H]⁺. Total run time 3.75 mins.
¹H NMR (d₆ DMSO): δ 1.33 (t, 3H, J = 7.6 Hz); 2.21 (s, 3H); 2.99 (q, 2H, J= 7.6 Hz); 7.17 (m, 1H); 7.25 (dd, 1H, J = 10.1 and 2.5 Hz); 7.44 (dd, 1H, J = 8.5 and 6.0 Hz); 8.50 (s, 1H); 13.18 (brs, 1H).
This compound had activity 'B' in the fluorescence polarization assay described below.

### Example 19

### 2-(2-Diethylamino-ethylsulfanyl)-4-(4-fluoro-2-methyl-phenyl)-7H-pyrrolo[2,3-d]pyrimidine-5-carbonitrile

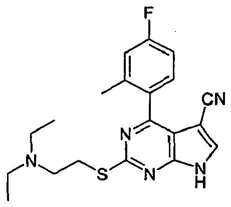

### Step 1

### 2-(2-Diethylamino-ethylsulfanyl)-4-(4-fluoro-2-methyl-phenyl)-7-(2-trimethylsilanyl-ethoxymethyl)-7H-pyrrolo[2,3-d]pyrimidine-5-carbonitrile

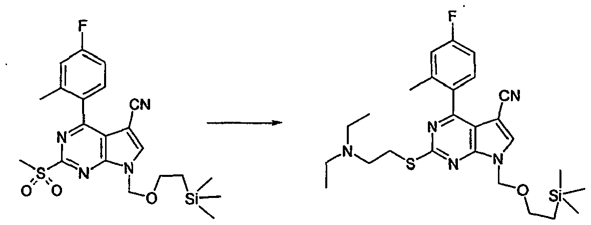

4-[(2-methyl-4-fluoro-phenyl]-2-methylsulfanyl-7-(2-trimethylsilanyl-ethoxymethyl)-7H-pyrrolo[2,3-d]pyrimidine-5-carbonitrile (example 12 step 1) (50 mg, 0.11 mmol), 2-diethylaminoethanethiol hydrochloride (37 mg, 0.22 mmol), Et₃N (0.03 ml, 0.22 mmol) and anhydrous DMF (2.0 ml) were combined under N₂ and heated at 100°C for 40 min. The reaction was allowed to cool to RT and was partitioned between EtOAc (2 x 15 ml) and NH₃ (aq) solution (15 ml). The organics were then passed through a hydrophobic frit and evaporated *in vacuo* to give the crude protected product. This was purified by flash chromatography on silica gel (10g) eluting with CH₂Cl₂-6% MeOH/CH₂Cl₂ (gradient) to afford the title compound as a yellow oil, 40 mg, 71 %.
LC/MS: RT=2.17 Min; *m*/*z* = 514 [M+H]⁺. Total run time 3.75 mins.

### Step 2

### 2-(2-Diethylamino-ethylsulfanyl)-4-(4-fluoro-2-methyl-phenyl)-7H-pyrrolo[2,3-d]pyrimidine-5-carbonitrile

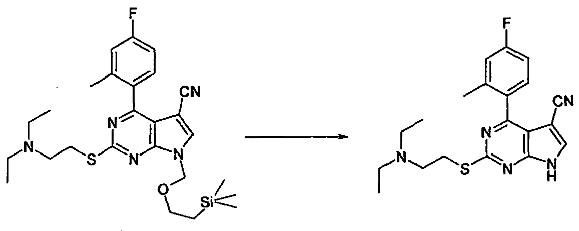

2-(2-Diethylamino-ethylsulfanyl)-4-(4 fluoro-2-methyl-phenyl)-7-(2-trimethylsilanyl-ethoxymethyl)-7H-pyrrolo[2,3-d]pyrimidine-5-carbonitrile was deprotected with tetrabutylammonium fluoride using the method outlined in example 1 step 5. Purification was by flash chromatography on silica gel (10g) eluting with CH₂Cl₂-13% MeOH/CH₂Cl₂ (gradient) to afford the title compound as a white solid, 20 mg, 67%.
LC/MS: RT=1.73 Min; *m*/*z* = 384 [M+H]⁺. Total run time 3.75 mins.
¹H NMR (d₆ DMSO): δ 0.93 (t, 6H, J = 7.1 Hz); 2.53 (q, 4H, J = 7.1 Hz); 2.75 (brt, 2H, J= 8.0 Hz); 3.28 (m, 2H); 7.17 (m, 1H); 7.25 (dd, 1H, J = 10.2 and 2.6 Hz); 7.44 (dd, 1H, J = 8.3 and 6.1 Hz); 8.43 (s, 1H); 12.91 (brs, 1H).
This compound had activity 'A' in the fluorescence polarization assay described below.

### Example 20

### 4-(4-Fluoro-2-methyl-phenyl)-2-(2-hydroxy-ethylsulfanyl)-7H-pyrrolo[2,3-d]pyrimidine-5-carbonitrile

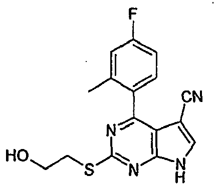

The title compound was prepared using the methods out lined in example 18, and the route outlined in scheme 2 and scheme 4. Thus 4-(4-Fluoro-2-methyl-phenyl)-2-methanesulfonyl-7-(2-trimethylsilanyl-ethoxymethyl)-7H-pyrrolo[2,3-d]pyrimidine-5-carbonitrile was reacted with 2-mercatoethanol and the resulting product de-protected with TBAF.
LC/MS: RT=2.073 Min; *m*/*z* = 329 [M+H]⁺. Total run time 3.75 mins.
¹H NMR (d₆ DMSO): δ 2.22 (s, 3H); 3.27 (t, 2H, J = 6.6 Hz); 3.68 (m, 2H); 4.99 (t, 1H, J = 5.3 Hz);7.18 (m, 1H); 7.26 (dd, 1H, J = 10.1 and 2.1 Hz); 7.44 (dd, 1H, J = 8.3 and 6.1 Hz); 8.43 (s, 1H); 13.17 (brs, 1H).
This compound had activity 'A' in the fluorescence polarization assay described below.

### Example 21

### 4-(4-Fluoro-2-methyl-phenyl)-2-(2-morpholin-4-yl-ethylsulfanyl)-7H-pyrrolo[2,3-d]pyrimidine-5-carbonitrile

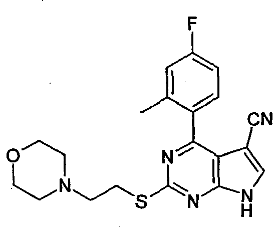

### Step 1

### 4-(4-Fluoro-2-methyl-phenyl)-2-(2-morpholin-4-yl-ethylsulfanyl)-7-(2-trimethylsilanyl-ethoxymethyl)-7H-pyrrolo[2,3-d]pyrimidine-5-carbonitrile

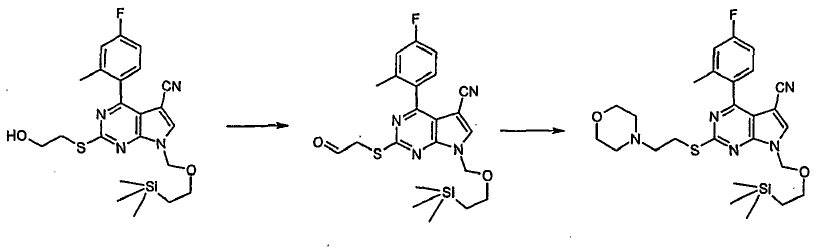

To a solution of 4-(4-Fluoro-2-methyl-phenyl)-2-(2-hydroxy-ethylsulfanyl)-7H-pyrrolo[2,3-d]pyrimidine-5-carbonitrile (precursor to example 20) (100 mg, 0.22 mmol) in CH₂Cl₂ (10 ml) Dess-Martin periodinane (111 mg, 0.26 mmol) was added. The reaction was stirred for 5 h. at RT. The mixture was then evaporated *in vacuo* to give the crude protected aldehyde. This was partially purified by flash chromatography on silica gel (20g) eluting with Hexane-40% EtOAc/Hexane (gradient) to afford the aldehyde as colourless oil, 73 mg. This was combined with morpholine (0.03 ml, 0.308 mmol), AcOH (0.04 ml, 0.77 mmol), powdered 3A molecular sieves, MeOH (3 ml) and Na(OAc)₃BH₃ (65 mg, 0.31 mmol) and stirred at RT under N₂ for 2 h. The mixture was then filtered and the filtrate evaporated *in vacuo*. This was then partitioned between CH₂Cl₂ (2 x 10 ml) and sat. NaHCO₃ solution (10 ml). The organics were then passed through a hydrophobic frit and evaporated *in vacuo* to give the crude protected product. This was purified by flash chromatography on silica gel (10g) eluting with 20% EtOAc/Hexane-70% EtOAc/Hexane (gradient) to afford the title compound as colourless oil, 47 mg, 41 %.
LC/MS:RT=2.25 Min; *m*/*z* = 528 [M+H]⁺. Total run time 3.75 mins.

### Step 2

### 4-(4-Fluoro-2-methyl-phenyl)-2-(2-morpholin-4-yl-ethylsulfanyl)-7H-pyrrolo[2,3-d]pyrimidine-5-carbonitrile

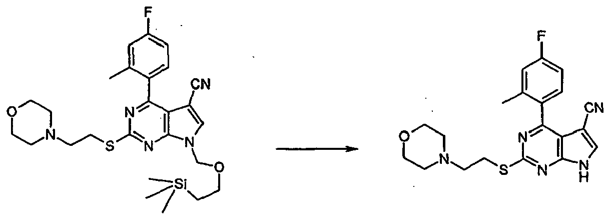

4-(4-Fluoro-2-methyl-phenyl)-2-(2-morpholin-4-yl-ethylsulfanyl)-7-(2-trimethylsilanyl-ethoxymethyl)-7H-pyrrolo[2,3-d]pyrimidine-5-carbonitrile was de-protected with tetrabutylammonium fluoride using the method outlined in example 1 step 5. Purification was by flash chromatography on silica gel (10g) eluting with CH₂Cl₂-5% MeOH/CH₂Cl₂ (gradient) to afford the title compound as a white solid, 19 mg, 54%.
LC/MS: RT=1.68 Min; *m*/*z* = 398 [M+H]⁺. Total run time 3.75 mins.
¹H NMR (d₆ DMSO): δ 2.22 (s, 3H); 2.43 (brm, 4H); 2.65 (t, 2H, J= 7.5 Hz); 3.30 (t, 2H, J = 7.5 Hz);3.55 (m, 4H); 7.18 (m, 1H); 7.26 (dd, 1H, J = 10.1 and 2.5 Hz); 7.44 (dd, 1H, J = 8.7 and 6.0 Hz); 8.44 (s, 1H); 13.17 (brs, 1H).
This compound had activity 'A' in the fluorescence polarization assay described below.

### Example 22

### 4-(2,4-Dichloro-5-methoxy-phenyl)-2-methylsulfanyl-7H-pyrrolo[2,3-d]pyrimidine-5-carbonitrile

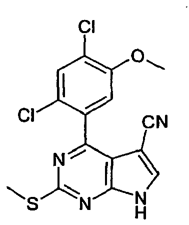

### Step 1

### 1-Benzyloxy-2,4-dichloro-5-nitro-benzene

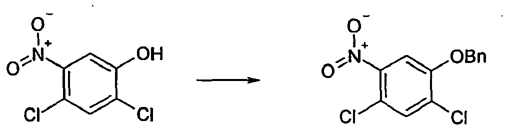

Potassium carbonate (12g, 87mmol) was added to a solution of 2,4-dichloro- 5-nitrophenol (Lancaster Synthesis, Morecambe, Lancashire, UK) (15.6g, 75mmol) in acetone. Benzyl bromide (9ml, 76mmol) was added and the suspension heated at 75°C (oil bath temperature) for ∼3hrs. The resulting suspension was allowed to cool and water (500ml) was added, the mixture was extracted with dichloromethane (2x200ml). The combined extracts were washed with aqueous sodium hydroxide (150ml, 2M), water (2x200ml) and saturated aqueous sodium chloride solution (150ml). The solution was dried over anhydrous sodium sulphate and concentrated to a pale yellow solid (21.5g, 96%)
R_{f} 0.73 CH₂Cl₂ (SiO₂)
LC retention time 2.915min [M+H]⁺ no ionisation (run time 3.75 min)

### Step 2

### 5-Benzyloxy-2,4-dichloro-phenylamine

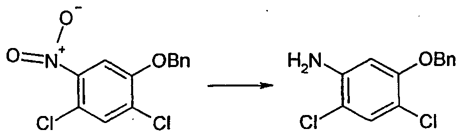

Iron powder (21 g, 376mmol) was added to a suspension 1-Benzyloxy-2,4-dichloro-5-nitro-benzene (21.5g, 72mmol) in acetic acid (300ml) / water (150ml) and the mixture was heated at 85°C (oil bath temperature) for ∼90mins. The resulting suspension was filtered. The filtrate was allowed to cool, water (750ml) was added and the mixture extracted with dichloromethane (3x150ml). The combined extracts were washed with aqueous sodium hydroxide (300ml, 2M), water (2x500ml) and saturated aqueous sodium chloride solution (200ml). The solution was dried over anhydrous sodium sulphate filtered and the filtrate solvents removed *in vacuo* to afford product as a pale brown solid (18.6g, 96%)
R_{f} 0.57 CH₂Cl₂ (SiO₂).
LC retention time 2.792min [M+H]⁺ 270/268 (run time 3.75 min)

### Step 3

### 1-Benzyloxy-2,4-dichloro-5-iodo-benzene

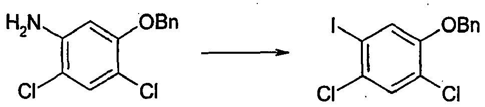

Hydrochloric acid (60ml, 6M) was added to a solution of the 5-Benzyloxy-2,4-dichloro-phenylamine (16.2g, 60mmol) in acetic acid (240ml) and the resulting suspension cooled (ice/water/salt). Aqueous sodium nitrite (4.8g, 69.5mmol in 40ml) was added slowly (keeping the temperature <5°C). On complete addition the resulting solution was stirred for ∼30mins. The resulting solution was poured into a solution of potassium iodide (20g, 120mmol) and iodine (4g, 16mmol) in water (200ml), and the mixture stirred for ∼90mins. Water (800ml) was added and the mixture extracted with dichloromethane (3x250ml). The combined extracts were washed with aqueous sodium thiosulphate solution (2x150ml, 10%), aqueous sodium hydroxide (250ml, 2M), water (2x250ml) and saturated aqueous sodium chloride solution (200ml). The solution was dried over anhydrous sodium sulphate and concentrated to a pale brown oil, solidified on standing. (20.6g, 90%).
R_{f} 0.82 CH₂Cl₂ (SiO₂)
LC retention time 3.084min [M+H]⁺ No ionisation (run time 3.75 min)

### Step 4

### 2,4-Dichloro-5-iodo-phenol

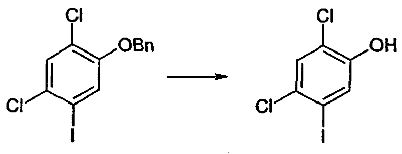

To a solution of 1-Benzyloxy-2,4-dichloro-5-iodo-benzene (3.0 g, 7.92 mmol) in CH₂Cl₂ (50 ml) at 0°C BCl₃ (23.8 ml, 23.8 mmol, 1 M solution in CH₂Cl₂) was added drop wise. After the addition was complete the reaction was allowed to warm to RT. The mixture was then partitioned between sat. NH₄Cl sol. (50 ml) and CH₂Cl₂ (2 x 50 ml). The combined organics were passed through a hydrophobic frit and evaporated *in vacuo* to give a crude oil. This was purified by flash chromatography on silica gel (70g) eluting with Hexane-10% EtOAc/Hexane (gradient) to afford the title compound as a yellow solid, 1.83 g, 80%.
LC/MS: RT=2.48 Min; *m*/*z* = 289, 287 [M-H]⁻. Total run time 3.75 mins.

### Step 5

### 2,5-Dichloro-2-iodo-methoxy-benzene

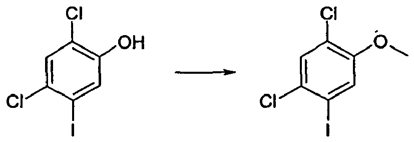

To a solution of 2,4-Dichloro-5-iodo-phenol (0.5 g, 1.73 mmol) in DMF (10 ml), K₂CO₃ (480 mg, 3.46 mmol) and Mel (0.12 ml, 1.90 mmol) were added sequentially and the resultant mixture stirred under N₂ at RT overnight. Added further equivalents of K₂CO₃ (480 mg, 3.46 mmol), Mel (0.12 ml, 1.90 mmol) and DMF (4 ml) and stirred at RT overnight. Partitioned the reaction mixture between NH₃ (aq) sol. (30 ml) and EtOAc (2 x 30 ml). Dried (Na₂SO₄) the combined organics and evaporated *in vacuo* to give the crude product. This was purified by flash chromatography on silica gel (50g) eluting with Hexane to afford the title compound as a yellow solid, 1.83 g, 80%.
LC/MS: RT=2.76 min; *m*/*z* = no mass. Total run time 3.75 mins.

### Step 6

### 4-(2,4-Dichloro-5-methoxy-phenyl)-2-methylsulfanyl-7-(2-trimethylsilanyl-ethoxymethyl)-7H-pyrrolo[2,3-d]pyrimidine-5-carbonitrile

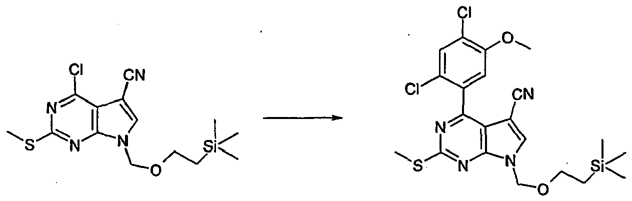

To a solution of 2,5-Dichloro-2-iodo-methoxy-benzene in THF at -78°C under N₂ triisopropyl borate (0.64 ml, 2.75mmol) was added followed by *ⁿ*BuLi (0.72 ml, 1.79 mmol, 2.5 M in Hexanes) drop wise. The reaction was allowed to warm to RT and was then evaporated *in vacuo* and partitioned between EtOAc (2 x 50 ml) and dil. HCl sol. (50 ml). The combined organics were dried (Na₂SO₄) and evaporated *in vacuo* to give the crude boronic acid as a white solid (320 mg). This was combined with 4-chloro-2-methylsulfanyl-7-(2-trimethylsilanyl-ethoxymethyl)-7H-pyrrolo[2,3-d]pyrimidine-5-carbonitrile (390 mg, 1.1 mmol), 1 M NaHCO₃ sol. (4.1 ml, 4.1 mmol), PdCl₂(PPh₃)₂ (48 mg, 0.07 mmol) and DMF (12 ml). The mixture was degassed by bubbling N₂ through it for 5 min. and was subsequently heated at 80°C for 3h under N₂. The reaction was allowed to cool before being partitioned between EtOAc (3 x 50 ml) and sat. NaHCO₃ sol. (50 ml): The combined organics were dried (Na₂SO₄) and evaporated *in vacuo* to give a crude oil. This was purified by flash chromatography on silica gel (70g) eluting with Hexane-20% EtOAc/Hexane (gradient) to afford the title compound as a yellow oil, 260 mg, 48%.
LC/MS: RT=2.96 Min; *m*/*z* = 495, 497 [M-H]⁻. Total run time 3.75 mins.

### Step 7

### 4-(2,4-Dichloro-5-methoxy-phenyl)-2-methylsulfanyl-7H-pyrrolo[2,3-d]pyrimidine-5-carbonitrile

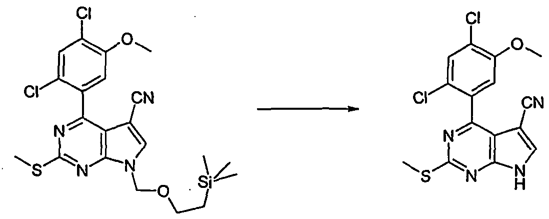

The title compound was prepared using the method out lined in example 1 step 5.
LC/MS: RT=2.52 min; *m*/*z* = 365, 367 [M+H]⁺. Total run time 3.75 mins.
¹H NMR (d₆ DMSO): δ 2.59 (s, 3H); 3.90 (s, 3H); 7.39 (s, 1H); 7.81 (s, 1H); 8.48 (s, 1H); 13.23.
This compound had activity'A' in the fluorescence polarization assay described below.

### Example 23

### 4-(2,4-Dichloro-5-methoxy-phenyl)-2-(2-diethylamino-ethylsulfanyl)-7H-pyrrolo[2,3-d]pyrimidine-5-carbonitrile

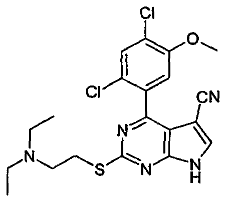

The title compound was prepared using the methods outlined in example 12, 19, and 22, and the route outlined in scheme 2 and scheme 4. Thus, 4-(2,4-dichloro-5-methoxyphenyl)-2-methylsulfanyl-7-(2-trimethylsilanyl-ethoxymethyl)-7H-pyrrolo[2,3-d]pyrimidine-5-carbonitrile (example 22 step 6) was oxidised with mcpba and the resulting sulphone displaced with 2-diethylaminoethanethiol. Removal of SEM protecting group with TBAF affords the title compound as a solid.
LC/MS: RT=1.84 Min; *m*/*z* = 454, 452 [M+H]⁺. Total run time 3.75 mins.
¹H NMR (d₆ DMSO): δ 0.98 (t, 6H, J = 7.0 Hz); 2.61-2.76 (m, 4H); 2.86-2.95 (m, 2H); 3.26-3.35 (m, 2H), 3.90 (s, 3H); 7.41 (s, 1H); 7.85 (s, 1H); 8.49 (s, 1H); 12.2-12.9 (brs, 1H).
This compound had activity 'A' in the fluorescence polarization assay described below.

### Example 24

### 4-(2,4-Dichloro-5-methoxy-phenyl)-2-(2-diethylamino-ethoxy)-7H-pyrrolo[2,3-d]pyrimidine-5-carbonitrile

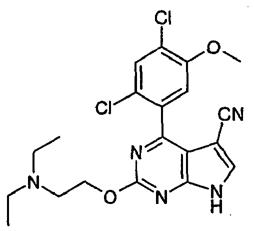

The title compound was prepared using the methods outlined in example 12 and 22, and the route outlined in scheme 2 and scheme 4. Thus, 4-(2,4-Dichloro-5-methoxy-phenyl)-2-methylsulfanyl-7-(2-trimethylsilanyl-ethoxymethyl)-7H-pyrrolo[2,3-d]pyrimidine-5-carbonitrile (example 22 step 6) was oxidised with mcpba and the resulting sulphone displaced with 2-diethylaminoethanol. Removal of SEM protecting group with TBAF affords the title compound as a solid.
LC/MS: RT=1.75 Min; *m*/*z* = 434, 436 [M+H]⁺. Total run time 3.75 mins.
¹H NMR (d₆ DMSO): δ 0.97 (t, 6H, J = 7.0 Hz); 2.57 (q, 4H, J = 7.0 Hz); 2.82 (t, 2H, J = 6.4 Hz); 3.90 (s, 3H); 4.41 (t, 2H, J = 6.4 Hz), 7.37 (s, 1H); 7.80 (s, 1H); 8.38 (s, 1H).
This compound had activity 'A' in the fluorescence polarization assay described below.

### Example 25

### 4-(2,4-Dichloro-5-methoxy-phenyl)-2-[2-(2-oxo-pyrrolidin-1-yl)-ethoxy]-7H-pyrrolo[2,3-d]pyrimidine-5-carbonitrile

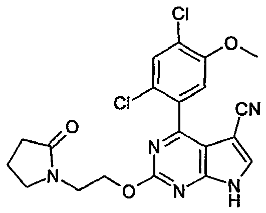

The title compound was prepared using the methods outlined in example 22, example 12, and the route outlined in scheme 2 and scheme 4. Thus, 4-(2,4-Dichloro-5-methoxyphenyl)-2-methylsulfanyl-7-(2-trimethylsilanyl-ethoxymethyl)-7H-pyrrolo[2,3-d]pyrimidine-5-carbonitrile (example 22 step 6) was oxidised with mcpba and the resulting sulphone displaced with 1-(2-hydroxy-ethyl-pyrrolidin-2-one. Removal of SEM protecting group with TBAF affords the title compound as a solid.
LC/MS: RT=2.14 Min; *m*/*z* = 446, 448 [M+H]⁺. Total run time 3.75 mins.
¹H NMR (d₆ DMSO): δ 1.89 (m, 2H); 2.20 (t, 2H, J = 8.0 Hz); 3.46 (t, 2H, J = 7.0 Hz)); 3.61 (t, 2H, J = 5.5 Hz); 3.90 (s, 3H); 4.47 (t, 2H, J = 5.5 Hz); 7.39 (s, 1H); 7.81 (s, 1H); 8.42 (d, 1H, J = 2.5 Hz); 13.13 (brs, 1H).
This compound had activity 'A' in the fluorescence polarization assay described below.

### Example 26

### 4-[2,4-Dichloro-5-(2-pyrrolidin-1-yl-ethoxy)-phenyl]-2-methylsulfanyl-7H-pyrrolo[2,3-d]pyrimidine-5-carbonitrile

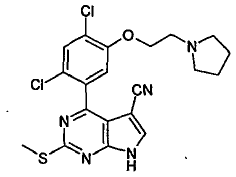

### Step 1

### 1-[2-(2,4-Dichloro-5-iodo-phenoxy)-ethyl]-pyrrolidine

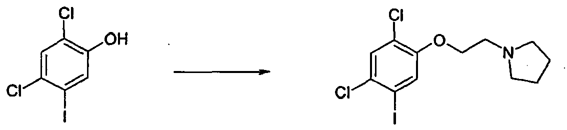

2,4-Dichloro-5-iodo-phenol (example 22, step 4) (1 g, 3.46 mmol), 1-(2-bromoethyl) pyrrolodine hydrobromide (3.81 mmol), CsCO₃ (2.8 g, 8.65 mmol) and DMF (15 ml) were combined under N₂ and heated at 110°C for 3 h. The reaction mixture was then partitioned between EtOAc (2 x 40 ml) and NH₃ sol. (40 ml). The combined organics were dried (Na₂SO₄) and evaporated *in vacuo* to give a crude oil. This was purified by flash chromatography on silica gel (25g) eluting with Hexane-45% EtOAc/Hexane (gradient) to afford the title compound as a yellow solid, 1.08 g, 80%.
LC/MS: RT=1.81 Min; *m*/*z* = 386, 388 [M+H]⁺. Total run time 3.75 mins.

### Step 2

### 4-[2,4-Dichloro-5-(2-pyrrolidin-1-yl-ethoxy)-phenyl]-2-methylsulfanyl-7H-pyrrolo[2,3-d]pyrimidine-5-carbonitrile

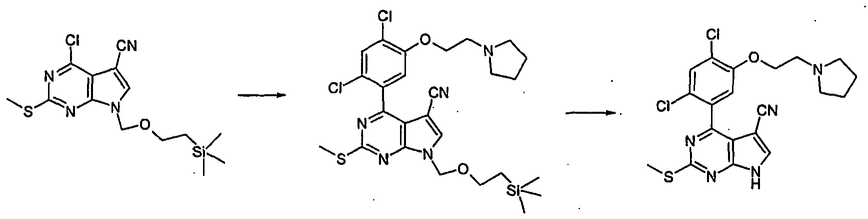

To a solution of 1-[2-(2,4-Dichloro-5-iodo-phenoxy)-ethyl]-pyrrolidine (200 mg, 0.518 mmol) in THF at -78°C under N₂ triisopropyl borate (0.24 ml, 1.04 mmol) was added followed by "BuLi (0.27 ml, 0.67 mmol, 2.5 M in Hexanes) drop wise. The reaction was allowed to warm to RT and was then evaporated *in vacuo* to give the crude boronic acid. This was combined with 4-chloro-2-methylsulfanyl-7-(2-trimethylsilanyl-ethoxymethyl)-7H-pyrrolo[2,3-d]pyrimidine-5-carbonitrile (92 mg, 0.26 mmol), 1M NaHCO₃ sol. (0.8 ml, 0.78 mmol), PdCl₂(PPh₃)₂ (9 mg, 0.01 mmol) and DMF (6 ml). The mixture was degassed by bubbling N₂ through it for 5 min. and was subsequently heated at 80°C for 2h under N₂. The reaction was allowed to cool before being partitioned between EtOAc (2 x 60 ml) and aqueous NH₃ sol. (60 ml). The combined organics were dried (Na₂SO₄) and evaporated *in vacuo* to give a crude oil. This was purified by flash chromatography on silica gel (50g) eluting with CH₂Cl₂-5% MeOH/CH₂Cl₂ (gradient) to afford the protected product as a yellow oil, 160 mg.. This product was de-protected using the method outlined in example 1 step 5 to afford the title compound as a yellow solid, 49 mg, 42%.
LC/MS: RT=1.83 min; *m*/*z* = 448, 450 [M+H]⁺. Total run time 3.75 mins.
¹H NMR (d₆ DMSO): δ 1.68 (m, 4H); 2.58 (s, 3H); 2.61 (m, 4H); 2.89 (t, 2H, J = 5.8 Hz); 4.22 (t, 2H, J = 5.7 Hz); 7.42 (s, 1H); 7.80 (s, 1H); 8.46 (s, 1H); 13.00 (brs, 1H).
This compound had activity 'A' in the fluorescence polarization assay described below.

### Example 27

### 4-[2,4-Dichloro-5-(2-diethylamino-ethoxy)-phenyl]-2-methylsulfanyl-7H-pyrrolo[2,3-d]pyrimidine-5-carbonitrile

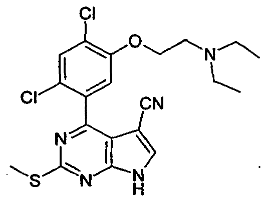

The title compound was prepared using the methods outlined in example 26, and the route outlined in scheme 2 and scheme 4. Thus [2-(2,4-dichloro-5-iodo-phenoxy)-ethyl]-diethylamine (prepared as for example 26 step 1) was converted to the 5-substituted boronic acid and reacted with 4-chloro-2-methylsulfanyl-7-(2-trimethylsilanyl-ethoxymethyl)-7H-pyrrolo[2,3-d]pyrimidine-5-carbonitrile. Removal of the SEM protecting group afforded the title compound as a solid.
LC/MS: RT=1.86 min; *m*/*z* = 450, 452 [M+H]⁺. Total run time 3.75 mins.
¹H NMR (d₆ DMSO): δ 0.99 (t, 6H, J = 7.1 Hz); 2.59 (s, 3H); 2.62 (q, 4H, J = 7.0 Hz); 2.90 (t, 2H, J = 5.1 Hz); 4.18 (t, 2H, J = 5.1 Hz); 7.42 (s, 1H); 7.80 (s, 1H); 8.47 (s, 1H); 12.8 (brs, 1H).
This compound had activity 'A' in the fluorescence polarization assay described below.

### Example 28

### 2-{5-Cyano-4-[2,4-dichloro-5-(2-diethylamino-ethoxy)-phenyl]-7H-pyrrolo[2,3-d]pyrimidin-2-ylsulfanyl}-N-methyl-acetamide

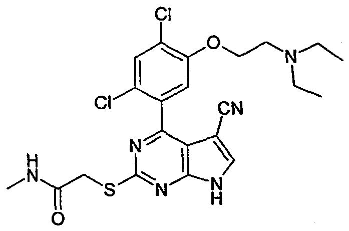

The title compound was prepared using the methods outlined in examples 12, 26, and 19, and the route outlined in scheme 2 and scheme 4.
LC/MS: RT=1.70 Min; *m*/*z* = 507, 509 [M+H]⁺. Total run time 3.75 mins.
¹H NMR (d₆ DMSO): δ 0.50 (t, 6H, J = 7.2 Hz); 1.92 (s, 3H); 2.38 (q, 4H, J = 7.2 Hz); 2.68 (t, 2H, J = 5.1 Hz); 3.14 (s, 2H); 3.62 (t, 2H, J = 5.1 Hz); 6.51 (s,1H); 6.88 (s, 1H), 7.37 (s, 1H); 7.72 (s, 1H).
This compound had activity 'A' in the fluorescence polarization assay described below.

### Example 29

### 4-(2-Chloro-4,5-dimethoxy-phenyl)-2-methylsulfanyl-7H-pyrrolo[2,3-d]pyrimidine-5-carbonitrile

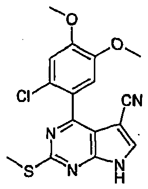

### Step 1

### 2-chloro-4,5-dimethoxyphenyl boronic acid

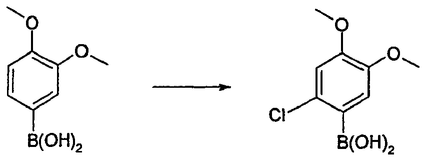

To a suspension of 3,4-dimethoxyboronic acid (364 mg) in acetonitrile (4 mL) were added TFA (50 uL) and NCS (294 mg). The reaction mixture was stirred for 6 h at RT, diluted with AcOEt and washed with brine. The organic phase was dried over sodium sulfate and the solvent was removed under reduced pressure. The crystalline crude material was triturated with AcOEt/Hexane to afford 2-chloro-4,5-dimethoxy-boronic acid (183 mg, 42%).

### Step 2

### 4-(2-Chloro-4,5-dimethoxy-phenyl)-2-methylsulfanyl-7H-pyrrolo[2,3-d]pyrimidine-5-carbonitrile

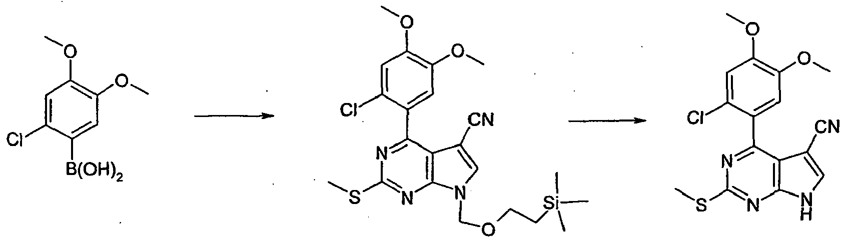

The title compound was prepared using the methods outlined in example 2 and the route outlined in scheme 2. Thus, 4-chloro-2-methylsulfanyl-7-(2-trimethylsilanyl-ethoxymethyl)-7H-pyrrolo[2,3-d]pyrimidine-5-carbonitrile was reacted with 2-chloro-4,5-dimethoxyphenyl boronic acid under Suzuki cross coupling reaction conditions. The SEM protecting group of the resulting product was removed with TBAF to afford a solid.
LC/MS: RT=2.24 Min; *m*/*z* = 361 [M+H]⁺. Total run time 3.75 mins.
¹H NMR (d₆ DMSO): δ 2.58 (s, 3H); 3.80 (s, 3H); 3.86 (s, 3H), 7.14 (s, 1H), 7.19 (s, 1H); 8.44 (s, 1H); 13.20 (brs, 1H).
This compound had activity 'A' in the fluorescence polarization assay described below.

### Example 30

### 4-(2-Chloro-4,5-dimethoxy-phenyl)-2-(2-diethylamino-ethylsulfanyl)-7H-pyrrolo[2,3-d]pyrimidine-5-carbonitrile

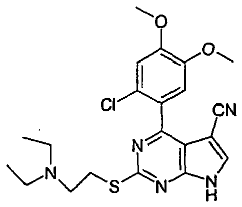

### Step 1

### 4-(2-Chloro-4,5-dimethoxy-phenyl)-2-methanesulfonyl-7-(2-trimethylsitanyl-ethoxymethyl)-7H-pyrrolo[2,3-d]pyrimidine-5-carbonitrile

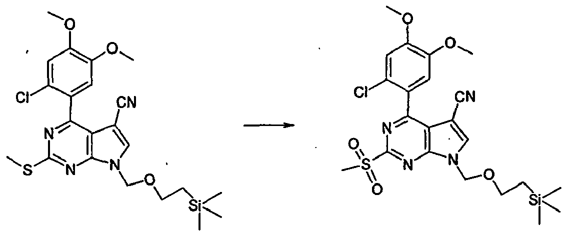

The title compound was prepared using the methods outlined in examples 12. Thus, 4-(2-chloro-4,5-dimethoxy-phenyl)-2-methylsulfanyl-7-(2-trimethylsilanyl-ethoxymethyl)-7H-pyrrolo[2,3-d]pyrimidine-5-carbonitrile (example 29) was oxidised with mcpba to afford the title compound as a light brown solid.
LC/MS: RT=2.588 min; *m*/*z* = 523, 525 [M+H]⁺. Total run time 3.75 mins.

### Step 2

### 4-(2-Chloro-4,5-dimethoxy-phenyl)-2-(2-diethylamino-ethylsulfanyl)-7H-pyrrolo[2,3-d]pyrimidine-5-carbonitrile

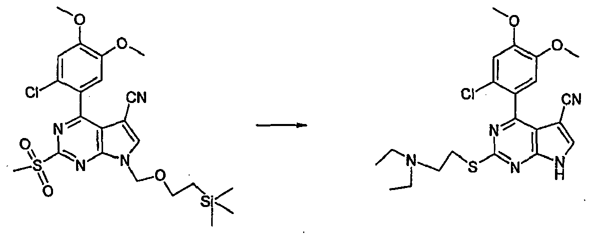

The title compound was prepared by way of the methods of example 19 step 1. Thus 4-(2-Chloro-4,5-dimethoxy-phenyl)-2-methanesulfonyl-7-(2-trimethylsilanyl-ethoxymethyl)-7H-pyrrolo[2,3-d]pyrimidine-5-carbonitrile was reacted with 2-diethylaminoethanethiol. The crude product from this reaction was de protected by way of the method of example 1 step 5, to afford product as a colourless solid following purification by flash chromatography (Silica gel; eluting with ethyl acetate / hexane mixture).
LC/MS: RT=1.68 Min; *m*/*z* = 446 [M+H]⁺. Total run time 3.75 mins.
¹H NMR (d₆ DMSO): δ 0.97 (t, 6H, J = 7.1 Hz); 2.57-2.68 (m, 4H); 2.82-2.90 (brm, 2H,); 3.25-3.35 (brm, 4H), 3.80 (s, 3H); 3.86 (s, 3H) 7.16 (s, 1H), 7.19 (s, 1H); 8.44 (s, 1H);
This compound had activity'A' in the fluorescence polarization assay described below.

### Example 31

### 4-(2-Chloro-4,5-dimethoxy-phenyl)-2-[2-(4,4-difluoro-piperidin-1-yl)-ethoxy]-7H-pyrrolo[2,3-d]pyrimidine-5-carbonitrile

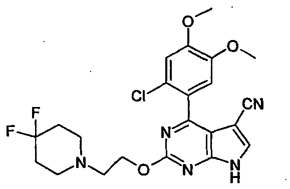

### Step 1

### Acetic acid-2-(4,4-difluoro-piperidin-1-yl)-2-oxo-ethyl ester

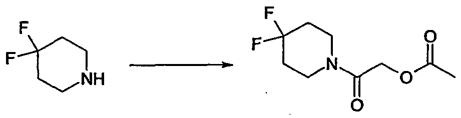

4,4-Difluoropiperidine hydrochloride (600mg, 3.8mmol) was stirred in DCM (10ml) with Et₃N (11.4mmol, 1.151 g, 1.59ml) and this mixture was cooled to 0°C. Acetoxy acetyl chloride (5.7mmol, 778mg, 0.612ml) in DCM (5ml) was added drop-wise and the reaction mixture was stirred overnight at RT. The reaction mixture was washed sequentially with saturated NaHCO₃ solution (x2) and brine(x2). The organic layer was dried (MgSO4), filtered, and the filtrate solvent was removed *in vacuo*. The residue was cooled and triturated with hexane to produce the title compound as a colourless oil, (773mg (92%).

### Step 2

### 2-(4,4-difluoro-piperidinyl-1-yl)ethanol

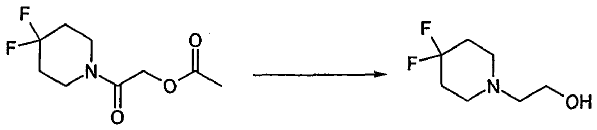

LiAlH₄ (15mmol, 15ml of a 1M solution in THF) was stirred in THF (20ml) at RT. Acetic acid-2-(4,4-difluoro-piperidin-1-yl)-2-oxo-ethyl ester (5mmol, 1.1g) in THF (15ml) was added drop-wise. After addition was complete, the reaction was heated to 40°C and held there for 4hrs. The reaction was stirred overnight at RT, and then cooled to 0°C. The reaction mixture was quenched by the careful addition of H₂O (2ml), aqueous 1 M NaOH soln. (1ml) and H₂O (1ml). The mixture was stirred for 30 mins and then filtered through celite, the filter cake being washed through several times with EtOAc. The filtrate was concentrated *in vacuo* to yield the title compound, 800mg (95%).

### Step 3

### 4-(2-Chloro-4,5-dimethoxy-phenyl)-2-[2-(4,4-difluoro-piperidin-1-yl)-ethoxy]-7-(2-trimethylsilylanyl-ethoxymethyl)-7H-pyrrolo[2,3-d]pyrimidine-5-carbonitrile

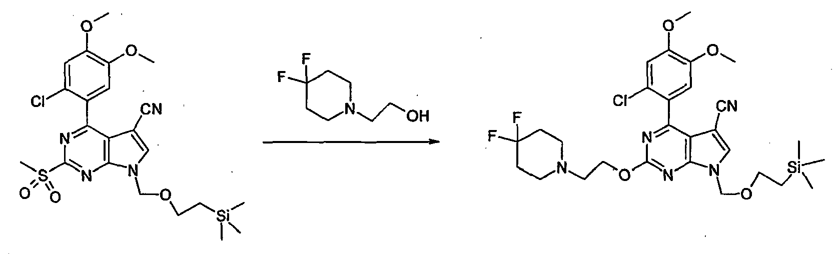

The title compound was synthesised by way of the methods used in example 12 step 2 using 4-(2-Chloro-4,5-dimethoxy-phenyl)-2-methanesulfonyl-7-(2-trimethylsilanyl-ethoxymethyl)-7H-pyrrolo[2,3-d]pyrimidine-5-carbonitrile (example 30, step 1) and 2-(4,4-difluoro-piperidinyl-1-yl)ethanol. This affords a crude product which was purified by flash chromatography on silica gel eluting with 1:1 ethyl acetate : hexane to afford product as an off-white solid (90% yield).
LC/MS: RT=2.33 min; *m*/*z* = 608,610 [M+H]⁺. Total run time 3.75 mins.

### Step 4

### 4-(2-Chloro-4,5-dimethoxy-phenyl)-2-[2-(4,4-difluoro-piperidin-1-yl)-ethoxy]-7H-pyrrolo[2,3-d]pyrimidine-5-carbonitrile

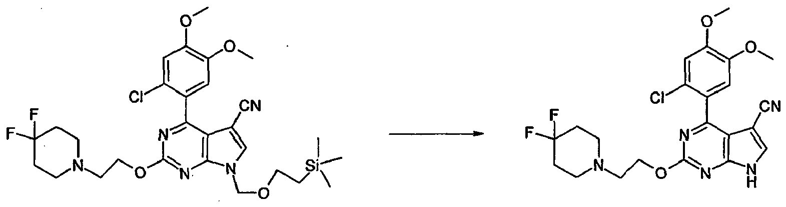

The title compound was made by way of the method of example 1 step 5. Thus 4-(2-Chloro-4,5-dimethoxy-phenyl)-2-[2-(4,4-difluoro-piperidin-1-yl)-ethoxy]-7-(2-trimethylsilylanyl-ethoxymethyl)-7H-pyrrolo[2,3-d]pyrimidine-5-carbonitrile was reacted with TBAF and ethylene diamine in THF to a afford a crude product which was purified by flash chromatography on silica gel, eluting with gradient of 1 to 5% Methanol in dichloromethane to afford product as off white solid. (39% yield).
LC/MS: RT=1.646 min; *m*/*z* = 478,480 [M+H]⁺. Total run time 3.75 mins.
¹H NMR (d₆ DMSO): δ 1.87-2.00 (m, 4H); 2.59-2.68 (m, 4H); 2.83 (brt, 2H, J = 5.3 Hz); 3.74 (s, 3H); 3.86 (s, 3H), 4.46 (brt, 2H, J = 5.3 Hz); 7.13 (s, 1H), 7.19 (s, 1H); 8.37 (s, 1H); 13.02 (brs, 1H).
This compound had activity 'A' in the fluorescence polarization assay described below.

### Example 32

### 4-(4,5-Dimethoxy-phenyl)-2-[2-(4,4-difluoro-piperidin-1-yl)-ethoxy]-7H-pyrrolo[2,3-d]pyrimidine-5-carbonitrile

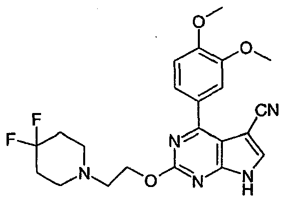

This compound was made by way of routes outlined in scheme 2 and scheme 4, utilizing methods of examples 2, 12 and 31, using appropriate boronic acids for coupling and alcohols for sulphone displacement. Final product was purified by flash chromatography on silica gel eluting with gradient of 0 to 5% Methanol in dichloromethane to afford product as off-white solid.
LC/MS: RT=1.583 min; *m*/*z* = 444 [M+H]⁺. Total run time 3.75 mins.
¹H NMR (d₆ DMSO): δ 1.88-2.00 (m, 4H); 2.59-2.65 (m, 4H); 2.82 (t, 2H, J = 5.6 Hz); 3.85 (s, 3H); 3.89 (s, 3H), 4.48 (t, 2H, J = 5.6 Hz); 7.14 (d, 1H, J = 8.7 Hz), 7.46-7.51 (m, 2H); 8.42 (s, 1H); 12.99 (brs, 1H).
This compound had activity 'A' in the fluorescence polarization assay described below.

### Example 33

### 2-[2-(3,3-Difluoro-pyrrolidin-1-yl)-ethoxy]-4-(3,4-dimethoxy-phenyl)-7H-pyrrolo[2,3-d]pyrimidine-5-carbonitrile

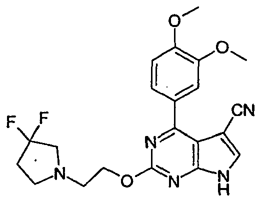

This compound was made by way of routes outlined in scheme 2 and scheme 4, utilizing methods of examples 2, 12 and 31, using appropriate boronic acids for coupling and alcohols for sulphone displacement. Final product was purified by Prep HPLC (pH4) to afford a colorless solid.
LC/MS: RT=1.736 min; *m*/*z* = 430 [M+H]⁺. Total run time 3.75 mins.
¹H NMR (d₆ DMSO): δ 2.17-2.28 (m, 2H); 2.80 (t, 2H, J = 6.8 Hz); 2.88 (t, 3H, J = 5.6 Hz); 3.00 (t, 2H, J = 13.5 Hz); 3.85 (s, 3H); 3.90 (s, 3H); 4.47 (t, 2H, J = 5.6 Hz); 7.14 (s, 1H); 7.46-7.51 (m, 2H), 8.42 (s, 1H), 13.00 (brs 1H).
This compound had activity 'B' in the fluorescence polarization assay described below.

### Example 34

### 4-(3,4-Dimethoxy-phenyl)-2-[2-(3(S)-fluoro-pyrrolidin-1-yl)-ethoxy]-7H-pyrrolo[2,3-d]pyrimidine-5-carbonitrile

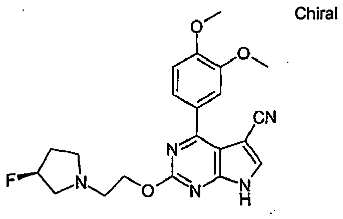

This compound was made by way of routes outlined in scheme 2 and scheme 4, utilizing methods of examples 2, 12 and 31, using appropriate boronic acids for coupling and alcohols for sulphone displacement. Final product was purified by flash chromatography on silica gel eluting with gradient of 0 to 5% Methanol in dichloromethane to afford product as white solid.
LC/MS: RT=1.474 min; *m*/*z* = 412 [M+H]⁺. Total run time 3.75 mins.
¹H NMR (d₆ DMSO): δ 1.78-1.94 (m, 1H), 2.02-2.21 (m, 1H), 2.35-2.47 (m, 1H); 2.63-2.75 (m, 1H), 2.80-2.95 (m, 4H), 3.85 (s, 3H), 3.90 (s, 3H), 4.47 (t, 2H, J = 5.8 Hz); 5.49 (dm, 1H); 7.14 (d, 1H, J = 8.3 Hz); 7.49 (m, 2H), 8.42 (s, 1H); 13.01 (brs, 1H).

This compound had activity 'B' in the fluorescence polarization assay described below.

### Example 35

### 4-(2-Chloro-4,6-dimethoxy-phenyl)-2-[2-(3(S)-fluoro-piperidin-1-yl)-ethoxy]-7H-pyrrolo[2,3-d]pyrimidine-5-carbonitrile

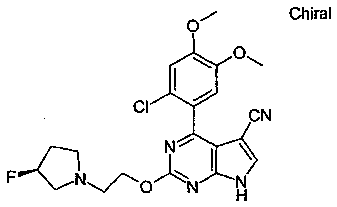

This compound was made by way of routes outlined in scheme 2 and scheme 4, utilizing methods of examples 2, 12 and 31, using appropriate boronic acids for coupling and alcohols for sulphone displacement. Final product was purified by flash chromatography on silica gel eluting with gradient of 3 to 5% Methanol in dichloromethane to afford product as white solid.
LC/MS: RT=1.502 min; *m*/*z =* 446 [M+H]⁺. Total run time 3.75 mins.
¹H NMR (d₆ DMSO): δ 1.76-1.93 (m, 1H), 2.04-2.19 (m, 1H), 2.40 (m, 1H), 2.62-2.77 (m, 1H), 2.80-2.88 (m, 4H), 3.79 (s, 3H), 3.86 (s, 3H), 4.46 (t, 2H, J = 5.8 Hz); 5.20 (dm, 1H); 7.13 (s, 1H), 7.18 (s, 1H), 8.35 (s, 1H); 13.00 (brs, 1H).
This compound had activity 'A' in the fluorescence polarization assay described below.

### Example 36

### 2-[2-(3,3-Difluoro-pyrrolidin-1-yl)-ethoxy]-4-(2-Chloro-3,4-dimethoxy-phenyl)-7H-pyrrolo[2,3-d]pyrimidine-5-carbonitrile

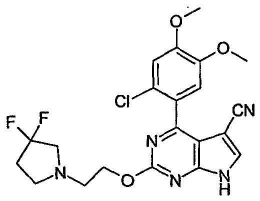

This compound was made by way of routes outlined in scheme 2 and scheme 4, utilizing methods of examples 2, 12 and 31, using appropriate boronic acids for coupling and alcohols for sulphone displacement Final product was purified by flash chromatography on silica gel eluting with gradient of 0 to 3% methanol in dichloromethane to afford product as white solid.
LC/MS: RT=1.816 min; *m*/*z* = 464 [M+H]⁺. Total run time 3.75 mins.
¹H NMR (d₆ DMSO): δ 2.17-2.28 (m, 2H); 2.80 (t, 2H, J = 6.7 Hz); 2.88 (t, 3H, J = 5.6 Hz); 2.99 (t, 2H, J = 13.4 Hz); 3.74 (s, 3H); 3.86 (s, 3H); 4.40 (t, 2H, J = 5.6 Hz); 7.14 (s, 1H); 7.18 (s, 1H), 8.36 (s, 1H), 13.00 (brs 1H).
This compound had activity 'A' in the fluorescence polarization assay described below.

### Example 37

### 4-(2-Chloro-4-cyano-5-methoxy-phenyl)-2-(2-diethylamino-ethylsulfanyl)-7H-pyrrolo[2,3-d]pyrimidine-5-carbonitrile

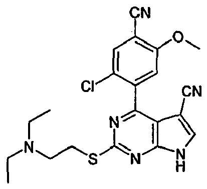

### Step 1

### 4-Amino-5-chloro-2-methoxybenzamide

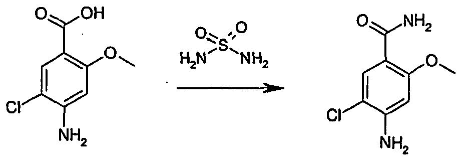

4-amino-5-chloro-2-methoxybenzoic acid (*commercially available*) (600mg, 2.98 mmol) was added to sulfamide (715 mg; 7.44 mmol, 2.5 equiv) and the mixture was then dissolved in pyridine (2.9 ml) and heated under nitrogen atmosphere for 2.5 hours. Reaction mixture was allowed to cool to ambient temperature and the pyridine was removed in vacuo. The resulting solids were washed with 10% MeOH in dichloromethane. Filtered and dried to afford a cream solid 550mg; 92%.

### Step 2

### 4-Amino-5-chloro-2-methoxybenzonitrile

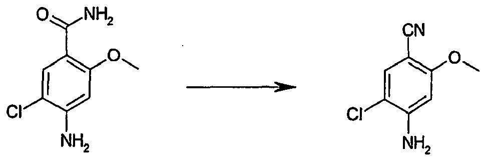

4-Amino-5-chloro-2-methoxybenzamide was added to acetonitrile) and POCl₃ (excess) was added to the resulting suspension and this mixture heated to 80°C for 3 hours (reaction mixture was homogeneous after 1.5 hours). Reaction mixture was allowed to cool to room temperature, then poured into ice water. After stirring for 2 hours a yellow solid was filtered off, this was dried overnight at 50°C.

### Step 3

### 4-lodo-5-chloro-2-methoxybenzonitrile

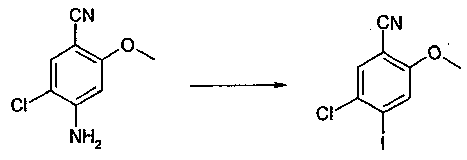

The title compound was made by way of method of example 22 step 3 (diazotization and quench with aqueous Iodine / sodium iodide solution).

### Step 4

### 4-(2-Chloro-4-cyano-5-methoxy-phenyl)-2-methylsulfanyl-7-(2-trimethylsilanyl-ethoxymethyl)-7H-pyrrolo[2,3-d]pyrimidine-5-carbonitrile

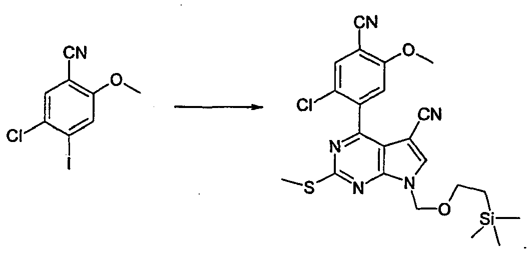

The title compound was prepared by way of the method of example 26 step 1 (boronic acid formation and subsequent cross coupling).
LC/MS: RT=2.884 min; *m*/*z* = 486, 488 [M+H]⁺. Total run time 3.75 mins.

### Step 5

### 4-(2-Chloro-4-cyano-5-methoxy-phenyl)-2-methanesulfonyl-7-(2-trimethylsilanyl-ethoxymethyl)-7H-pyrrolo[2,3-d]pyrimidine-5-carbonitrile

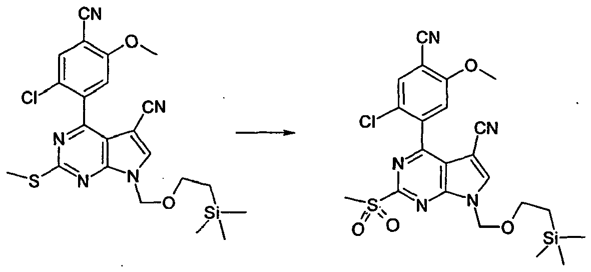

The title compound was prepared by way of the method of example 22 step 1 (oxidation with mcpba).

### Step 6

### 4-(2-Chloro-4-cyano-5-methoxy-phenyl)-2-(2-diethylamino-ethylsulfanyl)-7H-pyrrolo[2,3-d]pyrimidine-5-carbonitrile

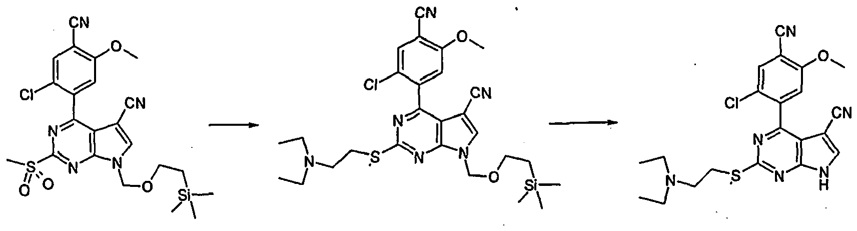

The title compound was prepared using the route outlined in scheme 4, and the methods of example 19 (sulphone displacement) and example 1 step 5 (deprotection).
LC/MS: RT=1.75 min; *m*/*z* = 457 [M+H]⁺. Total run time 3.75 mins.
¹H NMR (d₆ DMSO): δ 1.02 (t, 6H, J = 7.1 Hz); 2.72-2.81 (brm, 4H); 2.94-3.03 (brm, 4H), 3.26-3.37 (brm, 4H); 3.96 (s, 3H); 7.56 (s, 1H); 8.19 (s, 1H), 8.51 (s, 1H).
This compound had activity 'A' in the fluorescence polarization assay described below.

### Example 38

### 2-[5-Cyano-4-(4-fluoro-2-methyl-phenyl)-7H-pyrrolo[2,3-d]pyrimidin-2-ylsulfanyl]-N-methyl-acetamide

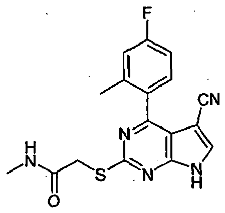

The title compound was prepared using the methods out lined in example 12, example 19, and the route outlined in scheme 2 and scheme 4.
LC/MS: RT=2.01 min; *m*/*z* = 356 [M+H]⁺. Total run time 3.75 mins.
This compound had activity 'A' in the fluorescence polarization assay described below.

### Example 39

### 4-(2,4-Dimethy-phenyl)-7H-pyrrolo[2,3-d]pyrimidine-5-carboxylic acid amide

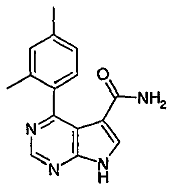

### Step 1

### 4-(2,4-Dimethy-phenyl)-7H-pyrrolo[2,3-d]pyrimidine-5-carboxylic acid amide

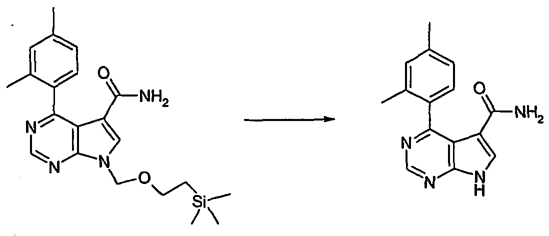

4-(2,4-Dimethyl-phenyl)-7-(2-trimethylsilanyl-ethoxymethyl)-7H-pyrrolo[2,3-d]pyrimidine-5-carboxylic acid amide (example 9 step 8) was de-protected using the method of example 1 step 5. Thus reaction with TBAF and ethylenediamine in THF afforded crude product which was purified by preparative HPLC (pH4) to give title compound as colorless solid.
LC/MS: RT=1.987 min; *m*/*z* = 267 [M+H]⁺. Total run time 7.5 mins.
¹H NMR (d₆ DMSO): δ 1.91 (s, 3H); 2.33 (s, 3H); 6.81 (brs, 1H); 7.05 (d, 1H, J=7.7 Hz); 7.07 (s, 1H); 7.12 (d, 1H, J=7.7 Hz); 7.97 (s, 1H); 8.83 (s, 1H).
This compound had activity'C' in the fluorescence polarization assay described below.

### Example 40

### 4-(4-Fluoro-2-methy-phenyl)-2-methylsulfanyl-7H-pyrrolo[2,3-d]pyrimidine-5-carboxylic acid amide

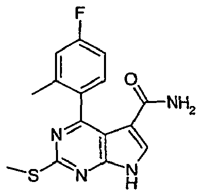

The title compound was synthesized by the routes outlined in scheme 2, utilizing the methods of example 2 and example 39.

### Step 1

### 4-[(2-methyl-4-fluoro-phenyl]-2-methylsulfanyl-7-(2-trimethylsilanyl-ethoxymethyl)-7H-pyrrolo[2,3-d]pyrimidine-5-carboxylic acid amide

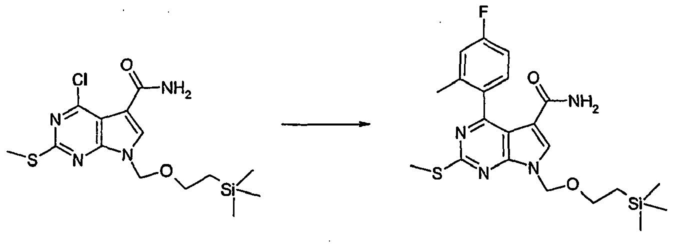

4-chloro-2-methylsulfanyl-7-(2-trimethylsilanyl-ethoxymethyl)-7H-pyrrolo[2,3-d]pyrimidine-5-carboxylic acid amide (example 2, step 3) was cross coupled with 4-fluoro-2-methylphenyl boronic acid, by way of the method of example 2 step 5. This afforded crude product which was purified by flash chromatography on silica gel, eluting with 20 to 65% ethyl acetate in hexane (gradient); affording title compound as colourless oil (90% yield).
LC/MS: RT= 2.676 min; *m*/*z* = 447 [M+H]⁺. Total run time 3.75 mins.

### Step 2

### 4-(4-Fluoro-2-methy-phenyl)-2-methylsulfanyl-7H-pyrrolo[2,3-d]pyrimidine-5-carboxylic acid amide

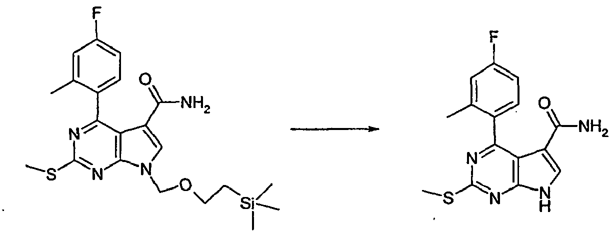

The title compound was prepared using the methods of example 1 step 5. Thus reaction with TBAF and ethylenediamine in THF afforded crude product which was purified by flash chromatography on silica gel, eluting with 0 to 4% methanol in dichloromethane (gradient) afforded title compound as a colorless solid.
LC/MS: RT= 1.920 min; *m*/*z* = 317 [M+H]⁺. Total run time 3.75 mins.
¹H NMR (d₆ DMSO): δ 2.56 (s, 3H); 6.68 (brs, 1H); 6.98-7.11 (m, 3H); 7.19-7.24 (m, 1H); 7.84 (d, 1H, J = 2.3 Hz).
This compound had activity'C' in the fluorescence polarization assay described below.

### Example 41

### 4-(Dichloro-phenyl)-2-methylsulfanyl-7H-pyrrolo[2,3-d]pyrimidine-5-carbonitrile

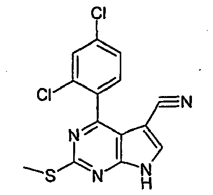

The title compound was made by way of the route outlined in scheme 2 and the methods of example 2. Thus 4-chloro-2-methylsulfanyl-7-(2-trimethylsilanyl-ethoxymethyl)-7H-pyrrolo[2,3-d]pyrimidine-5-carbonitrile was reacted with 2,4 dichlorophenyl boronic acid and the resulting product de-protected with TBAF and ethylene diamine in THF. Final product was purified by Preparative HPLC (pH4) to afford title compound as an off-white solid.
LC/MS: RT= 2.511 min; *m*/*z* = 335, 337 [M+H]⁺. Total run time 3.75 mins.
¹H NMR (d₆ DMSO): δ 2.58 (s, 3H); 7.62 (m, 2H); 8.86 (m, 1H); 8.49 (s, 1H).
This compound had activity 'A' in the fluorescence polarization assay described below.

### Example 42

### 4-(2-Chloro-4-cyano-5-methoxy)-2-methylsulfanyl-7H-pyrrolo[2,3-d]pyrimidine-6-carbonitrile

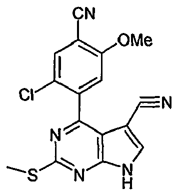

4-(2-Chloro-4-cyano-5-methoxy-phenyl)-2-methylsulfanyl-7-(2-trimethylsilanyl-ethoxymethyl)-7H-pyrrolo[2,3-d]pyrimidine-5-carbonitrile (example 37 step 4) was deprotected by way of the method of example 1 step 5. Purification by flash chromatography on silica gel eluting 20-50% ethyl acetate in hexane to afford product as a solid.
LC/MS: RT= 2.33 min; *m*/*z* = 356 [M+H]⁺. Total run time 3.75 mins.
¹H NMR (d₆ DMSO): δ 2.59 (s, 3H); 3.96 (s, 3H); 7.54 (s, 1H); 8.19 (s, 1H); 8.51 (s, 1H). This compound had activity 'A' in the fluorescence polarization assay described below.

### Example 43

### 4-(2,3-Dihydro-benzo[1,4]dioxin-6-yl)-2-methylsulfanyl-7H-pyrrolo[2,3-d]pyrimidine-5-carbonitrile

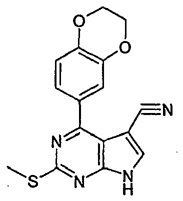

The title compound was made by way of the route outlined in scheme 2 and the methods of example 2. Thus 4-chloro-2-methylsulfanyl-7-(2-trimethylsilanyl-ethoxymethyl)-7H-pyrrolo[2,3-d]pyrimidine-5-carbonitrile was reacted with 1,4-benzodioxane-6-boronic acid and the resulting product de-protected with TBAF and ethylenediamine in THF. Final product was purified by flash chromatography on silica gel, eluting 1:1 ethyl acetate in hexane to afford title compound as a solid.
LC/MS: RT= 2.26 min; *m*/*z* = 325 [M+H]⁺. Total run time 3.75 mins.
¹H NMR (d₆ DMSO): δ 2.58 (s, 3H); 4.30-4.37 (m, 4H); 7.03 (dd, 1H, J = 7.1, 1.3 Hz);7.39 (s, 1H); 7.40 (dd, 1H, J = 7.1, 2.4 Hz).
This compound had activity 'C' in the fluorescence polarization assay described below.

### Example 44

### 4-(Dimethoxy-phenyl)-2-methylsulfanyl-7H-pyrrolo[2,3-d]pyrimidine-5-carbonitrile

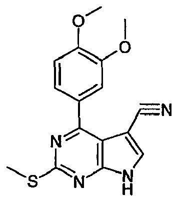

The title compound was made by way of the route outlined in scheme 2 and the methods of example 2. Thus 4-chloro-2-methylsulfanyl-7-(2-trimethylsilanyl-ethoxymethyl)-7H-pyrrolo[2,3-d]pyrimidine-5-carbonitrile was reacted with 3,4- dimethoxyphenyl boronic acid and the resulting product de-protected with TBAF and ethylenediamine in THF. Final product was purified by flash chromatography on silica gel, eluting 1:1 ethyl acetate in hexane to afford title compound as a solid.
LC/MS: RT= 2.17 min; *m*/*z* = 327 [M+H]⁺. Total run time 3.75 mins.
¹H NMR (d₆ DMSO): δ 2.60 (s, 3H); 3.86 (s, 3H); 3.89 (s, 3H); 7.15 (d, 1H, J = 8.4 Hz); 7.46-7.51 (m, 2H); 8.50 (s, 1H).
This compound had activity 'A' in the fluorescence polarization assay described below.

### Example 45

### 4-[2-Chloro-5-(2-diethylamino-ethoxy)-4-methoxy-phenyl]-2-isopropylsulfanyl-7H-pyrrolo[2,3-d]pyrimidine-5-carbonitrile

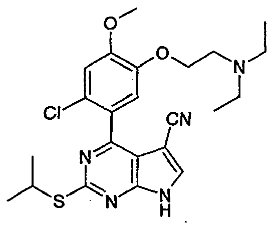

### Step 1

### 5-Bromo-2-methoxy-phenol

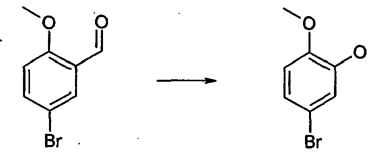

To a solution of 5-Bromo-2-methoxy-benzaldehyde (15 g, 69.8 mmol) in CH₂Cl₂ (200 ml), mCPBA (19.0 g, 82.4 mmol) was added and the resultant mixture stirred at RT for 48 h. This was then partitioned between CH₂Cl₂ (150 ml) and sat. NaHCO₃ solution (400 ml). The organic phase was dried (Na₂SO₄) and evaporated *in vacuo*. The residue was then dissolved in a minimum of EtOAc and passed through a plug of SiO₂ washing through with further EtOAc. The filtrate was evaporated *in vacuo* and redissolved in MeOH (50 ml). 1M LiOH aq. solution (50 ml) was added and the mixture stirred for 10 min. 2M HCl (aq) was then added cautiously to acidify the reaction mixture to pH 6-7.
This was extracted with EtOAc (3 x 100 ml) and the combined organics dried (Na₂SO₄) and evaporated *in vacuo*. The resultant crude product was purified by flash chromatography on SiO₂ eluting with Hexane then 10% EtOAc/Hexane to afford the title compound as a white solid, 10.21 g, 72%.
LC/MS: RT=2.11 Min; *m*/*z* = 201, 203 [M-H]. Total run time 3.75 mins.

### Step 2

### 5-Bromo-4-chloro-2-methoxy-phenol

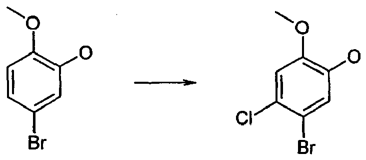

To a solution of 5-Bromo-2-methoxy-phenol (10.08 g, 49.66 mmol) in MeCN (110 ml), TFA (1.15 ml, 14.9 mmol) and NCS (7.29 g, 54.63 mmol) were added sequentially and the resultant mixture stirred at RT for 16 h. This was then partitioned between EtOAc (200 ml) and brine (400 ml). The organic phase was dried (Na₂SO₄) and evaporated *in vacuo*. The resultant crude product was purified by flash chromatography on SiO₂ eluting with Hexane then 10% EtOAc/Hexane to afford the title compound as a white solid, 10.5 g, 89%.
LC/MS: RT=2.28 Min; *m*/*z* = 235, 237 [M-H]. Total run time 3.75 mins.

### Step 3

### 1-Bromo-2-chloro-4-methoxy-5-methoxymethoxy-benzene

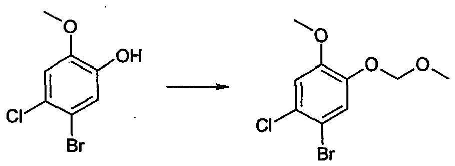

To a solution of 5-Bromo-4-chloro-2-methoxy-phenol (1.0 g, 4.21 mmol) in dimethoxymethane (28 ml) and CHCl₃ (28 ml) at 0°C under N₂ P₂O₅ (5.68 g, 40 mmol) was added in one portion. After 5 min. the reaction was allowed to warm to RT. The reaction mixture was then poured on to ice and extracted with CH₂Cl₂ (2 x 50 ml). The combined organic phases were dried (Na₂SO₄) and evaporated *in vacuo*. The resultant crude product was purified by flash chromatography on SiO₂ eluting with Hexane-10% EtOAc/Hexane (gradient) to afford the title compound as a white solid, 1.03 g, 87%.
LC/MS: RT=2.57 Min; no mass detected. Total run time 3.75 mins.

### Step 4

### 4-(2-Chloro-4-methoxy-5-methoxymethoxy-phenyl)-2-methylsulfanyl-7-(2-trimethylsilanyl-ethoxymethyl)-7H-pyrrolo[2,3-d]pyrimidine-5-carbonitrile

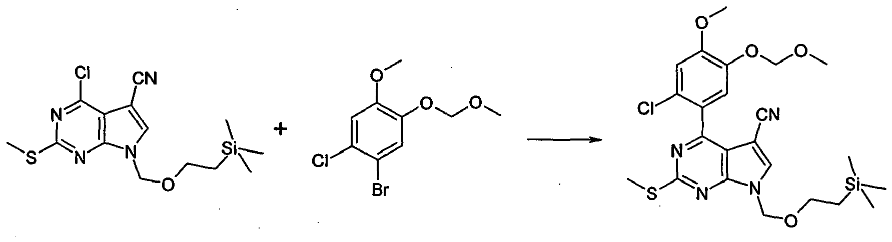

The title compound was prepared by the route outlined in scheme 2 and by the way of the methods of examples 2 and 22, using 1-Bromo-2-chloro-4-methoxy-5-methoxymethoxy-benzene and 4-chloro-2-methylsulfanyl-7-(2-trimethylsilanyl-ethoxymethyl)-7H-pyrrolo[2,3-d]pyrimidine-5-carbonitrile in the appropriate step (cross coupling).
LC/MS: RT=2.84 Min; *m*/*z* = 521, 523 [M+H]⁺. Total run time 3.75 mins.

### Step 5

### 4-(2-Chloro-4-methoxy-5-methoxymethoxy-phenyl)-2-methanesulfonyl-7-(2-trimethylsilanyl-ethoxymethyl)-7H-pyrrolo[2,3-d]pyrimidine-5-carbonitrile

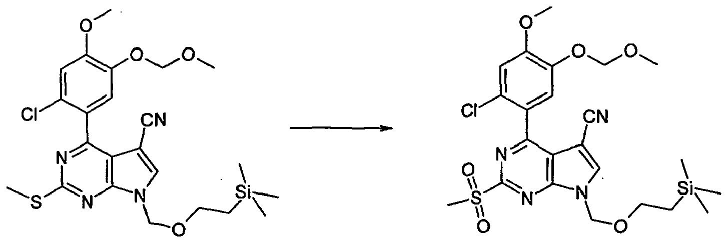

The title compound was prepared by the route outlined in scheme 4 and by the way of the methods of example 12 (step 1), using 4-(2-Chloro-4-methoxy-5-methoxymethoxyphenyl)-2-methylsulfanyl-7-(2-trimethylsilanyl-ethoxymethyl)-7H-pyrrolo[2,3-d]pyrimidine-5-carbonitrile.
LC/MS: RT=2.62 Min; *m*/*z* = 553, 555 [M+H]⁺. Total run time 3.75 mins.

### Step 6

### 4-(2-Chloro-4-methoxy-5-methoxymethoxy-phenyl)-2-isopropylsulfanyl-7-(2-trimethylsilanyl-ethoxymethyl)-7H-pyrrolo[2,3-d]pyrimidine-5-carbonitrile

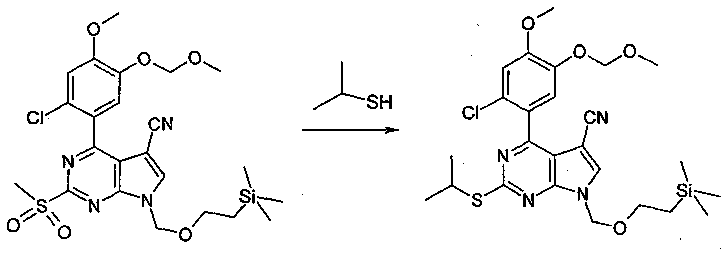

The title compound was prepared by the route outlined in scheme 4 and by the way of the methods of example 12 step 2, using 4-(2-Chloro-4-methoxy-5-methoxymethoxyphenyl)-2-methanesulfonyl-7-(2-trimethylsilanyl-ethoxymethyl)-7H-pyrrolo[2,3-d]pyrimidine-5-carbonitrile in the appropriate step (Nucleophilic displacement).
LC/MS: RT=2.96 Min; *m*/*z* = 549, 551 [M+H]⁺. Total run time 3.75 mins.

### Step 7

### 4-(2-Chloro-5-hydroxy-4-methoxy-phenyl)-2-isopropylsulfanyl-7-(2-trimethylsilanyl-ethoxymethyl)-7H-pyrrolo[2,3-d]pyrimidine-5-carbonitrile

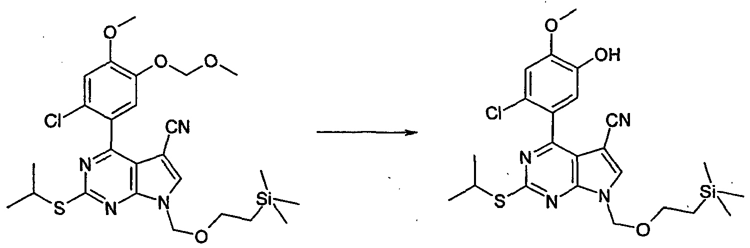

4-(2-Chloro-4-methoxy-5-methoxymethoxy-phenyl)-2-isopropylsulfanyl-7-(2-trimethylsilanyl-ethoxymethyl)-7H-pyrrolo[2,3-d]pyrimidine-5-carbonitrile (94 mg, 0.17 mmol), pyridinium p-toluenesulfonate (9 mg, 0.034 mmol) and *ⁱ*PrOH were combined under N₂ and heated at 85°C for 5 h. The reaction was allowed to cool and partitioned between EtOAc (20 ml) and brine (20 ml). The organic phase was dried (Na₂SO₄) and evaporated *in vacuo.* The resultant crude product was purified by flash chromatography on SiO₂ eluting with Hexane-30% EtOAc/Hexane (gradient) to afford the title compound as a white solid, 85 mg, 99%.
LC/MS: RT=2.86 Min; *m*/*z* = 505, 507 [M+H]⁺. Total run time 3.75 mins.

### Step 8

### 4-[2-Chloro-5-(2-diethylamino-ethoxy)-4-methoxy-phenyl]-2-isopropylsulfanyl-7-(2-trimethylsilanyl-ethoxymethyl)-7H-pyrrolo[2,3d]pyrimidine-5-carbonitrile

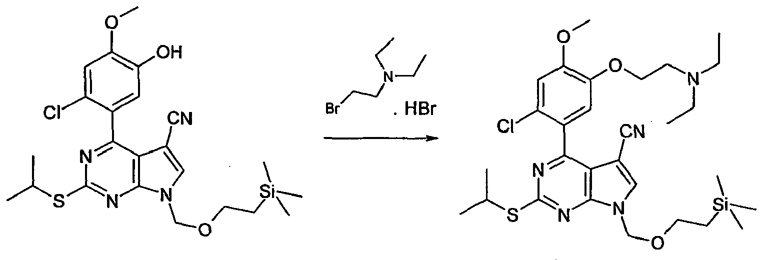

The title compound was prepared by the route outlined in scheme 5 and by the way of the methods of example 26 step 1, using 4-(2-Chloro-5-hydroxy-4-methoxy-phenyl)-2-isopropylsulfanyl-7-(2-trimethylsilanyl-ethoxymethyl)-7H-pyrrolo[2,3-d]pyrimidine-5-carbonitrile and (2-bromo-ethyl)-diethyl-amine in the appropriate step (alkylation).
LC/MS: RT=2.37 Min; *m*/*z* = 604, 606 **[M+H]⁺.** Total run time 3.75 mins.

### Step 9

### 4-[2-Chloro-5-(2-diethylamino-ethoxy)-4-methoxy-phenyl]-2-isopropylsulfanyl-7H-pyrrolo[2,3-d]pyrimidine-5-carbonitrile

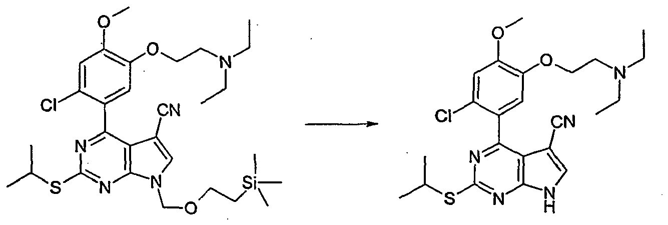

The title compound was prepared by the methods of example 1 step 5. using 4-[2-Chloro-5-(2-diethylamino-ethoxy)-4-methoxy-phenylj-2-isopropylsulfanyl-7-(2-trimethylsilanyl-ethoxymethyl)-7H-pyrrolo[2,3d]pyrimidine-5-carbonitrile and TBAF /ethylenediamine in THF.
LC/MS: RT=1.90 Min; *m*/*z* = 474, 476 [M+H]⁺. Total run time 3.75 mins.
¹H NMR (d₆ DMSO): δ 0.96 (t, 6H, J = 7.1 Hz); 1.41 (d, 6H, J = 6.8 Hz); 2.58 (q, 4H, J = 7.1 Hz), 2.84 (t, 2H, J = 6.0 Hz); 3.86 (s, 3H), 3.94 (sept, 1H, J = 6.8 Hz); 4.06 (t, 2H, J = 6.0 Hz); 7.18 (s, 1H), 7.19 (s, 1H); 8.43 (s, 1H); NH not observed.
This compound had activity 'A' in the fluorescence polarization assay described below.

### Example 46

### 4-(2-Chloro-4,5-dimethoxy-phenyl)-2-(2-diethylamino-ethanesulfinyl)-7H-pyrrolo[2,3-d]pyrimidine-5-carbonitrile

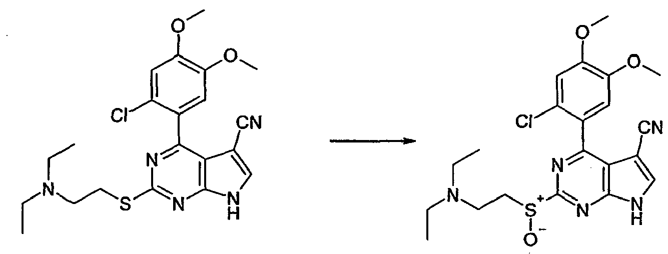

To a solution of 4-(2-Chloro-4,5-dimethoxy-phenyl)-2-(2-diethylamino-ethylsulfanyl)-7H-pyrrolo[2,3-d]pyrimidine-5-carbonitrile (example 30) (30 mg, 0.067 mmol) in MeCN at 0°C MeCN:BF₃ complex (0.85 ml, 16% BF₃ in MeCN)) was added drop wise. A solution of *m*CPBA (15 mg, ~0.067 mmol) in MeCN (0.5 ml) was then added slowly and the reaction allowed to warm to RT. After 1 h the reaction mixture was partitioned between EtOAc (15 ml) and sodium thiosulfate solution (15 ml). The organic phase was washed with sat. NaHCO₃ sol. (15 ml) dried (Na₂SO₄) and evaporated *in vacuo.* The resultant crude product was purified by preparative HPLC to afford the titled compound as a white solid, 2 mg, 6%.
LC/MS: RT=1.54 Min; *m*/*z* = 462, 464 [M+H]⁺. Total run time 3.75 mins.
This compound had activity 'B' in the fluorescence polarization assay described below.

### Example 47

### 4-(2-Chloro-4,5-dimethoxy-phenyl)-2-methanesulfinyl-7H-pyrrolo[2,3-d]pyrimidine-5-carbonitrile

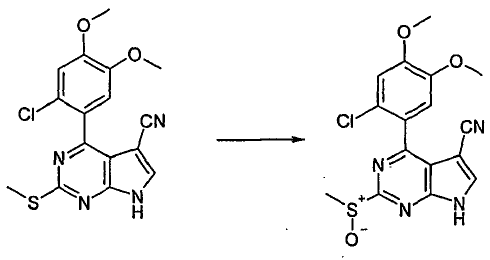

To a solution of 4-(2-Chloro-4,5-dimethoxy-phenyl)-2-methylsulfanyl-7H-pyrrolo[2,3-d]pyrimidine-5-carbonitrile (example 29) (50 mg, 0.139 mmol) in CH₂Cl₂ at 0°C a solution of *m*CPBA (31 mg, ~0.139 mmol) in CH₂Cl₂ (2.5 ml) was added and the reaction allowed to warm to RT. The reaction mixture was then partitioned between CH₂Cl₂ (2 x 15 ml) and sodium thiosulfate solution (15 ml). The organic phase was washed with sat. NaHCO₃ sol. (15 ml), dried (Na₂SO₄) and evaporated *in vacuo.* The resultant crude product was purified by flash chromatography on SiO₂ eluting with CH₂Cl₂ - 5% MeOH/CH₂Cl₂ (gradient) to afford the title compound as a white solid, 27 mg, 52%.
LC/MS: RT=1.81 Min; *m*/*z* = 377, 379 [M+H]⁺. Total run time 3.75 mins.
¹H NMR (d₆ DMSO): δ 2.94 (s, 3H); 3.81 (s, 3H); 3.88 (s, 3H); 7.24 (s, 1H); 7.25 (s, 2H); 8.77 (s, 1H); 13.8 (brs, 1H).
This compound had activity 'B' in the fluorescence polarization assay described below.

### Example 48

### 4-(2-Chloro-4,5-dimethoxy-phenyl)-2-methanesulfonyl-7H-pyrrolo[2,3-d]pyrimidine-5-carbonitrile

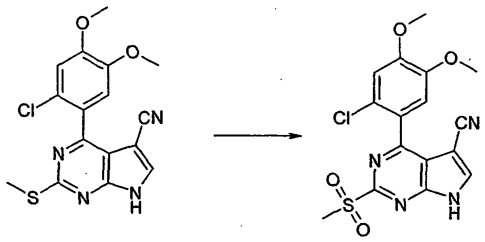

The title compound was prepared by the route outlined in scheme 4 and by the way of the methods of example 12 (step 1), using 4-(2-chloro-4,5-dimethoxy-phenyl)-2-methylsulfanyl-7H-pyrrolo[2,3-d]pyrimidine-5-carbonitrile (example 29) in the appropriate step (oxidation).
LC/MS: RT=1.97 Min; *m*/*z* = 393, 395 [M+H]⁺. Total run time 3.75 mins.
¹H NMR (d₆ DMSO): δ 3.49 (s, 3H); 3.87 (s, 3H); 3.89 (s, 3H); 7.26 (s, 1H); 7.27 (s, 2H); 8.90 (s, 1H); 14.0 (brs, 1H).
This compound had activity 'B' in the fluorescence polarization assay described below.

### Example 49

### (4-{2-Chloro-5-[2-(3,3-difluoro-pyrrolidin-1-yl)-ethoxy]-4-methoxy-phenyl}-5-cyano-7H-pyrrolo[2,3-d]pyrimidin-2-ylsulfanyl)-acetic acid ethyl ester.

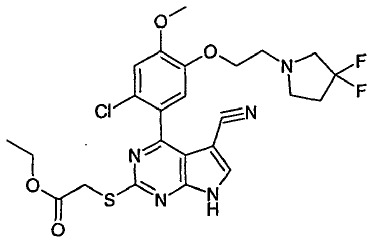

### Step 1

### [4-(2-Chloro-4-methoxy-5-methoxymethoxy-phenyl)-5-cyano-7-(2-trimethylsilanyl-ethoxymethyl)-7H-pyrrolo[2,3-d]pyrimidin-2-ylsulfanyl]-acetic acid ethyl ester.

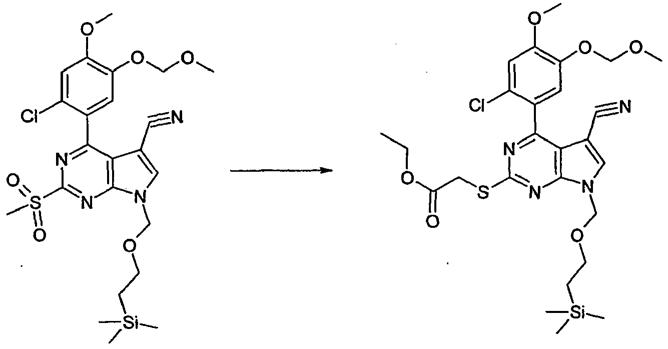

The title compound was prepared by the routes outlined in scheme 2 and 4 and by the way of the methods of example 12 (step 2), using Ethyl thioglycolate, sodium hydride and 4-(2-Chloro-4-methoxy-5-methoxymethoxy-phenyl)-2-methanesulfonyl-7-(2-trimethylsilanyl-ethoxymethyl)-7H-pyrrolo[2,3-d]pyrimidine-5-carbonitrile (example 45, step 6). The resultant crude product was purified by flash chromatography on SiO₂ eluting with Hexane then 30% EtOAc/Hexane to afford the title compound as an oil, 240mg, 81%.
LC/MS: RT=2.82 Min (270nm); *m*/*z* = 593, 595 [M+H]⁺. Total run time 3.75 min (short pos).

### Step 2

### [4-(2-Chloro-5-hydroxy-4-methoxy-phenyl)-6-cyano-7-(2-trimethylsilanyl-ethoxymethyl)-7H-pyrrolo[2,3-d]pyrimidin-2-ylsulfanyl]-acetic acid ethyl ester.

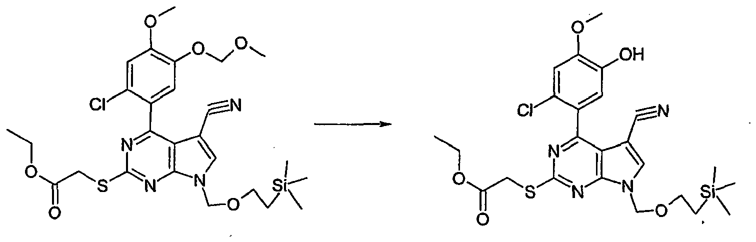

The title compound was prepared by the way of the methods of example 45 step 7, using [4-(2-Chloro-4-methoxy-5-methoxymethoxy-phenyl)-5-cyano-7-(2-trimethylsilanyl-ethoxymethyl)-7H-pyrrolo[2,3-d]pyrimidin-2-ylsulfanyl]-acetic acid ethyl ester and pyridine p-toluenesulfonate in the appropriate step (MOM deprotection). After a full aqueous work up the title compound was isolated as a cream-coloured foam and used without further purification, 172mg, 81 %.
LC/MS: RT=2.73 Min (270nm); *m*/*z* = 549, 551 [M+H]⁺. Total run time 3.75 min (short pos).

### Step 3

### [4-{2-Chloro-5-[2-(3,3-difluoro-pyrrolidin-1-yl)-ethoxy]-4-methoxy-phenyl}-5-cyano-7-(2-trimethylsilanyl-ethoxymethyl)-1H-pyrrolo[2,3-d]pyrimidin-2-ylsulfanyl]-acetic acid ethyl ester.

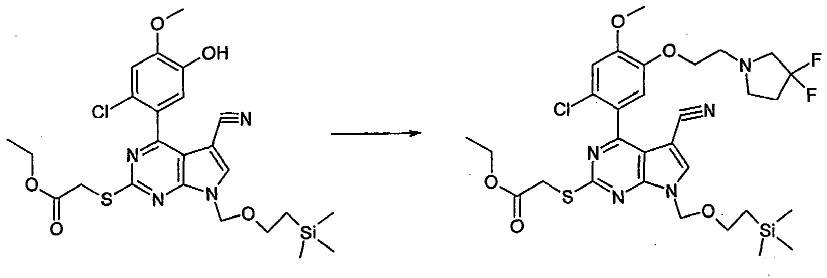

To a solution of compound 2 (164mg, 0.298mmol) in THF (5 ml) was added triphenylphosphine (118mg, 0.448mmol) and 2-(3,3-difluoro-pyrrolidin-1-yl)-ethanol *(prepared as for 2-(4,4-difluoro-piperidinyl)-ethanol example 32 step 1 and 2*) (68mg, 0.448mmol). The reaction was stirred at RT for 15mins and then cooled to 0°C. Diisopropyl azodicarboxylate (91 mg, 0.448mmol) in THF (3ml) was added drop wise and after addition the reaction was allowed to attain RT over 15mins. The reaction mixture was stirred 18hrs at RT and then partitioned between EtOAc and water. The organic layer was separated and the aqueous extracted with a further portion of EtOAc and these combined organic layers were washed successively with saturated sodium bicarbonate solution and saturated brine solution. The organics were dried (Na₂SO₄) and evaporated *in vacuo.* The resultant crude product was purified by flash chromatography on SiO₂ eluting with 30% EtOAc/Hexane - 50% EtOAc/Hexane (gradient) to afford the title compound as an oil, 120mg, 60%.
LC/MS: RT=2.79 Min (270nm); *m*/*z* = 682, 684 [M+H]⁺. Total run time 3.75 min (short pos).

### Step 4

### (4-{2-Chloro-5-[2-(3,3-difluoro-pyrrolidin-1-yl)-ethoxy]-4-methoxy-phenyl}-5-cyano-7H-pyrrolo[2,3-d]pyrimidin-2-ylsulfanyl)-acetic acid ethyl ester.

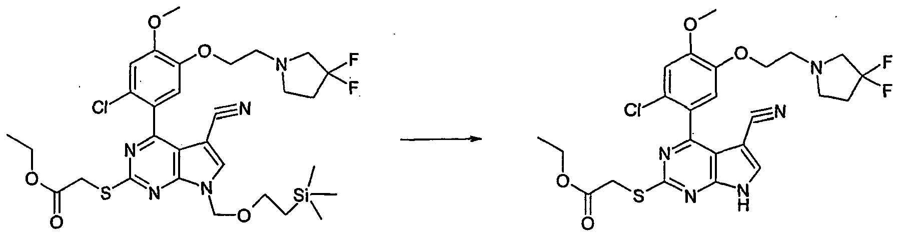

The title compound was prepared by way of the method of example 1 step 5, using [4-{2-chloro-5-[2-(3,3-difluoro-pyrrolidin-1-yl)-ethoxy]-4-methoxy-phenyl}-5-cyano-7-(2-trimethylsilanyl-ethoxymethyl)-7H-pyrrolo[2,3-d]pyrimidin-2-ylsulfanyl]-acetic acid ethyl ester, tetrabutyl ammonium fluoride solution 1M and 1,2-diaminoethane in THF. The resultant crude product was purified by flash chromatography on SiO₂ eluting first with 50% EtOAc/Hexane and then 50% EtOAc/DCM to afford the title compound as a pale yellow solid, 30mg, 31%.
LC/MS: RT=2.12 Min (270nm); *m*/*z* = 552, 554 [M+H]⁺. Total run time 3.75 min (short pos).
¹H NMR (d₄ MeOH): δ 1.15 (t, 3H); 2.15-2.27 (septet, 2H); 2.82-2.91 (m, 4H); 3.04 (t, 2H); 3.88 (s, 3H); 4.01 (s, 2H); 4.08-4.16 (m, 4H); 7.09 (s, 1H); 7.11 (s, 1H); 8.07 (s, 1H) NH not seen.
This compound had activity 'A' in the fluorescence polarization assay described below.

### Example 50

### (4-{2-Chloro-5-[2-(3,3-difluoro-pyrrolidin-1-yl)-ethoxy]-4-methoxy-phenyl}-5-cyano-7H-pyrrolo[2,3-d]pyrimidin-2-ylsulfanyl)-acetic acid.

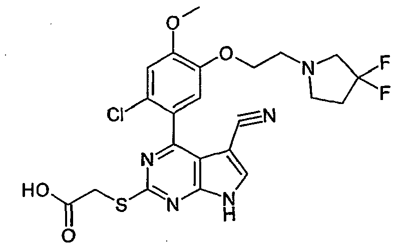

To a solution of (4-{2-Chloro-5-[2-(3,3-difluoro-pyrrolidin-1-yl)-ethoxy]-4-methoxyphenyl}-5-cyano-7H-pyrrolo[2,3-d]pyrimidin-2-ylsulfanyl)-acetic acid ethyl ester, (20mg, 0.0362mmol) in MeOH (1 mL) was added KOH (8mg, 0.145mmol) in water (1mL) and the reaction was refluxed for 1.5hr. The reaction was cooled to RT and concentrated *in vacuo.* The residue was dissolved in the minimum amount of water and carefully acidified to pH4 using 2M HCl. The resulting aqueous solution was extracted with EtOAc (3x10mL) and the combined extracts were washed with saturated brine solution, dried (Na₂SO₄) and concentrated *in vacuo* to afford the title compound as a white solid, 15.5mg, 82%.
LC/MS: RT=1.77 Min (270nm); *m*/*z* = 524, 526 [M+H]⁺. Total run time 3.75 min (short pos).
¹H NMR (d₄ MeOH): δ 2.29-2.41 (septet, 2H); 3.01-3.09 (m, 4H); 3.23 (t, 2H); 3.98 (s, 3H); 4.09 (s, 2H); 4.25 (t, 2H); 7.19 (s, 1H); 7.21 (s, 1H); 8.15 (s, 1H); NH and OH not seen.
This compound had activity 'A' in the fluorescence polarization assay described below.

Further compounds of the invention are listed in table 1. These compounds are made by way of the routes outlined in schemes 1-5 and utilizing methods outlined in examples 1-50.

**Table 1**

| **EXAMPLE** | **STRUCTURE** | **RETENTION TIME (Mins)** | **[M+H]⁺** | **ACTIVITY*** |
|---|---|---|---|---|
| **51** | 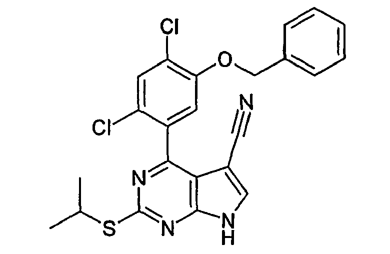 | 2.85 | 469, 471 | B |
| **52** | 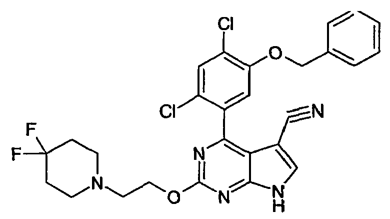 | 2.18 | 558 | B |
| **53** | 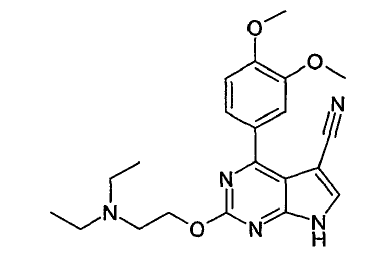 | 1.537 | 396 | B |
| **54** | 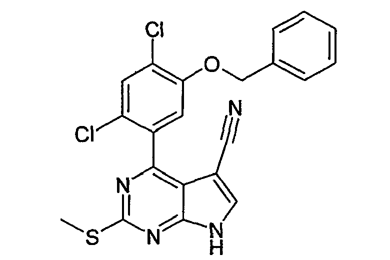 | 2.72 | 441, 443 | B |
| **55** | 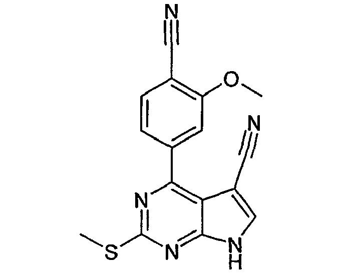 | 2.28 | 322 | B |
| **56** | 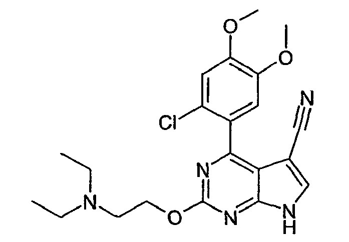 | 1.59 | 430 | A |
| **57** | 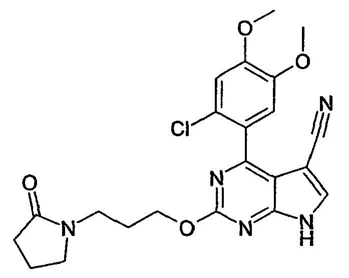 | 1.95 | 456 | A |
| **58** | 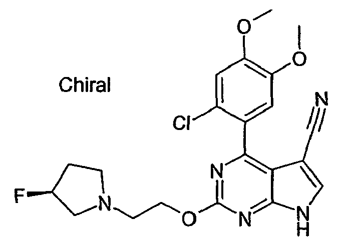 | 1.65 | 446 | A |
| **59** | 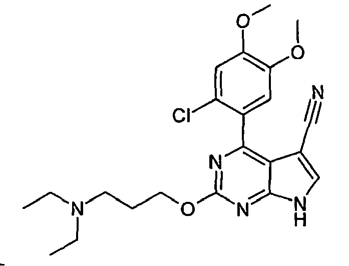 | 1.62 | 444, 446 | A |
| **60** | 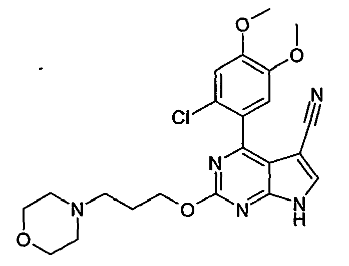 | 1.57 | 458, 460 | A |
| **61** | 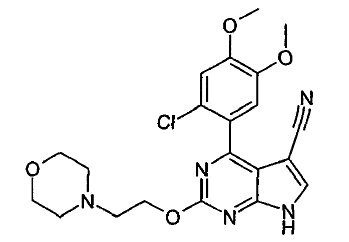 | 1.54 | 444, 446 | A |
| **62** | 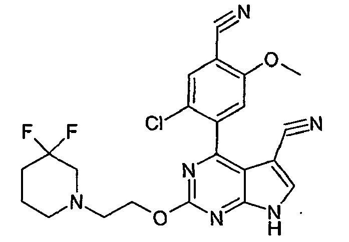 | 2.05 | 473 | A |
| **63** | 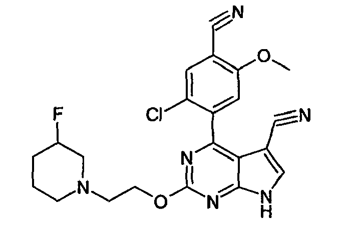 | 1.66 | 455 | A |
| **64** | 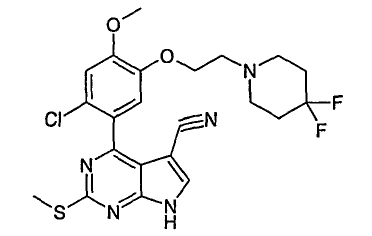 | 1.84 | 494 | A |
| **65** | 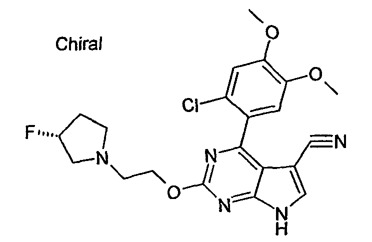 | 1.53 | 446 | A |
| **66** | 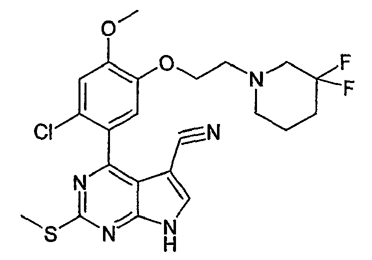 | 2.039 | 494 | A |
| **67** | 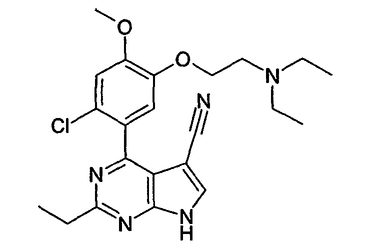 | 1.64 | 428, 430 | A |
| **68** | 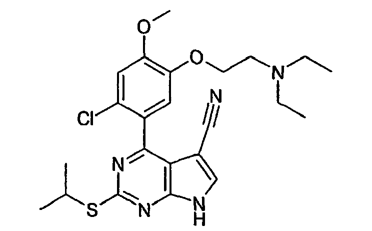 | 1.90 | 474, 476 | A |
| **69** | 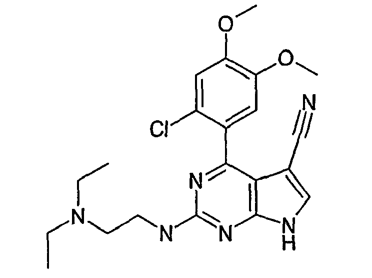 | 1.60 | 429, 431 | B |
| **70** | 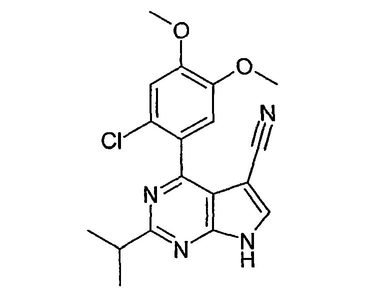 | 2.22 | 357, 359 | B |
| **71** | 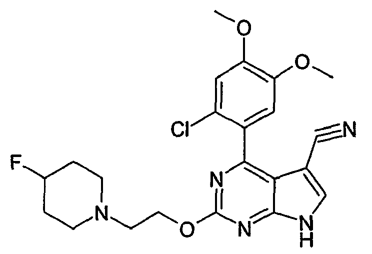 | 1.57 | 460 | A |
| **72** | 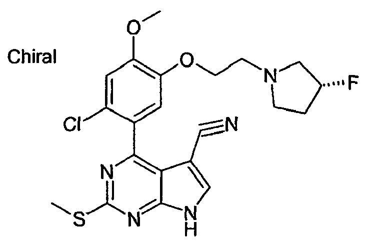 | 1.72 | 462 | A |
| **73** | 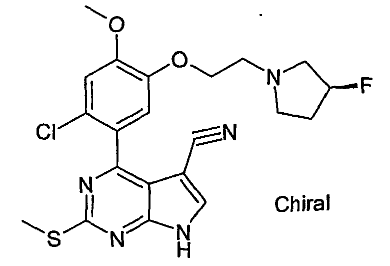 | 1.72 | 462 | A |
| **74** | 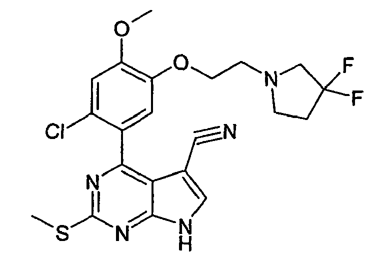 | 2.07 | 480 | A |
| **75** | 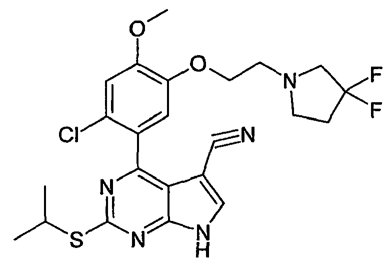 | 2.32 | 508 | A |
| **76** | 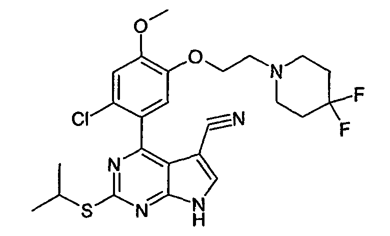 | 2.02 | 522 | A |
| **77** | 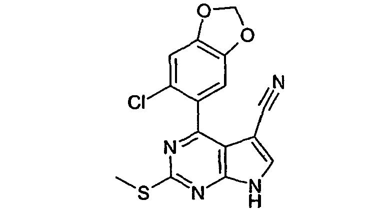 | 2.30 | 345, 347 | A |
| **78** | 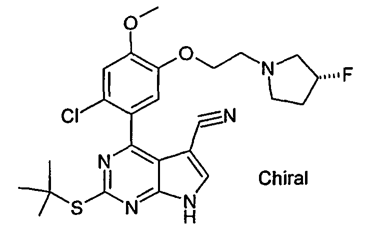 | 1.94 | 504 | A |
| **79** | 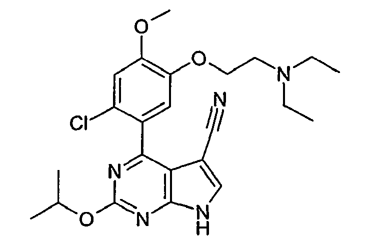 | 1.78 | 458, 460 | A |
| **80** | 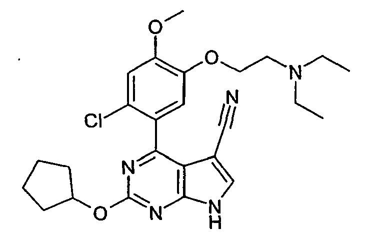 | 1.88 | 484, 486 | A |
| **81** | 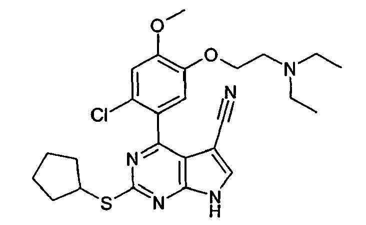 | 1.99 | 500, 502 | A |
| **82** | 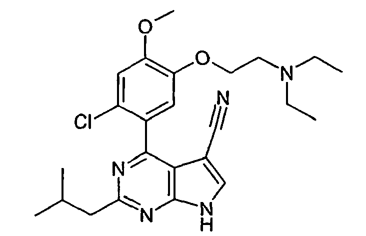 | 1.79 | 456, 458 | B |
| **83** | 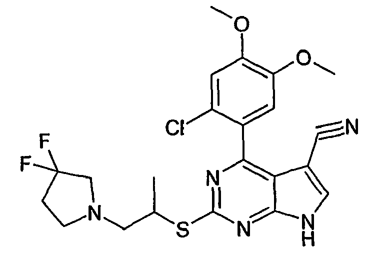 | 2.34 | 494 | A |
| **84** | 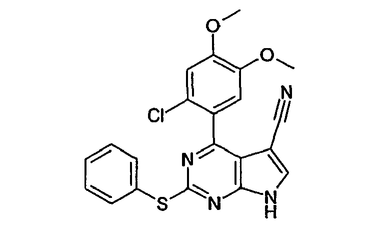 | 2.48 | 423, 425 | A |
| **85** | 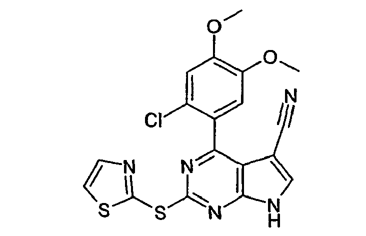 | 2.26 | 430, 432 | A |
| **86** | 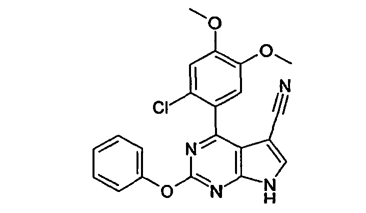 | 2.34 | 407, 409 | A |
| **87** | 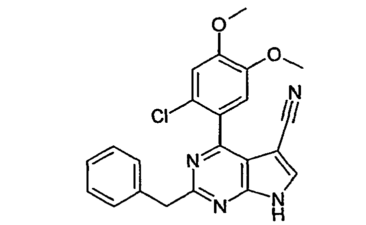 | 2.33 | 405, 407 | A |
| **88** | 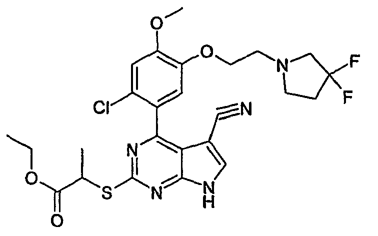 | 3.50 | 566 | A |
| **89** | 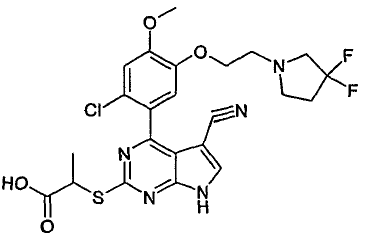 | 1.87 | 538 | A |
| **90** | 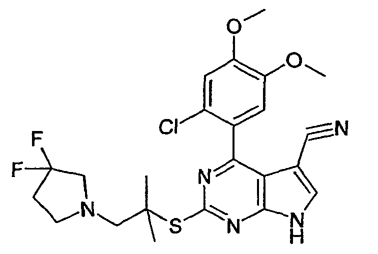 | 2.48 | 508 | A |
| **91** | 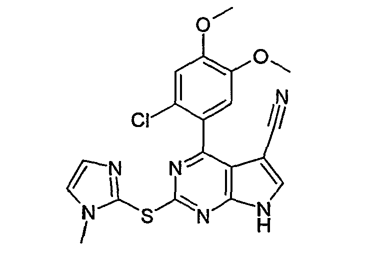 | 1.96 | 427, 429 | B |
| **92** | 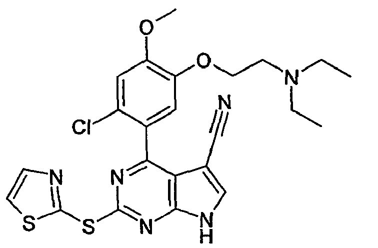 | 1.77 | 515, 517 | A |

| | | | | |
|---|---|---|---|---|
| *Activity in the FP binding assay described below | | | | |

### Example 93

### 4-(1,3-Dihydro-isoindole-2-carbonyl)-2-methylsulfanyl-7H-pyrrolo[2,3-d]pyrimidine-5-carbonitrile

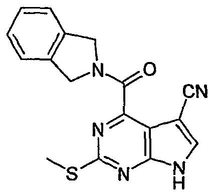

### Step 1

### 5-Cyano-2-methylsulfanyl-7-(2-trimethylsilanyl-ethoxymethyl)-7H-pyrrolo[2,3-d]pyrimidine-4-carboxylic acid

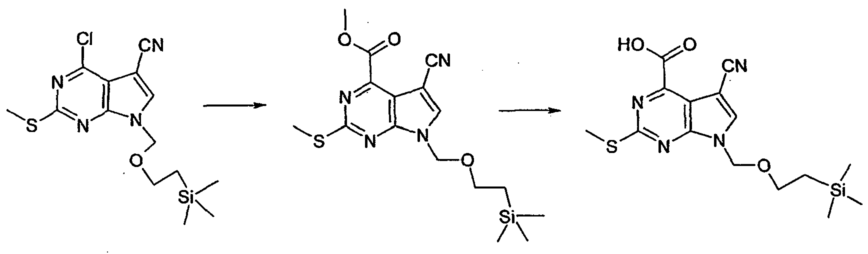

4-Chloro-2-methylsulfanyl-7-(2-trimethylsilanyl-ethoxymethyl)-7H-pyrrolo[2,3-d]pyrimidine-5-carbonitrile (100 mg, 0.282 mmol), triethylamine (0.083 ml, 0.592 mmol), MeOH (0.16 ml, 3.93 mmol), 1,3-bis(diphenylphosphino)propane (96 mg, 0.23 mmol), Pd(OAc)₂ (48 mg, 0.214 mmol) and anhydrous DMF (2 ml) were combined. CO was then bubbled through the mixture for 2 min. The reaction was then heated at 70°C under a CO atmosphere for 4h. The reaction mixture was partitioned between EtOAc (2 x 15 ml) and sat. NH₄Cl sol. (20 ml). The combined organic phases were dried (Na₂SO₄) and evaporated *in vacuo* to give a crude oil. This was purified by flash chromatography on silica gel (20g) eluting with Hexane-30% EtOAc/Hexane (gradient) to afford the impure methyl ester as a yellow oil. This was dissolved in DMA (1 ml) and 2N NaOH (0.1 ml, 0.212 mmol) added. After stirring for 2h at RT water (15 ml) was added and the solution acidified to pH 4-5 by cautious addition of 1N HCl (aq) solution. This was extracted with EtOAc (3 x 20 ml), the organics dried (Na₂SO₄) and evaporated *in vacuo* to give the title compound as a yellow oil, 50mg. LC/MS: RT=2.50 Min; *m*/*z* = 365 [M+H]⁺. Total run time 3.75 mins.

### Step 2

### 4-(1,3-Dihydro-isoindole-2-carbonyl)-2-methylsulfanyl-7-(2-trimethylsilanyl-ethoxymethyl)-7H-pyrrolo[2,3-d]pyrimidine-5-carbonitrile

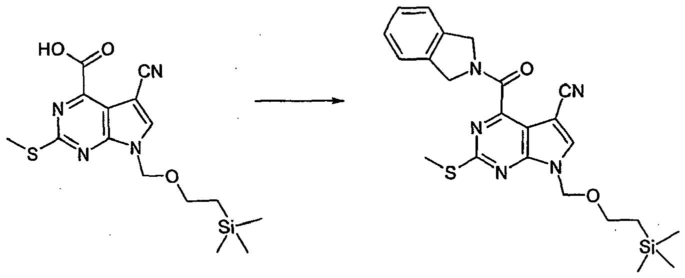

5-Cyano-2-methylsulfanyl-7-(2-trimethylsilanyl-ethoxymethyl)-7H-pyrrolo[2,3-d]pyrimidine-4-carboxylic acid (25 mg, ~0.05 mmol), isoindoline (0.106 mmol), HBTU (40 mg, 0.106 mmol), DIPEA (0.018 ml, 0.106 mmol) and DMA (1 mL) were combined under N₂ and stirred at RT for 1 h. The reaction was then partitioned between EtOAc (2 x 15 ml) and sat. NH₄Cl sol. (20 ml). The combined organic phases were dried (Na₂SO₄) and evaporated *in vacuo.* The resultant crude product was purified by flash chromatography on SiO₂ eluting with hexane - ethyl acetate mixtures (gradient) to afford the title compound as a yellow oil.
LC/MS: RT=2.84 Min; *m*/*z* = 466 [M+H]⁺. Total run time 3.75 mins.

### Step 3

### 4-(1,3-Dihydro-isoindole-2-carbonyl)-2-methylsulfanyl-7H-pyrrolo[2,3-d]pyrimidine-5-carbonitrile

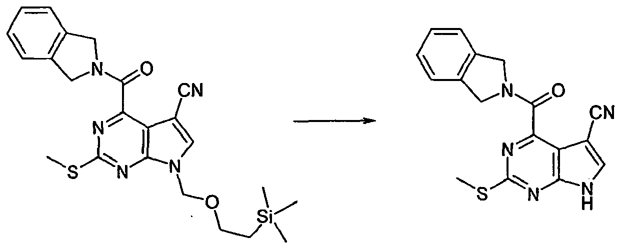

The title compound was prepared by the way of the methods of example 1 step 5, using 4-(1,3-dihydro-isoindole-2-carbonyl)-2-methylsulfanyl-7-(2-trimethylsilanyl-ethoxymethyl)-7H-pyrrolo[2,3-d]pyrimidine-5-carbonitrile tetrabutyl ammonium fluoride solution 1M and 1,2-diaminoethane in THF.
LC/MS: RT=2.19 Min; *m*/*z* = 336 [M+H]⁺. Total run time 3.75 mins.
This compound had activity 'B' in the fluorescence polarization assay described below.

### Fluorescence Polarization Assay

Fluorescence polarization {also known as fluorescence anisotropy} measures the rotation of a fluorescing species in solution, where the larger molecule the more polarized the fluorescence emission. When the fluorophore is excited with polarized light, the emitted light is also polarized. The molecular size is proportional to the polarization of the fluorescence emission.

The fluoroscein-labelled probe - 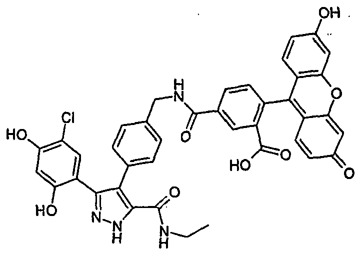 binds to HSP90 {full-length human, full-length yeast or N-terminal domain HSP90} and the anisotropy {rotation of the probe:protein complex} is measured.

Test compound is added to the assay plate, left to equilibrate and the anisotropy measured again. Any change in anisotropy is due to competitive binding of compound to HSP90, thereby releasing probe.

### Materials

Chemicals are of the highest purity commercially available and all aqueous solutions are made up in AR water.
1) Costar 96-well black assay plate #3915
2) Assay buffer of (a)100mM Tris pH7.4; (b) 20mM KCI; (c) 6mM MgCl₂. Stored at room temperature.
3) BSA (bovine serum albumen) 10 mg/ml (New England Biolabs # B9001S)
4) 20 mM probe in 100 % DMSO stock concentration. Stored in the dark at RT. Working concentration is 200 nM diluted in AR water and stored at 4 °C. Final concentration in assay 80 nM.
5) E. coli expressed human full-length HSP90 protein, purified >95% (see, e.g., Panaretou et al., 1998) and stored in 50µL aliquots at -80°C .

### Protocol

1) Add 100µl 1x buffer to wells 11A and 12A (=FP BLNK)
2) Prepare assay mix - all reagents are kept on ice with a lid on the bucket as the probe is light-sensitive.

| | i. Final Concⁿ | |
|---|---|---|
| • 1x Hsp90 FP Buffer | 10 ml | 1x |
| • BSA 10mg/ml (NEB) | 5.0 µl | 5 µg/ml |
| • Probe 200µM | 4.0 µl | 80 nM |
| • Human full-length Hsp90 | 6.25 µl | 200 nM |

3) Aliquot 100µl assay mix to all other wells
4) Seal plate and leave in dark at room temp for 20 minutes to equilibrate

### Compound Dilution Plate - 1 x 3 dilution series

1) In a clear 96-well v-bottom plate - {# VWR 007/008/257} add 10 µl 100% DMSO to wells B1 to H11
2) To wells A1 to A11 add 17.5µl 100% DMSO
3) Add 2.5 µl cpd to A1. This gives 2.5 mM {50x} stock cpd - assuming cpds 20 mM.
4) Repeat for wells A2 to A10. Control in columns 11 and 12.
5) Transfer 5 µl from row A to row B- not column 12. Mix well.
6) Transfer 5 µl from row B to row C. Mix well.
7) Repeat to row G.
8) Do not add any compound to row H - this is the 0 row.
9) This produces a 1x3 dilution series from 50 µM to 0.07 µM.
10) In well B12 prepare 20 µl of 100 µM standard compound.
11) After first incubation the assay plate is read on a Fusion™ α-FP plate reader (Packard BioScience, Pangbourne, Berkshire,UK).
12) After the first read, 2 µl of diluted compound is added to each well for columns 1 to 10. In column 11 {provides standard curve} only add compound B11 - H11. Add 2 µl of 100mM standard cpd to wells B12 - H12 {is positive control}
13) The Z' factor is calculated from zero controls and positive wells. It typically gives a value of 0.7 - 0.9.

The compounds tested in the above assay were assigned to one of three activity ranges, namely A = <0.5µM; B = 0.5µM to 10µM; C = >10µM, and those assignments are reported above.

A growth inhibition assay was also employed for the evaluation of test compounds:
Assessment of cytotoxicity by Sulforhodamine B (SRB) assay: calculation of 50% inhibitory concentration (IC₅₀).

### Day 1

1) Determine cell number by haemocytometer.
2) Using an 8 channel multipipettor, add 160µl of the cell suspension (3600 cells/well or 2 x 10⁴ cells/ml) to each well of a 96-well microtitre plate.
3) Incubate overnight at 37°C in a CO₂ incubator.

### Day 2

4) Stock solutions of drugs are prepared, and serial dilutions of each drug are performed in medium to give final concentrations in wells.
5) Using a multipipettor, 40µl of drug (at 5x final concentration) is added to quadruplicate wells.
6) Control wells are at either side of the 96 well plates, where 40µl of medium is added.
7) Incubate plates in CO₂ incubator for 4 days (48 hours).

### Day 6

8) Tip off medium into sink and immerse plate slowly into 10% ice cold trichloroacetic acid (TCA). Leave for about 30mins on ice.
9) Wash plates three times in tap water by immersing the plates into baths of tap water and tipping it off.
10) Dry in incubator.
11) Add 100µl of 0.4% SRB in 1% acetic acid to each well (except the last row (right hand)of the 96 well plate, this is the 0% control, ie no drug, no stain. The first row will be the 100% control with no drug, but with stain). Leave for 15 mins.
12) Wash off unbound SRB stain with four washes of 1% acetic acid.
13) Dry plates in incubator.
14) Solubilise SRB using 100µl of 10mM Tris base and put plates on plate shaker for 5 mins.
15) Determine absorbance at 540nm using a plate reader. Calculate mean absorbance for quadruplicate wells and express as a percentage of value for control, untreated wells.
16) Plot % absorbance values versus log drug concentration and determine the GI₅₀, ie the concentration of test compound required to inhibit growth of the cells by 50%.

The compounds tested in the above assay were assigned to one of three activity ranges, namely A = <0.5µM; B = 0.5µM to 10µM; C = >10µM, and the results for six of the compounds of the invention are reported in Table 2.

**Table 2**

| **EXAMPLE** | **STRUCTURE** | **GI₅₀*** |
|---|---|---|
| **51** | 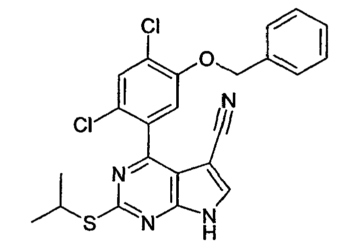 | B |
| **28** | 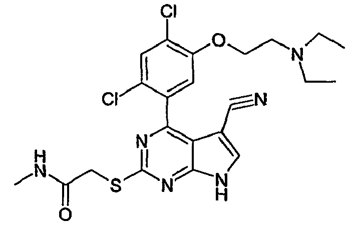 | A |
| **24** | 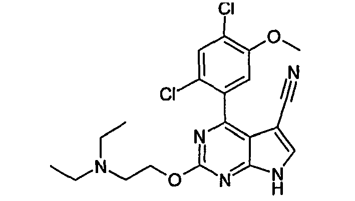 | B |
| **31** | 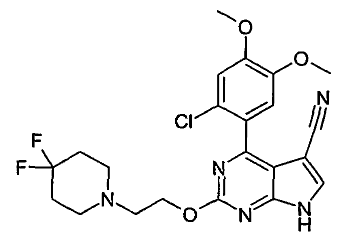 | A |
| **72** | 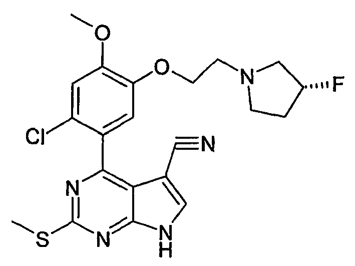 | A |
| **19** | 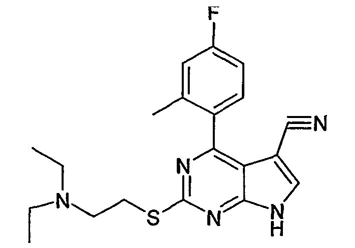 | B |

| | | |
|---|---|---|
| GI₅₀: Growth inhibition in BT474 cells as described. | | |

### REFERENCES

A number of publications are cited above in order to more fully describe and disclose the invention and the state of the art to which the invention pertains. Full citations for these references are provided below.

Reviews for detailed background and drug discovery:
Isaacs JS. 2005 Heat-shock protein 90 inhibitors in antineoplastic therapy: is it all wrapped up?, Expert Opin. Investig. Drugs 14, 569-589.
Maloney A and Workman P. 2002 Hsp90 as a new therapeutic target for cancer therapy: the story unfolds, Expert Opin. Biol. Ther. 2, 3-24.
Whitesell L and Lindquist SL. 2005 Hsp90 and the chaperoning of cancer, Nature Rev. Cancer 5, 761-772.
Janin Y, Heat Shock Protein 90 Inhibitors. A Text Book Example of Medicinal Chemistry? J. Med. Chem. 2005, 48, 7503.
Aoyagi S and Archer TK. 2005 Modulating molecular chaperone Hsp90 functions through reversible acetylation, Trends Cell Biol. 15, 565-7.
Argon Y and Simen BB. 1999 Grp94, an ER chaperone with protein and peptide binding properties, Semin. Cell Dev. Biol. 10, 495-505.
Bijlmakers M-JJE, Marsh M. 2000 Hsp90 is essential for the synthesis and subsequent membrane association, but not the maintenance, of the Src-kinase p56lck, Molecular Biology of the Cell 11, 1585-1595.
Bucci M; Roviezzo F; Cicala C; Sessa WC, Cirino G. 2000 Geldanamycin, an inhibitor of heat shock protein 90 (Hsp90) mediated signal transduction has anti-inflammatory effects and interacts with glucocorticoid receptor in vivo, Brit. J. Pharmacol. 131, 13-16.
Chen C-F, Chen Y, Dai KD, Chen P-L, Riley DJ and Lee W-H. 1996 A new member of the hsp90 family of molecular chaperones interacts with the retinoblastoma protein during mitosis and after heat shock, Mol. Cell. Biol. 16, 4691-4699.
Chiosis G, Timaul MN, Lucas B, Munster PN, Zheng FF, Sepp-Lozenzino L and Rosen N. 2001 A small molecule designed to bind to the adenine nucleotide pocket of Hsp90 causes Her2 degradation and the growth arrest and differentiation of breast cancer cells, Chem. Biol. 8, 289-299.
Conroy SE and Latchman DS. 1996 Do heat shock proteins have a role in breast cancer?, Brit. J. Cancer 74, 717-721.
Dymock B, Barril X, Beswick M, Collier A, Davies N, Drysdale M, Fink A, Fromont C, Hubbard RE, Massey A, Surgenor A, Wright L. Adenine Derived inhibitors of the molecular chaperone HSP90-SAR explained through multiple X-ray structures. Bioorg. Med. Chem. Lett., 2004, 14, 325.
da Rocha Dias S, Friedlos F, Light Y, Springer C, Workman P and Marais R. 2005 Activated B-Raf is an Hsp90 client protein that is targeted by the anticancer drug 17-Allylamino-17-demethoxygeldanamycin, Cancer Res. 65, 10686-10691.
Felts SJ, Owen BAL, Nguyen P, Trepel J, Donner DB and Toft DO. 2000 The Hsp90-related protein TRAP1 is a mitochondrial protein with distinct functional properties, J. Biol. Chem. 5, 3305-3312.
Fuller W, Cuthbert AW. 2000 Post-translational disruption of the delta F508 cystic fibrosis transmembrane conductance regulator (CFTR)-molecular Chaperone complex with geldanamycin stabilizes delta F508 CFTR in the rabbit reticulocyte lysate, J. Biol. Chem. 275(48), 37462-37468.
Hickey E, Brandon SE, Smale G, Lloyd D and Weber LA. 1999 Sequence and regulation of a gene encoding a human 89-kilodalton heat shock protein, Mol. Cell. Biol. 9, 2615-2626.
Hoang AT, Huang J, Rudra-Gonguly N, Zheng J, Powell WC, Rabindron SK, Wu C and Roy-Burman P. 2000 A novel association between the human heat shock transcription factor I (HSF1) and prostate adenocarcinoma, Am. J. Pathol. 156, 857-864.
Hostein I, Robertson D, Di Stefano F, Workman P and Clarke PA. 2001 Inhibition of signal transduction by the Hsp90 inhibitor 17-allylamino-17-demethoxygeldanamycin results in cytostasis and apoptosis, Cancer Res. 61, 4003-4009.
Hur E, Kim H-H, Choi SM, Kim JH, Yim S, Kwon HJ, Choi Y, Kim DK, Lee M-O, Park H. 2002 Reduction of hypoxia-induced transcription through the repression of hypoxia-inducible factor-1□/aryl hydrocarbon receptor nuclear translocator DNA binding by the 90-kDa heat-shock protein inhibitor radicicol, Mol. Pharmacol. 62(5), 975-982.
Hutter et al. 1996 Circulation 94, 1408.
Jameel A, Skilton RA, Campbell TA, Chander SK, Coombes RC and Luqmani YA. 1992 Clinical and biological significance of Hsp89a in human breast cancer, Int. J. Cancer 50, 409-415.
Jolly C and Morimoto RI. 2000 Role of the heat shock response and molecular chaperones in oncogenesis and cell death, J. Natl. Cancer Inst. 92, 1564-1572.
Kamal A, Thao L, Sensintaffar J, Zhang L, Boehm MF, Fritz LC and Burrows FJ. 2003 A high-affinity conformation of Hsp90 confers tumour selectivity on Hsp90 inhibitors, Nature 425, 407-410.
Kasibhatla SR, Hong K, Zhang L, Biamonte MA, Boehm MF, Shi J, Fan J. PCT Int Appl. WO 2003037860.
Kawanishi K, Shiozaki H, Doki Y, Sakita I, Inoue M, Yano M, Tsujinata T, Shamma A and Monden M. 1999 Prognostic significance of heat shock proteins 27 and 70 in patients with squamous cell carcinoma of the esophagus, Cancer 85, 1649-1657.
Kelland LR, Abel G, McKeage MJ, Jones M, Goddard PM, Valenti M, Murrer BA and Harrap KR. 1993 Preclinical antitumour evaluation of bis-acetaloamino-dichloro-cyclohexylamine platinum (IV): an orally active platinum drug, Cancer Research 53, 2581-2586.
Kelland LR, Sharp SY, Rogers PM, Myers TG and Workman P. 1999 DT-diaphorase expression and tumor cell sensitivity to 17-allylamino, 17-demethoxygeldanamycin, an inhibitor of heat shock protein 90, J. Natl. Cancer Inst. 91, 1940-1949.
Kovacs JJ, Murphy PJM, Gaillard S, Zhao X, Wu J-T, Nicchitta CV, Yoshida M, Toft DO, Pratt WB and Yao T-P. 2005 HDAC6 regulates Hsp90 acetylation and chaperone-dependent activation of the glucocorticoid receptor, Mol. Cell 18, 601-607.
Kurebayashi J, Otsuki T, Kurosumi M, Soga S, Akinaga S, Sonoo, H. 2001 A radicicol derivative, KF58333, inhibits expression of hypoxia-inducible factor-1□ and vascular endothelial growth factor, angiogenesis and growth of human breast cancer xenografts, Jap. J. Cancer Res. 92(12), 1342-1351.
Kwon HJ, Yoshida M, Abe K, Horinouchi S and Bepple T. 1992 Radicicol, an agent inducing the reversal of transformed phentoype of src-transformed fibroblasts, Biosci., Biotechnol., Biochem., 56, 538-539.
Lebeau J, Le Cholony C, Prosperi MT and Goubin G. 1991 Constitutive overexpression of 89 kDa heat shock protein gene in the HBL100 mammary cell line converted to a tumorigenic phenotype by the EJ/T24 Harvey-ras oncogene, Oncogene 6, 1125-1132.
Llauger L, He, H, Kim J, Aguirre J, Rosen N, Peters U,; Davies P, Chiosis G, J. Med. Chem. 2005, 48, 2892
Marcu MG, Chadli A, Bouhouche I, Catelli M and Neckers L. 2000a The heat shock protein 90 antagonist novobiocin interacts with a previously unrecognized ATP-binding domain in the carboxyl terminus of the chaperone, J. Biol. Chem. 275, 37181-37186.
Marcu MG, Schulte TW and Neckers L. 2000b Novobiocin and related coumarins and depletion of heat shock protein 90-dependent signaling proteins, J. Natl. Cancer Inst. 92, 242-248.
Martin KJ, Kritzman BM, Price LM, Koh B, Kwan CP, Zhang X, MacKay A, O'Hare MJ, Kaelin CM, Mutter GL, Pardee AB and Sager R. 2000 Linking gene expression patterns to therapeutic groups in breast cancer, Cancer Res. 60, 2232-2238.
Neckers L, Schulte TW and Momnaaugh E. 1999 Geldanamycin as a potential anticancer agent: its molecular target and biochemical activity, Invest. New Drugs 17, 361-373.
Page J, Heath J, Fulton R, Yalkowsky E, Tabibi E, Tomaszewski J, Smith A and Rodman L. 1997 Comparison of geldanamycin (NSC-122750) and 17-allylaminogeldanamycin (NSC-330507D) toxicity in rats, Proc. Am. Assoc. Cancer Res. 38, 308.
Panaretou B, Prodromou C, Roe SM, O'Brien R, Ladbury JE, Piper PW and Pearl LH. 1998 ATP binding and hydrolysis are essential to the function of the Hsp90 molecular chaperone in vivo, EMBO J. 17, 4829-4836.
Plumier et al. 1997 Cell. Stress Chap., 2, 162
Pratt WB. 1997 The role of the Hsp90-based chaperone system in signal transduction by nuclear receptors and receptors signalling via MAP kinase, Annu. Rev. Pharmacol. Toxicol. 37, 297-326.
Prodromou C and Pearl LH. 2000a Structure and in vivo function of Hsp90, Curr. Opin. Struct. Biol. 10, 46-51.
Prodromou C, Roe SM, O'Brien R, Ladbury JE, Piper PW and Pearl LH. 1997 Identification and structural characterization of the ATP/ADP-binding site in the Hsp90 molecular chaperone, Cell 90, 65-75.
Prodromou C, Panaretou B, Chohan S, Siligardi G, O'Brien R, Ladbury JE, Roe SM, Piper PW and Pearl LH. 2000b The ATPase cycle of Hsp90 drives a molecular 'clamp' via transient dimerization of the N-terminal domains, EMBO J. 19, 4383-4392.
Rajder et al. 2000 Ann. Neurol. 47, 782.
Roe SM, Prodromou C, O'Brien R, Ladbury JE, Piper PW and Pearl LH. 1999 Structural basis for inhibition of the Hsp90 molecular chaperone by the antitumour antibiotics radicicol and geldanamycin, J. Med. Chem. 42, 260-266.
Rutherford SL and Lindquist S. 1998 Hsp90 as a capacitor for morphological evolution. Nature 396, 336-342.
Schulte TW, Akinaga S, Murakata T, Agatsuma T, Sugimoto S, Nakano H, Lee YS, Simen BB, Argon Y, Felts S, Toft DO, Neckers LM and Sharma SV. 1999 Interaction of radicicol with members of the heat shock protein 90 family of molecular chaperones, Mol. Endocrinology 13, 1435-1448.
Schulte TW, Akinaga S, Soga S, Sullivan W, Sensgard B, Toft D and Neckers LM. 1998 Antibiotic radicicol binds to the N-terminal domain of Hsp90 and shares important biologic activities with geldanamcyin, Cell Stress and Chaperones 3, 100-108.
Schulte TW and Neckers LM. 1998 The benzoquinone ansamycin 17-allylamino-17-deemthoxygeldanamcyin binds to Hsp90 and shares important biologic activities with geldanamycin, Cancer Chemother. Pharmacol. 42, 273-279.
Siligardi G, Hu B, Panaretou B, Piper PW, Pearl LH and Prodromou C. 2004 Co-chaperone regulation of conformational switching in the Hsp90 ATPase cycle, J. Biol. Chem. 279, 51989-51998.
Sittler et al. 2001 Hum. Mol. Genet. 10, 1307.
Smith DF. 2001 Chaperones in signal transduction, in: Molecular chaperones in the cell (P Lund, ed.; Oxford University Press, Oxford and NY), 165-178.
Smith DF, Whitesell L and Katsanis E. 1998 Molecular chaperones: Biology and prospects for pharmacological intervention, Pharmacological Reviews 50, 493-513.
Song HY, Dunbar JD, Zhang YX, Guo D and Donner DB. 1995 Identification of a protein with homology to hsp90 that binds the type 1 tumour necrosis factor receptor, J. Biol. Chem. 270, 3574-3581.
Stebbins CE, Russo A, Schneider C, Rosen N, Hartl FU and Pavletich NP. 1997 Crystal structure of an Hsp90-geldanamcyin complex: targeting of a protein chaperone by an antitumor agent, Cell 89, 239-250.
Supko JG, Hickman RL, Grever MR and Malspeis L. 1995 Preclinical pharmacologic evaluation of geldanamycin as an antitumour agent, Cancer Chemother. Pharmacol. 36, 305-315.
Tratzelt et al. 1995 Proc. Nat. Acad. Sci. 92, 2944.
Trost et al. 1998 J. Clin. Invest. 101, 855.
Tytell M and Hooper PL. 2001 Heat shock proteins: new keys to the development of cytoprotective therapies, Emerging Therapeutic Targets 5, 267-287.
Uehara U, Hori M, Takeuchi T and Umezawa H. 1986 Phenotypic change from transformed to normal induced by benzoquinoid ansamycins accompanies inactivation of p60src in rat kidney cells infected with Rous sarcoma virus, Mol. Cell. Biol. 6, 2198-2206.
Waxman, Lloyd H. Inhibiting hepatitis C virus processing and replication. (Merck & Co., Inc., USA). PCT Int. Appl. (2002), WO 0207761
Winklhofer et al. 2001 J. Biol. Chem. 276, 45160.
Whitesell L, Mimnaugh EG, De Costa B, Myers CE and Neckers LM. 1994 Inhibition of heat shock protein Hsp90-pp60v-src heteroprotein complex formation by benzoquinone ansamycins: essential role for stress proteins in oncogenic transformation, Proc. Natl. Acad. Sci. USA. 91, 8324-8328.
Yorgin et al. 2000 Effects of geldanamycin, a heat-shock protein 90-binding agent, on T cell function and T cell nonreceptor protein tyrosine kinases, J. Immunol. 164(6), 2915-2923.
Young JC, Moarefi I and Hartl FU. 2001 Hsp90: a specialized but essential protein-folding tool, J. Cell. Biol. 154, 267-273.
Zhao JF, Nakano H and Sharma S. 1995 Suppression of RAS and MOS transformation by radicicol, Oncogene 11, 161-173.

## Claims

1. A compound of formula (I), or a pharmaceutically acceptable salt thereof: 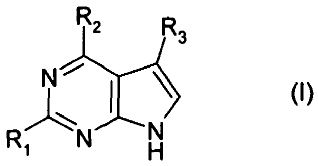 wherein
R₁ is hydrogen, fluoro, chloro, bromo, or a radical of formula ((1A):
-X-Alk¹-(Z)ₘ-(Alk²)ₙ-Q (IA)
wherein
X is -O-, -S- -S(O)-, -SO₂-, or -NH-,
Z is -O-, -S-, -(C=O)-, -(C=S)-, -S(O)-, -SO₂-, -NR^{A}-, or, in either orientation -C(=O)O-, -C(=O)NR^{A}-, -C(=S)NR^{A}-, -SO₂NR^{A}-, -NR^{A}C(=O)-, or -NR^{A}SO₂- wherein R^{A} is hydrogen or C₁-C₆ alkyl Alk¹ and Alk² are optionally substituted divalent C₁-C₃ alkylene or C₂-C₃ alkenylene radicals,
m, n and p are independently 0 or 1, and
Q is hydrogen or an optionally substituted carbocyclic or heterocyclic radical;
R₂ is optionally substituted phenyl;
R₃ is cyano (-CN), fluoro, chloro, bromo, methyl in which in which one or more hydrogen atoms are optionally replaced by fluorine atoms, ethyl in which in which one or more hydrogen atoms are optionally replaced by fluorine atoms, cyclopropyl, -OH, -CH₂OH,-C(=O)NH₂, -C(=O)CH₃, or -NH₂;
and wherein the term "optionally substituted" means unsubstituted or substituted with at least one substituent selected from (C₁-C₆)alkyl, (C₁-C₆)alkoxy, hydroxy, hydroxy(C₁-C₆)alkyl, mercapto, mercapto(C₁-C₆)alkyl, (C₁-C₆)alkylthio, monocyclic carbocyclic of 3-6 ring carbon atoms, monocyclic heterocyclic of 5 or 6 ring atoms, halo, trifluoromethyl, trifluoromethoxy, nitro, nitrile, oxo, -COOH, -COOR^{A}, -COR^{A}, -SO₂R^{A}, -CONH₂, -SO₂NH₂, -CONHR^{A}, -SO₂NHR^{A}, -CONR^{A}R^{B}, -SO₂NR^{A}R^{B}, -NH₂, -NHR^{A}, -NR^{A}R^{B}, -OCONH₂, -OCONHR^{A}, -OCONR^{A}R^{B}, -NHCOR^{A}, -NHCOOR^{A}, -NR^{B}COOR^{A}, -NHSO₂OR^{A}, -NR^{B}SO₂OR^{A}, -NHCONH₂, -NR^{A}CONH₂, -NHCONHR^{B}, -NR^{A}CONHR^{B}, -NHCONR^{A}R^{B}, or -NR^{A}CONR^{A}R^{B} wherein R^{A} and R^{B} are independently a (C₁-C₆)alkyl group; and in the case where the said substituent contains an alkyl radical, that alkyl radical may be substituted by a monocyclic carbocyclic group of 3-6 ring carbon atoms, or a monocyclic heterocyclic group of 5 or 6 ring atoms; and in the case where the said substituent is or comprises a monocyclic carbocyclic group of 3-6 ring carbon atoms, or a monocyclic heterocyclic group of 5 or 6 ring atoms, that ring may itself be substituted by any of the foregoing non-cyclic optional substituents.

2. A compound as claimed in claim 1 wherein R₃ is cyano (-CN)

3. A compound as claimed in claim 1 or claim 2 wherein R₁ is hydrogen, methoxy, ethoxy, methylthio, ethylthio, hydroxyeththylthio, methylamino, diethylaminomethylthio, methylaminocarbonylmethylthio, or a group of formula (A) -(H): 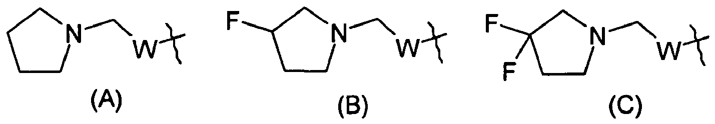 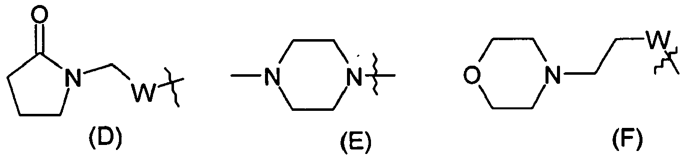 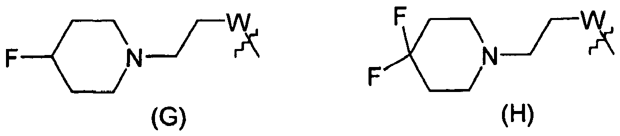 wherein W is -O- or -S-.

4. A compound as claimed in any of the preceding claims wherein R₂ is phenyl, optionally substituted by a one or more substituents selected from methyl, trifuoromethyl, ethyl, n- or isopropyl, vinyl, allyl, methoxy, trifuoromethoxy, ethoxy, methylenedioxy, ethylenedioxy, n-propyloxy, benzyloxy, allyloxy, cyanomethoxy, fluoro, chloro, bromo, cyano, formyl, methyl-, ethyl-, or n-propyl-carbonyloxy, methyl- or ethylaminocarbonyl, and substituents of formula -O(CH₂)ₙZ¹ or -S(CH₂)ₙZ¹ wherein n is 1, 2 or 3 and Z¹ is a primary, secondary, tertiary or cyclic amino group the latter being optionally substituted, or a C₁-C₆alkoxy group; or of formula -(Alk³)ₘZ¹ wherein Alk³ is a divalent straight or branched chain (C₁-C₃) alkylene, m is 0 or 1, and Z¹ is a primary, secondary, tertiary or cyclic amino group, the latter being optionally substituted, or a C₁-C₆alkoxy group.

5. A compound as claimed in claim 4 wherein optional substituents are in the 2- and/or 4- and/or 5-position of the phenyl ring.

6. A compound as claimed in claim 1, having the formula (II): 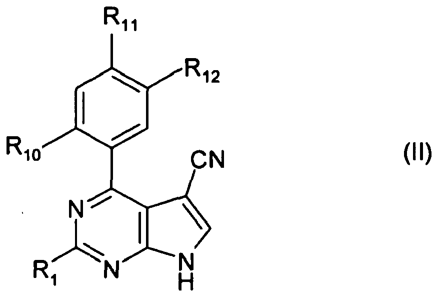 wherein
R₁ is (a) C₁-C₆ alkylthio or C₁-C₆ alkoxy in either of which one or more hydrogen atoms are optionally replaced by fluorine atoms, or (b) a substituent of formula -O(CH₂)ₙZ¹ or -S(CH₂)ₙZ¹ wherein n is 1, 2 or 3 and Z¹ is a primary, secondary, tertiary or cyclic amino group the latter being optionally substituted.
R₁₀ is H, Cl, Br, or -CH₃;
R₁₁ is hydrogen, Cl, Br, CN, methyl, ethyl, n- or iso-propyl, methoxy, ethoxy, vinyl or allyl; and
R₁₂ is (i) a radical of formula -O(CH₂)ₙZ¹ or -S(CH₂)ₙZ¹ wherein n is 1, 2 or 3 and Z¹ is (i) a primary, secondary, tertiary or cyclic amino group, or a C₁-C₆alkoxy group; or (ii) a radical of formula -(Alk³)ₘZ¹ wherein Alk³ is a divalent straight or branched chain (C₁-C₃) alkylene, m is 0 or 1, and Z¹ is a primary, secondary, tertiary or cyclic amino group, or a C₁-C₆alkoxy group.

7. A pharmaceutical or veterinary composition comprising a compound as claimed in any of the preceding claims, together with one or more pharmaceutically or veterinarily acceptable carriers and/or excipients.

8. The use of a compound as claimed in any of claims 1 to 13 in the preparation of a composition for immunosuppression or the treatment of viral disease, drug resistant fungal infection, inflammatory diseases such as rheumatoid arthritis, asthma, multiple sclerosis, Type I diabetes, lupus, psoriasis and inflammatory bowel disease; cystic fibrosis angiogenesis-related disease such as diabetic retinopathy, haemangiomas, and endometriosis; or for protection of normal cells against chemotherapy-induced toxicity; or diseases where failure to undergo apoptosis is an underlying factor; or protection from hypoxia-ischemic injury due to elevation of Hsp70 in the heart and brain; scrapie/CJD, Huntingdon's or Alzheimer's disease.

9. The use as claimed in claim 8 for the treatment of cancer.

## Patentansprüche

1. Verbindung der Formel (I) oder ein pharmazeutisch akzeptables Salz davon: 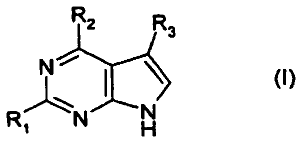 wobei
R₁ Wasserstoff, Fluor, Chlor, Brom oder ein Radikal der Formel (1A) ist:
-X-Alk¹-(Z)ₘ-(Alk²)ₙ-Q (IA)
wobei
X -O-, -S-, -S(O)-, -SO₂- oder -NH ist,
Z -O-, -S-, -(C=O)-, -(C=S)-, -S(O)-, -SO₂-, -NR^{A}- oder, in beiden Orientierungen, -C(=O)O-, -C(=O)NR^{A}-, -C(=S)NR^{A}, -SO₂NR^{A}, NR^{A}C(=O)- oder -NR^{A}SO₂- ist, wobei R^{A} Wasserstoff oder C₁-C₆-Alkyl ist,
Alk¹ und Alk² gegebenenfalls substituierte, divalente C₁-C₃-Alkylen- oder C₂-C₃-Alkenylenradikale sind,
m, n und p unabhängig voneinander 0 oder 1 sind und
Q Wasserstoff oder ein gegebenenfalls substituiertes, carbocyclisches oder heterocyclisches Radikal ist;
R₂ gegebenenfalls substituiertes Phenyl ist;
R₃ Cyano (-CN), Fluor, Chlor, Brom, Methyl, in dem ein oder mehrere Wasserstoffatome gegebenenfalls durch Fluoratome ersetzt sind, Ethyl, in dem ein oder mehrere Wasserstoffatome gegebenenfalls durch Fluoratome ersetzt sind, Cyclopropyl, -OH, -CH₂OH, -C(=O)NH₂, -C(=O)CH₃ oder -NH₂ ist;
und wobei der Begriff "gegebenenfalls substituiert" unsubstituiert oder substituiert mit mindestens einem Substituenten, ausgewählt aus (C₁-C₆)-Alkyl, (C₁-C₆)-Alkoxy, Hydroxy, Hydroxy(C₁-C₆)alkyl, Mercapto, Mercapto(C₁-C₆)alkyl, (C₁-C₆)-Alkylthio, monocyclisch carbocyclisch mit 3 bis 6 Ring-Kohlenstoffatomen, monocyclisch heterocyclisch mit 5 oder 6 Ringatomen, Halo, Trifluormethyl, Trifluormethoxy, Nitro, Nitril, Oxo, -COOH, -COOR^{A}, -COR^{A}, -SO₂R^{A}, -CONH₂, -SO₂NH₂, -CONHR^{A}, SO₂NHR^{A}, -CONR^{A}R^{B}, -SO₂NR^{A}R^{B}, -NH₂, -NHR^{A}, -NR^{A}R^{B}, -OCONH₂, -OCONHR^{A}, -OCONR^{A}R^{B}, -NHCOR^{A}, -NHCOOR^{A}, -NR^{B}COOR^{A}, -NHSO₂OR^{A}, -NR^{B}SO₂OR^{A}, -NHCONH₂, -NR^{A}CONH₂, -NHCONHR^{B}, -NR^{A}CONHR^{B}, -NHCONR^{A}R^{B} oder -NR^{A}CONR^{A}R^{B} bedeutet, wobei R^{A} und R^{B} unabhängig voneinander eine (C₁-C₆)-Alkylgruppe sind; und in dem Fall, in dem der Substituent ein Alkylradikal enthält, kann dieses Alkylradikal durch eine monocyclische, carbocyclische Gruppe mit 3 bis 6 Ring-Kohlenstoffatomen oder eine monocyclische, heterocyclische Gruppe mit 5 oder 6 Ringatomen substituiert sein; und in dem Fall, in dem der Substituent eine monocyclische, carbocyclische Gruppe mit 3 bis 6 Ring-Kohlenstoffatomen oder eine monocyclische, heterocyclische Gruppe mit 5 oder 6 Ringatomen ist oder umfasst, kann dieser Ring selbst durch einen der vorhergehenden, nicht-cyclischen optionalen Substituenten substituiert sein.

2. Verbindung gemäß Anspruch 1, wobei R₃ Cyano (-CN) ist.

3. Verbindung gemäß Anspruch 1 oder Anspruch 2, wobei R₁ Wasserstoff, Methoxy, Ethoxy, Methylthio, Ethylthio, Hydroxyethylthio, Methylamino, Diethylaminomethylthio, Methylaminocarbonylmethylthio oder eine Gruppe der Formel (A) bis (H) ist: 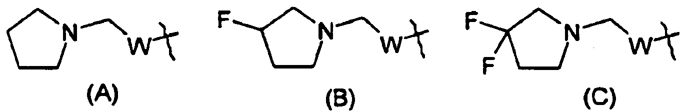 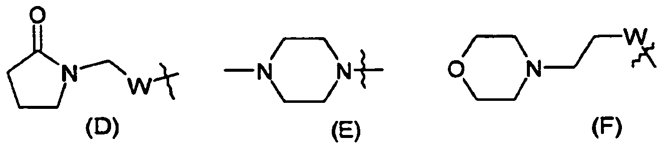 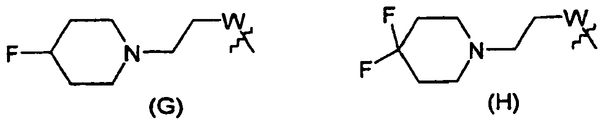 wobei W -O- oder -S- ist.

4. Verbindung gemäß einem der vorhergehenden Ansprüche, wobei R₂ Phenyl ist, gegebenenfalls substituiert durch einen oder mehrere Substituenten, ausgewählt aus Methyl, Trifluormethyl, Ethyl, n- oder Isopropyl, Vinyl, Allyl, Methoxy, Trifluormethoxy, Ethoxy, Methylendioxy, Ethylendioxy, n-Propyloxy, Benzyloxy, Allyloxy, Cyanomethoxy, Fluor, Chlor, Brom, Cyano, Formyl, Methyl-, Ethyl- oder n-Propylcarbonyloxy, Methyl- oder Ethylaminocarbonyl und Substituenten der Formel -O(CH₂)ₙZ¹ oder -S(CH₂)ₙZ¹, wobei n 1, 2 oder 3 ist und Z¹ eine primäre, sekundäre, tertiäre oder cyclische Aminogruppe ist, wobei letztere gegebenenfalls substituiert ist, oder eine C₁-C₆-Alkoxygruppe ist; oder der Formel -(Alk³)ₘZ¹, wobei Alk³ ein divalentes, gerades oder verzweigtes (C₁-C₃)-Alkylen ist, m 0 oder 1 ist und Z¹ eine primäre, sekundäre, tertiäre oder cyclische Aminogruppe, wobei letztere gegebenenfalls substituiert ist, oder eine C₁-C₆-Alkoxygruppe ist.

5. Verbindung gemäß Anspruch 4, wobei optionale Substituenten in der 2- und/oder 4- und/oder 5-Position des Phenylrings vorliegen.

6. Verbindung gemäß Anspruch 1 der Formel (II): 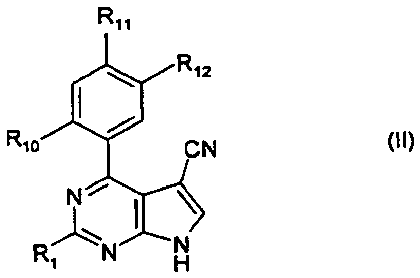 wobei
R₁ (a) C₁-C₆-Alkylthio oder C₁-C₆-Alkoxy, in dem ein oder mehrere Wasserstoffatome gegebenenfalls durch Fluoratome ersetzt sind, oder (b) ein Substituent der Formel -O(CH₂)ₙZ¹ oder -S(CH₂)ₙZ¹ ist, wobei n 1, 2 oder 3 ist und Z¹ eine primäre, sekundäre, tertiäre oder cyclische Aminogruppe ist, wobei letztere gegebenenfalls substituiert ist,
R₁₀ H, Cl, Br oder -CH₃ ist;
R₁₁ Wasserstoff, Cl, Br, CN, Methyl, Ethyl, n- oder Isopropyl, Methoxy, Ethoxy, Vinyl oder Allyl ist; und
R₁₂ (i) ein Radikal der Formel -O(CH₂)ₙZ¹ oder -S(CH₂)ₙZ¹ ist, wobei n 1, 2 oder 3 ist und Z¹ eine primäre, sekundäre, tertiäre oder cyclische Aminogruppe oder eine C₁-C₆-Alkoxygruppe ist; oder (ii) ein Radikal der Formel -(Alk³)ₘZ¹ ist, wobei Alk³ ein divalentes, gerades oder verzweigtes (C₁-C₃)-Alkylen ist, m 0 oder 1 ist und Z¹ eine primäre, sekundäre, tertiäre oder cyclische Aminogruppe oder eine C₁-C₆-Alkoxygruppe ist.

7. Pharmazeutische oder tiermedizinische Zusammensetzung, umfassend eine Verbindung gemäß einem der vorhergehenden Ansprüche, zusammen mit einem oder mehreren pharmazeutisch oder tiermedizinisch akzeptablen Trägerstoffen und/oder Exzipienten.

8. Verwendung einer Verbindung gemäß einem der Ansprüche 1 bis 13 zur Herstellung einer Zusammensetzung zur Immunsuppression oder Behandlung einer Viruserkrankung, Arzneimittel-resistenten Pilzinfektion, Entzündungserkrankung, wie z.B. Gelenkrheumatismus, Asthma, Multiple Sklerose, Typ I-Diabetes, Lupus, Psoriasis sowie entzündlichen Darmerkrankung; Mukoviszidose Angiogenese-verwandten Krankheit, wie z.B. diabetische Retinopathie, Hämangiome sowie Endometriose; oder zum Schutz normaler Zellen gegen Toxizität, die durch Chemotherapie verursacht wird; oder Krankheiten, denen eine Störung der Apoptose zugrunde liegt; oder Schutz vor Hypoxie-ischämischer Schädigung aufgrund einer Erhöhung der Hsp70 im Herzen und Gehirn; Scrapie/Creutzfeld-Jakob-Krankheit, Huntington-Erkrankung oder Alzheimer-Erkrankung.

9. Verwendung gemäß Anspruch 8 zur Krebsbehandlung.

## Revendications

1. Composé de formule (I), ou sel de celui-ci acceptable sur le plan pharmaceutique : 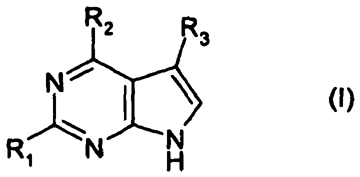 dans lequel
R₁ est l'hydrogène, un groupe fluoro, chloro, bromo, ou un radical de formule (1A) :
-X-Alk¹-(Z)ₘ-(Alk²)ₙ-Q (IA)
où
X est -O-, -S-, -S(O)-, -SO₂- ou -NH-,
Z est -O-, -S-, -(C=O), -(C=S)-, -S(O)-, -SO₂-, -NR^{A}- ou -C(=O)O-selon une orientation quelconque, -C(=O)NR^{A}-, -C(=S)NR^{A}, -SO₂NR^{A}-, -NR^{A}C(=O)- ou -NR^{A}SO₂-, où R^{A} est l'hydrogène ou un groupe alkyle en C₁ à C₆,
Alk¹ et Alk² sont des radicaux alkylène en C₁-C₃ ou alcénylène en C₂-C₃ divalents éventuellement substitués ;
m, n et p valent indépendamment 0 ou 1, et
Q est l'hydrogène ou un radical carbocyclique ou hétérocyclique éventuellement substitué ;
R₂ est un groupe phényle éventuellement substitué ;
R₃ est un groupe cyano (-CN), fluoro, chloro, bromo, méthyle dans lequel un ou plusieurs des atomes d'hydrogène sont éventuellement remplacés par des atomes de fluor, éthyle dans lequel un ou plusieurs atomes d'hydrogène sont éventuellement remplacés par des atomes de fluor, cyclopropyle, -OH-, CH₂OH-, -C(=O)NH₂, -C(=O)CH₃ ou NH₂ ;
et dans lequel le terme « éventuellement substitué » signifie non substitué ou substitué par au moins un substituant sélectionné parmi un groupe alkyle en C₁-C₆, alcoxy en C₁-C₆, hydroxy, hydroxy-alkyle en C₁-C₆, mercapto, mercapto-alkyle en C₁-C₆, alkyl(en C₁-C₆)thio, carbocyclique monocyclique comportant 3 à 6 atomes de carbone cyclique, hétérocyclique monocyclique comportant 5 ou 6 atomes de carbone cyclique, halo, trifluorométhyle, trifluorométhoxy, nitro, nitrile, oxo, -COOH, -COOR^{A}, -COR^{A}, -SO₂R^{A}, -CONH₂, -SO₂NH₂, -CONHR^{A}, -SO₂NHR^{A}, -CONR^{A}R^{B}, -SO₂NR^{A}R^{B}, -NH₂, -NHR^{A}, -NR^{A}R^{B}, -OCONH₂, -OCONHR^{A}, -OCONR^{A}R^{B}, -NHCOR^{A}, -NHCOOR^{A}, -NR^{B}COOR^{A}, -NHSO₂OR^{A}, -NR^{B}SO₂OR^{A}, -NHCONH₂, -NR^{A}CONH₂, -NHCONHR^{B}, -NR^{A}CONHR^{B}, -NHCONR^{A}R^{B} ou -NR^{A}CONR^{A}R^{B}, où R^{A} et R^{B} sont indépendamment un groupe alkyle en C₁ à C₆ ; et si ledit substituant contient un radical alkyle, ce radical alkyle peut être substitué par un groupe carbocyclique monocyclique comportant 3 à 6 atomes de carbone cyclique, ou un groupe hétérocyclique monocyclique comportant 5 ou 6 atomes de carbone cyclique ; et si ledit substituant est ou comprend un groupe carbocyclique monocyclique comportant 3 à 6 atomes de carbone cyclique, ou un groupe hétérocyclique monocyclique comportant 5 ou 6 atomes de carbone cyclique, ce cycle peut lui-même être substitué par l'un quelconque des substituants optionnels non cycliques précédents.

2. Composé selon la revendication 1, dans lequel R₃ est un groupe cyano (-CN).

3. Composé selon la revendication 1 ou dans la revendication 2, dans lequel R₁ est l'hydrogène, un groupe méthoxy, éthoxy, méthylthio, éthylthio, hydroxyéthylthio, méthylamino, diéthylaminométhylthio, méthylaminocarbonylméthylthio ou un groupe de formule (A)-(H) : 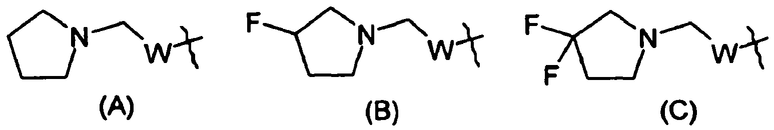 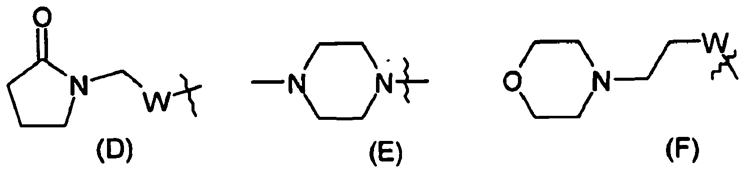 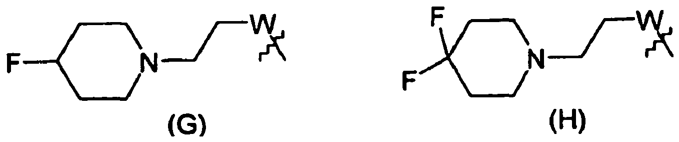 dans laquelle W est -O- ou -S-.

4. Composé selon l'une quelconque des revendications précédentes, dans lequel R₂ est un groupe phényle, éventuellement substitué par un ou plusieurs substituants sélectionnés parmi un groupe méthyle, trifluorométhyle, éthyle, n- ou isopropyle, vinyle, allyle, méthoxy, trifluorométhoxy, éthoxy, méthylènedioxy, éthylènedioxy, n-propyloxy, benzyloxy, allyloxy, cyanométhoxy, fluoro, chloro, bromo, cyano, formyle, méthyl-, éthyl- ou n-propyl-carbonyloxy, méthyl- ou éthyl-aminocarbonyle, et des substituants de formule -O(CH₂)ₙZ¹ ou -S(CH₂)ₙZ¹, où n vaut 1, 2 ou 3 et Z¹ est un groupe amino primaire, secondaire, tertiaire ou cyclique, ce dernier étant éventuellement substitué, ou un groupe alcoxy en C₁-C₆ ; ou de formule -(Alk³)ₘZ¹, dans laquelle Alk³ est un groupe alkylène en C₁-C₃ divalent à chaîne linéaire ou ramifiée, m vaut 0 ou 1, et Z¹ est un groupe amino primaire, secondaire, tertiaire ou cyclique, ce dernier étant éventuellement substitué, ou un groupe alcoxy en C₁-C₆.

5. Composé selon la revendication 4, dans lequel les substituants éventuels sont en position 2 et/ou 4 et/ou 5 du noyau phényle.

6. Composé selon la revendication 1, de formule (II) : 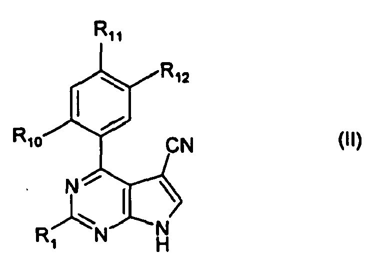 dans laquelle
R₁ est (a) un groupe alkyl(en C₁-C₆)thio ou alcoxy en C₁-C₆ dans chacun desquels un ou plusieurs atomes d'hydrogène sont éventuellement remplacés par des atomes de fluor, ou (b) un substituant de formule -O(CH₂)ₙZ¹ ou -S(CH₂)ₙZ¹, dans laquelle n vaut 1, 2 ou 3 et Z¹ est un groupe amino primaire, secondaire, tertiaire ou cyclique, ce dernier étant éventuellement substitué ;
R₁₀ est H, Cl, Br ou -CH₃ ;
R₁₁ est l'hydrogène, Cl, Br, CN, un groupe méthyle, éthyle, n- ou iso-propyle, méthoxy, éthoxy, vinyle ou allyle ; et
R₁₂ est (i) un radical de formule -O(CH₂)ₙZ¹ ou -S(CH₂)ₙZ¹, dans laquelle n vaut 1, 2 ou 3 et Z¹ est (i) un groupe amino primaire, secondaire, tertiaire ou cyclique, ou un groupe alcoxy en C₁-C₆ ; ou (ii) un radical de formule -(Alk³)ₘZ¹ dans laquelle Alk³ est un groupe alkylène en C₁-C₃ divalent à chaîne linéaire ou ramifiée, m vaut 0 ou 1 et Z¹ est un groupe amino primaire, secondaire, tertiaire ou cyclique, ou un groupe alcoxy en C₁-C₆.

7. Composition pharmaceutique ou vétérinaire comprenant un composé selon l'une quelconque des revendications précédentes, avec un ou plusieurs supports et/ou excipients acceptables sur le plan pharmaceutique ou vétérinaire.

8. Utilisation d'un composé selon l'une quelconque des revendications 1 à 13 dans la préparation d'une composition destinée à l'immunosuppression ou au traitement d'une maladie virale, d'une infection fongique résistant aux médicaments, de maladies inflammatoires telles que l'arthrite rhumatoïde, l'asthme, la sclérose en plaques, le diabète de type I, le lupus, le psoriasis et une maladie intestinale inflammatoire; la mucoviscidose ; une maladie liée à l'angiogenèse telle que la rétinopathie diabétique, l'hémangiome et l'endométriose ; ou destinée à la protection de cellules normales contre une toxicité induite par une chimiothérapie ; ou des maladies dans lesquelles l'impossibilité de subir une apoptose est un facteur sous-jacent ; ou destinée à la protection d'une lésion d'hypoxie-ischémie due à une élévation de Hsp70 dans le coeur et le cerveau ; la tremblante du mouton/maladie de Creutzfeldt-Jakob, la chorée de Huntington et la maladie d'Alzheimer.

9. Utilisation selon la revendication 8 pour le traitement du cancer.
